# FASCICULE DE BREVET EUROPEEN

(11) **EP 1 660 118 B1**
(45) Date de publication et mention de la délivrance du brevet: **09.04.2008**
(21) Numéro de dépôt: 04767439.5
(22) Date de dépôt: 24.06.2004
(51) Int. Cl.: A61K 39/00, C12N 5/06

(54) **PROCEDE D'IDENTIFICATION DE LYMPHOCYTES TR1 REGULATEURS PAR LA PRESENCE ET LA SUREXPRESSION DE MOLECULES SPECIFIQUES ET SES APPLICATIONS**
VERFAHREN ZUR IDENTIFIZIERUNG VON REGULATORISCHEN TR1-LYMPHOZYTEN DURCH ANWESENHEIT UND ÜBEREXPRESSION VON SPEZIFISCHEN MOLEKÜLEN UND SEINE ANWENDUNG
METHOD FOR IDENTIFICATION OF REGULATORY TR1 LYMPHOCYTES BY THE PRESENCE AND OVEREXPRESSION OF SPECIFIC MOLECULES AND APPLICATION THEREOF

(30) Priorité: 24.06.2003 FR 0307601
(43) Date de publication de la demande: 31.05.2006
(73) Titulaire: Txcell, Hopital de l'Archet 06200 Nice (FR)
(72) Inventeur: GROUX, Hervé, F-06410 Biot (FR)
(74) Mandataire: de Mareüil-Villette, Caroline
(86) Numéro de dépôt international: PCT/FR2004/001583
(87) Numéro de publication internationale: WO 2005/000344

(56) Documents cités:
- WO-A-02/092793
- US-B1- 6 277 635
- MOTTET CHRISTIAN ET AL: "Cutting edge: Cure of colitis by CD4+CD25+ regulatory T cells." JOURNAL OF IMMUNOLOGY, vol. 170, no. 8, 15 avril 2003 (2003-04-15), pages 3939-3943, XP002275079 ISSN: 0022-1767 (ISSN print)
- GROUX HERVE ET AL: "A CD4+ T-cell subset inhibits antigen-specific T-cell responses and prevents colitis" NATURE, MACMILLAN JOURNALS LTD. LONDON, GB, vol. 389, no. 6652, 16 octobre 1997 (1997-10-16), pages 737-742, XP002164695 ISSN: 0028-0836
- BARRAT FRANCK J ET AL: "In vitro generation of interleukin 10-producing regulatory CD4(+) T cells is induced by immunosuppressive drugs and inhibited by T helper type 1 (Th1)- and Th2-inducing cytokines." THE JOURNAL OF EXPERIMENTAL MEDICINE. UNITED STATES 4 MAR 2002, vol. 195, no. 5, 4 mars 2002 (2002-03-04), pages 603-616, XP002275080 ISSN: 0022-1007
- GROUX H: "TYPE 1 T-REGULATORY CELLS: THEIR ROLE IN THE CONTROL OF IMMUNE RESPONSES" TRANSPLANTATION, WILLIAMS AND WILKINS, BALTIMORE, MD, US, vol. 75, no. 9, SUPPL, 15 mai 2003 (2003-05-15), pages 8S-12S, XP008025406 ISSN: 0041-1337
- MALLAT ZIAD ET AL: "Induction of a regulatory T cell type 1 response reduces the development of atherosclerosis in apolipoprotein E-knockout mice." CIRCULATION, vol. 108, no. 10, 9 septembre 2003 (2003-09-09), pages 1232-1237, XP009028370 ISSN: 0009-7322 (ISSN print)

## Description

L'invention a pour objet un procédé d'identification de lymphocytes Tr1 régulateurs dans un échantillon biologique basé sur la détermination de la présence simultanée du groupe de molécules CD4, CD18 et/ou CD11a, CD49b et, le cas échéant, par la mise en évidence d'une surexpression des gènes codant pour les molécules CD4, PSGL-1, PECAM-1 et alphaV/beta3. L'invention a également pour objet un procédé de quantification et un procédé de pronostic ou de diagnostic de maladies auto-immunes ou inflammatoires basé sur ledit procédé d'identification. L'invention a également pour objet un procédé d'enrichissement en lymphocytes Tr1 régulateurs basé sur la détermination de la présence simultanée de ces molécules. L'invention a enfin pour objet l'utilisation d'une composition enrichie selon ledit procédé d'enrichissement pour le traitement d'une maladie auto-immune ou inflammatoire, notamment la maladie de Crohn.

La tolérance immunitaire est obtenue par différents mécanismes permettant au système de distinguer le soi du non soi. De plus, le système immunitaire est exposé à des stimulations répétées d'antigènes non pathogènes. Afin d'éviter des activations cellulaires et des inflammations chroniques non souhaitées, le système immunitaire présente des mécanismes coopérant dans le maintien d'une tolérance faisant participer des cellules T anergiques (Blackman et al., 1990, Nature 345, 540-542 ; Jones et al, 1990b, J Exp Med 172, 1277-1285), l'inactivation des cellules T par apoptose (Jones et al, 1990a, Science 250, 1726-1729 ; Webb et al, 1990, Cell 63, 1249-1256), et une suppression immunitaire active (Rocken et al, 1996, Immunol Rev 149, 175-194 ; Weiner et al, 1997, Annu Rev Med 48, 341-351). La suppression immunitaire active est médiée par des cellules T spécialisées dont la fonction est de supprimer la prolifération et l'activation des cellules T effectrices. Des études récentes ont démontré que des cellules régulatrices désignées Tr1 font partie des cellules T CD4+ (Chen et al, 1994, Science 265, 1237-1240 ; Groux et al., 1997, Nature 389, 737-742 ; Mc Guirck et al, 2002, J Exp Med 195, 221-231 ; Powrie et al, 1994, J Exp Med 179, 589-600), et que leur fonction est dépendante de la présence d'IL-10 (Asseman et al, 1999, J Exp Med 190, 995-1004 ; Barrat et al, 2002 J Exp Med 195, 603-616 ; Groux et al, 1997) et de TGF-β (Groux et al, 1997 ; Kitani et al, 2000, J Immunol 165, 691-702).

Parmi les lymphocytes T CD4+, également dénommés lymphocytes T auxiliaires (T helpers), on distingue classiquement 2 types principaux de lymphocytes T auxiliaires: les lymphocytes Thl, impliqués dans le développement de la réponse immune cellulaire, produisent des cytokines pro-inflammatoires telles que l'interleukine-2 (IL-2) et l'interféron gamma (IFNγ) et présentent des effets activateurs des macrophages; les lymphocytes Th2, qui produisent des cytokines telles que les interleukines IL-4, IL-6, IL-10 et IL-13, favorisent la sécrétion d'anticorps.

Dans le thymus, la tolérance centrale est un mécanisme bien établi qui implique une délétion des cellules T autoréactives après interaction avec des cellules dendritiques (DC) dérivées de la moelle osseuse.

Toutefois, les mécanismes par lesquels les cellules Tr se forment in vivo et exercent leurs effets immunorégulateurs restent à définir et font l'objet d'intenses recherches.

En particulier, certaines maladies autoimmunes et/ou inflammatoires font intervenir des cellules Tr1.

Les maladies auto-immunes sont dues à une dérégulation du système immunitaire, qui se traduit par une réponse immune indésirable d'un organisme vis-à-vis de ses propres antigènes. On a tenté de manipuler les antigènes à l'origine de ces pathologies ou les cellules T agressive spécifiques de ces antigènes, mais les résultats obtenus ont souvent été très limités notamment par l'absence d'une connaissance de tous les antigènes impliqués dans la pathologie concernée. En effet, les auto antigènes proprement dits, ou les antigènes responsables des désordres inflammatoires et auto-immuns ne sont pas toujours connus, ou sont différents d'un individu à l'autre (part de l'héritage génétique).

Les traitements utilisés actuellement dans ces maladies sont soit des traitements palliatifs (insuline dans le cas du diabète, anti-histaminiques dans le cas des désordres allergiques) soit des traitements systémiques par des anti-inflammatoires (AINS) et/ou des immuno-suppresseurs (glucocorticoïdes, cyclosporine, anticorps...). Il y a donc clairement un besoin pour un traitement immunosuppresseur puissant mais limité à l'organe atteint ou plus précisément à la zone d'hyperactivité du système immunitaire.

Les cellules Tr1, lorsqu'elles sont re-stimulées par l'antigène utilisé pour leur induction, ne prolifèrent que faiblement, produisent des quantités très élevées d'IL-10, de très faibles quantités d'IL-2, et ne produisent pas d'IL-4. Lorsque des cellules Tr1 activées sont cultivées en présence d'autres cellules T CD4+ elles suppriment la prolifération de celles-ci en réponse à un antigène; cet effet résulte de la sécrétion de cytokines, et notamment d'IL-10, par les lymphocytes Tr, et non d'une action directe de ceux-ci sur les cellules T CD4+ ; il peut donc être obtenu sans avoir à connaître l'antigène responsable de la prolifération de ces cellules. Ceci présente un avantage important dans le cas de maladies auto-immunes, dont on peut ainsi envisager le traitement sans qu'il soit nécessaire de connaître l'antigène exact contre lequel sont dirigées les cellules pathogéniques.

Il a ainsi été observé, dans un modèle expérimental de maladie de Crohn chez la souris dans lequel les cellules pro-inflammatoires sont dirigées contre des bactéries commensales de la flore digestive, que l'administration aux animaux des cellules Tr1 dirigées contre l'ovalbumine, accompagnée de l'administration d'ovalbumine dans la nourriture, permettait de prévenir l'instauration d'une inflammation chronique du colon (Groux et al, 1997, Nature 389, 737-742).

D'autre part, dans des travaux sur différents modèles animaux de maladie de Crohn, de sclérose en plaques, ou de réaction du greffon contre l'hôte, les inventeurs ont montré que les cellules inhibitrices Tr1 étaient capables non seulement de prévenir, mais aussi de guérir ces différentes pathologies (Foussat et al. Submitted, Barrat et al. J. Exp. Med. 2002, 4, 603).
Le document MOTTET et al. (Journal of Immunology, vol.170, no 8, 15 avril 2003, pages 3939-3943) concerne l'étude de la capacité des cellules T CD4+CD25+ régulatrices, connues pour leur rôle dans la prévention de pathologies immunes médiées par des cellules T, à améliorer une inflammation établie. L'étude montre que les cellules T CD4+CD25+ sont capables de traiter une inflammation intestinale et suggère que de telles cellules CD4+CD25+ peuvent être bénéfiques dans le traitement de maladies inflammatoires chroniques.

Des cellules Tr1 obtenues à partir des cellules T d'un patient sont donc potentiellement utilisables dans le cadre d'une thérapie cellulaire pour réguler la réponse immunitaire chez ce patient. Elles sont ainsi utilisables notamment pour prévenir ou soigner, non seulement les maladies auto-immunes et inflammatoires mentionnées ci-dessus, mais également toute autre pathologie caractérisée par une réponse inflammatoire aberrante, comme le diabète, le psoriasis, l'athérosclérose, la polyarthrite rhumatoïde ou l'asthme; elles sont également utilisables dans le traitement des rejets de greffe ou de la réaction du greffon contre l'hôte.

Ainsi, parmi les maladies auto-immunes dans lesquelles des cellules Tr1 peuvent être utilisées, on cite les maladies choisies dans le groupe constitué de l'hépatite active chronique, la maladie d'Addison, le syndrome anti-phospholipide, l'allergie atopique, la gastrite atrophique auto-immune, l'achlorhydrie auto-immune, la maladie céliaque, la maladie de Crohn, le syndrome de Cushings, la dermatomyosite , le diabète de type I, le lupus discoïde, l'érythématose, le syndrome de Goodpasture, la maladie de Grave, la thyroïdite d'Hashimoto, l'atrophie adrénale idiopathique, le diabète insulinodépendant, le syndrome de Lambert-Eaton, l'hépatite lupoïde, certains cas de lymphopénie, la sclérose en plaques, le pemphigus vulgaris ou foliacé, le pemphigoïde bulleux, l'anémie pernicieuse, l'uvéitite phacogénique, la polyarthrite, la cirrhose biliaire primaire, la cholangite sclérosante primaire, le psoriasis, le syndrome de Reiter, la polychondrite, l'arthrite rhumatoïde, le syndrome de Schmidt, la sclérodermose, le syndrome de Sjogren, le Lupus Erythémateux Systémique, l'artérite de Takayasu, l'artérite temporaire, la thyrotoxicose, la résistance à l'insuline de type B , la colite ulcérative, la granulomatose de Wegener, la myasthénie gravis, la maladie de Guillain-Barré, l'uréite auto-immune, l'anémie auto-immune hémolytique, la thrombocytopénie auto-immune, l'oophorite auto-immune, la maladie de Behcet, la dermatite herpétiforme, la polymyosite, la dermatomyosite, les spondyloarthropathies comme la spondylite ankylosante, le vitiligo.

Une des difficultés dans l'utilisation des lymphocytes Tr1 régulateurs est de pouvoir les identifier de manière simplifiée et sûre à partir d'une population lymphocytaire. Les techniques actuelles d'identification des lymphocytes Tr1 régulateurs consistent à étudier le profil de production des cytokines par une population de lymphocytes susceptible de comprendre des lymphocytes Tr1 régulateurs. Notamment, Groux et al. (Nature, 389, 737-742, 1997) décrit que cette population de lymphocytes produit des quantités très élevées d'interleukine 10 (IL-10), des quantités importantes de TGF-β (tumor growth factor β), de très faibles quantités d'interleukine 2 (IL-2) et ne produit pas d'interleukine 4 (IL-4). L'homme de l'art peut confirmer la présence de lymphocytes Tr1 régulateurs dans une population lymphocytaire en étudiant la réponse proliférative des lymphocytes T CD4+ présents dans ladite population : lorsque les lymphocytes Tr1 régulateurs sont cultivés en présence de lymphocytes T CD4+, ils suppriment la prolifération de ceux-ci en réponse à un antigène (Groux et al., Nature, 389, 737-742, 1997). Ces techniques d'identification présentent l'inconvénient d'être longues et laborieuses.

Ainsi, il existe aujourd'hui un besoin de disposer d'une technique rapide et efficace pour identifier la présence ou l'absence de lymphocytes Tr1 régulateurs à partir d'un échantillon contenant une population lymphocytaire. Un tel procédé pourrait être également avantageux car il permettrait d'enrichir une population lymphocytaire en lymphocytes Tr1 régulateurs.

Ceci est justement l'objet de la présente invention.

L'invention a ainsi pour objet un procédé d'identification de lymphocytes Tr1 régulateurs présents dans un échantillon biologique comprenant des lymphocytes, caractérisé en ce qu'il comprend les étapes suivantes :
a) déterminer la présence simultanée des produits d'expression par lesdits lymphocytes des gènes codant pour la molécule CD4 et l'ensemble des molécules du groupe A, ledit groupe A étant constitué par les molécules CD18 et/ou CD11a, et CD49b ; et
b) identifier comme lymphocytes Tr1 régulateurs les lymphocytes qui expriment simultanément les gènes codant pour la molécule CD4 et l'ensemble des molécules du groupe A.

Par groupe A constitué par les molécules CD18 et/ou CD11a, et CD49b, on entend désigner le groupe A constitué par :
- soit les molécules CD18 et CD49b ;
- soit les molécules CD11a et CD49b ; ou
- soit les molécules CD18, CD11a et CD49b.

Parmi les échantillons biologiques comprenant des lymphocytes dans lesquels on cherche à identifier la présence de lymphocytes Tr1 régulateurs, on préfère les échantillons biologiques issus de sang périphérique prélevé chez un sujet, les échantillons biologiques issus d'un procédé de préparation *in vitro* de lymphocytes Tr1 régulateurs à partir d'une population de cellules, notamment des lymphocytes, le cas échéant issues d'un prélèvement chez un sujet, ou encore issus de cellules progénitrices.

Le procédé d'identification de lymphocytes Tr1 régulateurs selon la présente invention peut être réalisé sur tout échantillon biologique comprenant une population de lymphocytes dont on cherche à déterminer si elle contient des lymphocytes Tr1 régulateurs.

Le sujet chez qui le prélèvement de l'échantillon biologique est effectué peut être tout mammifère, notamment la souris, de préférence l'homme. De préférence, ce sujet peut être soit sain, soit atteint d'une maladie auto-immune ou inflammatoire. L'expression « sujet sain » selon l'invention comprend tout sujet, de préférence l'homme, non atteint d'une maladie auto-immune ou inflammatoire. Une liste de maladies auto-immunes a été donnée plus haut ; les maladies inflammatoires sont des maladies dans lesquelles on observe une infiltration de cellules mononucléaires, une prolifération de fibroblastes et de nouveaux vaisseaux sanguins, menant à une augmentation des tissus connectifs, et une destruction tissulaire. On note notamment les maladies inflammatoires chroniques de l'intestin.

L'échantillon biologique peut être également issu de procédés de préparation *in vitro* de lymphocytes Tr1 régulateurs, ces procédés étant bien connus de l'homme du métier. Parmi ces procédés de préparation, on peut citer par exemple le procédé décrit dans la publication d'un inventeur (Groux et al., Nature, 389, 737-742, 1997), qui consiste à stimuler de façon répétée des cellules T CD4+ avec l'antigène en présence d'IL-10. On peut également citer le procédé de préparation in *vitro* de lymphocytes Tr1 régulateurs décrit dans la demande de brevet internationale d'un inventeur publiée le 21 novembre 2002 sous le numéro WO 02/092793 : ce procédé consiste en la mise en culture de lymphocytes T CD4+ en présence de cellules présentatrices d'antigène artificielles exprimant une molécule HLA de classe II et la molécule LFA-3 (CD58) humaine, mais n'exprimant aucune des molécules co-stimulatrices B7-1 (CD80), B7-2 (CD86), B7-H1, CD40, CD23 et ICAM-1 (CD54). Un autre procédé de préparation *in vitro* de lymphocytes Tr1 régulateurs est donné dans l'exemple 5 ci-après, procédé qui consiste à obtenir in *vitro* une population de cellules dendritiques humaines à partir de cellules progénitrices humaines, lesquelles cellules dendritiques sont capables d'induire la différenciation de lymphocytes T humains en lymphocytes Tr1 régulateurs.

Par produit d'expression d'un gène codant pour une molécule dans le procédé d'identification de lymphocytes Tr1 régulateurs selon la présente invention, on entend désigner, pour chacune de ces molécules, le produit d'expression du gène codant pour ladite molécule, celui-ci pouvant être soit le produit de traduction dudit gène, à savoir le peptide codé par ledit gène, ou encore le produit de transcription dudit gène, à savoir l'ARNm codant pour ladite molécule. De préférence, le produit d'expression est ladite molécule exprimée à la surface desdits lymphocytes ou l'un de ses fragments représentatifs, à savoir un fragment de ladite molécule dont la présence à la surface des lymphocytes permet de conclure à l'expression de cette molécule à la surface des lymphocytes (phénotype « + » pour ladite molécule).

Les inventeurs ont en effet mis en évidence que les lymphocytes Tr1 régulateurs peuvent être identifiés en déterminant la présence simultanée du produit d'expression par cesdits lymphocytes du gène codant pour la molécule CD4 et des gènes codant pour les molécules du groupe A (voir exemples 2 et 3).

Les molécules CD11a et CD18 sont associées à la surface des lymphocytes pour former la β₂-intégrine dimérique CD 11a/CD 18, qui porte également le nom de LFA-1 pour Lymphocytes Function Associated Antigen-1 (antigène 1 associé à la fonction des lymphocytes). La présence de cette β₂-intégrine pourra donc être identifiée en déterminant la présence soit du produit d'expression par cesdits lymphocytes du gène codant pour la molécule CD11a, soit du produit d'expression du gène codant pour la molécule CD 18, soit simultanément de ces deux produits d'expression.

Les inventeurs ont mis en outre en évidence que les lymphocytes Tr1 régulateurs peuvent être également identifiés indépendamment du procédé d'identification ci-avant par la surexpression d'au moins 1, de préférence d'au moins 2, 3, 4 ou de tous les gènes codant pour les molécules CD11a, CD18, PSGL-1, PECAM-1 et/ou alphaV/beta 3, par comparaison avec l'expression par des lymphocytes connus comme étant de type Th1 ou Th2, notamment des lymphocytes Th1 ou Th2 issus de clones (voir exemple 4).

La molécule PSGL-1, ou P-selectin glycoprotein-ligand-1 (ligand majeur de type glycoprotéine pour la P-sélectine) est une molécule d'adhésion cellulaire (CAM pour Cell Adhesion Molecule) appartenant à la famille des sélectines.

La molécule PECAM-1 ou Platelet-Endothelial Cell Adhesion Molecule-1 (molécule d'adhésion cellulaire de l'endothélium et des plaquettes) également dénommée molécule CD31, est une molécule CAM appartenant à la famille des molécules Ig-like.

La molécule alphaV/beta3 également dénommée molécule CD51/CD61, ou VNR pour vitronectin receptor (récepteur de la vitronectine), est une molécule CAM appartenant à la sous-famille des β₃-intégrines.

Selon un mode de réalisation particulier de l'invention, le procédé d'identification de lymphocytes Tr1 régulateurs ci-avant basé sur l'expression des molécules du groupe A est caractérisé en ce que :
- à l'étape (a) on compare en outre l'expression par lesdits lymphocytes d'au moins un gène choisi parmi les gènes codant pour les molécules du groupe B suivant : CD11a, CD18, PSGL-1, PECAM-1 et alphaV/beta 3, ladite expression étant comparée avec l'expression dudit même gène par des lymphocytes de type Th1 ou Th2 ; et
- en ce qu'à l'étape (b) on identifie comme lymphocytes Tr1 régulateurs les lymphocytes qui surexpriment au moins l'un desdits gènes codant pour les molécules du groupe B.

Par lymphocytes qui surexpriment au moins l'un des gènes codant pour les molécules du groupe B on entend désigner des lymphocytes dont l'expression d'au moins l'un desdits gènes est significativement plus importante que l'expression de ces mêmes molécules par les lymphocytes de type Th1 ou Th2 ; de préférence, ladite surexpression par les lymphocytes Tr1 régulateurs par rapport à celle obtenue par les lymphocytes Th1 ou Th2 est au moins 2, 3, 4, 5, 7 ou 10 fois supérieure à l'expression sur les lymphocytes de type Th1 ou Th2.

De préférence, les lymphocytes Th1 ou Th2 sont des lymphocytes Th1 ou Th2 issus de clones habituellement obtenus sur des sites inflammatoires notamment cutanés qui sont différenciés in vitro en présence d'IL-4 pour les Th2 ou d'IL-12 pour les Th1.

Selon un autre mode de réalisation particulier de l'invention, le procédé d'identification de lymphocytes Tr1 régulateurs est caractérisé en ce qu'à l'étape (a) on compare l'expression d'au moins deux desdits gènes du groupe B et en ce qu'à l'étape (b) on identifie comme lymphocytes Tr1 régulateurs les lymphocytes qui surexpriment les deuxdits gènes du groupe B.

Selon encore un autre mode de réalisation particulier de l'invention, le procédé d'identification de lymphocytes Tr1 régulateurs est caractérisé en ce qu'à l'étape (a) on compare l'expression de tous les gènes du groupe B et en ce qu'à l'étape (b) on identifie comme lymphocytes Tr1 régulateurs les lymphocytes qui surexpriment tous les gènes du groupe B.

De manière préférée, le procédé d'identification selon l'invention est caractérisé en ce qu'à l'étape (a) on compare l'expression d'au moins trois desdits gènes du groupe B et en ce qu'à l'étape (b) on identifie comme lymphocytes Tr1 régulateurs les lymphocytes qui surexpriment les troisdits gènes du groupe B

De manière encore préférée, le procédé d'identification selon l'invention est caractérisé en ce qu'à l'étape (a) on compare l'expression d'au moins quatre desdits gènes du groupe B et en ce qu'à l'étape (b) on identifie comme lymphocytes Tr1 régulateurs les lymphocytes qui surexpriment les quatre dits gènes du groupe B

L'invention peut également avoir pour objet un procédé d'identification de lymphocytes Tr1 régulateurs présents dans un échantillon biologique comprenant des lymphocytes, caractérisé en ce qu'il comprend les étapes suivantes :
a) comparer l'expression par lesdits lymphocytes d'au moins un gène choisi parmi les gènes codant pour les molécules du groupe B suivant : CD11a, CD18, PSGL-1, PECAM-1 et alphaV/beta 3, ladite expression étant comparée avec l'expression dudit même gène par des lymphocytes de type Th1 ou Th2 ; et
b) identifier comme lymphocytes Tr1 régulateurs les lymphocytes qui surexpriment au moins l'un desdits gènes codant pour les molécules du groupe B.

En outre, les lymphocytes Tr1 régulateurs peuvent également être identifiés par le procédé d'identification selon l'invention dans lequel on détermine en outre la présence du produit d'expression par cesdits lymphocytes du gène codant pour la molécule CD3.

Ainsi, de manière encore préférée, l'invention a pour objet un procédé d'identification selon l'invention caractérisé en ce qu'à l'étape (a) on détermine en outre et simultanément la présence du produit d'expression par lesdits lymphocytes du gène codant pour la molécule CD3 et en ce qu'à l'étape (b) on identifie comme lymphocytes Tr1 régulateurs les lymphocytes qui expriment en outre simultanément le gène codant pour la molécule CD3.

Selon le procédé selon l'invention, l'identification des molécules à la surface des lymphocytes pourra être réalisée par toute méthode appropriée permettant d'identifier spécifiquement la présence simultanée de ces molécules.

Parmi ces méthodes, on préfère les méthodes pour lesquelles l'identification de ces molécules à la surface des lymphocytes est réalisée à l'aide d'anticorps, polyclonaux, monoclonaux, ou recombinants, ou leurs fragments, ou encore de ligands, capables de reconnaître spécifiquement ces molécules, ces anticorps ou fragments d'anticorps pouvant être, le cas échéant, marqués.

De préférence, le procédé d'identification selon l'invention est caractérisé en ce qu'à l'étape (a) on détermine la présence simultanée desdites molécules du groupe A exprimées à la surface desdits lymphocytes.

Dans un mode préféré de réalisation du procédé d'identification selon l'invention, on détermine caractérisé en ce qu'à l'étape (a) on détermine la présence simultanée desdites molécules exprimées à la surface desdits lymphocytes au moyen d'anticorps spécifiques desdites molécules.

Parmi les anticorps spécifiques desdites molécules qui peuvent être utilisés dans le procédé d'identification selon l'invention, on peut citer notamment, mais sans s'y limiter les anticorps anti-CD4 humain ou murin tels que ceux sécrétés par les clones RPA-T4 et H129-19 (Becton Dickinson, Le Pont de Claix - FR), les anticorps anti-CD18 humain ou murin tels que ceux sécrétés par les clones 6.7, C71/16, et Game-46 (Becton Dickinson), les anticorps anti-CD11a murin tels que ceux sécrétés par le clone M17/4 (Biocompare, South San Fransisco - US), les anticorps anti-CD49b humain ou murin tels que ceux sécrétés par les clones AK-7 et Ha1/29 (Becton Dickinson), les anticorps anti-CD31 murin tels que ceux sécrétés par le clone MEC 13.3 (Becton Dickinson), et les anticorps anti-CD3 humain tels que ceux sécrétés par le clone UCHT-1 (Caltag, Burlingame, US).

Comme exemples de ligands naturels autres que des anticorps capables de reconnaître ces molécules, on pourra citer par exemple, mais sans s'y limiter, pour la molécule CD18 (LFA-1), les ligands hu-soluble ICAM-1 (CD54), hu-ICAM-1Fc chimera, hu-ICAM-2Fc chimera (CD102), huICAM-3Fc chimera (CD50), mu ICAM-1 (CD54), mu-ICAM-2Fc chimera (CD102), muICAM-3Fc chimera (CD50), muICAM-SFc chimera (CD50), JAM-1 , JAM-2, JAM-3 ; tous ces ligands sont disponibles dans le commerce (notamment R&D Systems, Minneapolis - US).

Dans un mode de réalisation particulièrement préféré du procédé d'identification selon l'invention, l'identification des molécules dont on cherche à déterminer la présence simultanée à la surface des lymphocytes présents dans ledit échantillon biologique à tester est réalisée de préférence par immunofluorescence, ou encore par une méthode radio-immunologique ou immuno-enzymatique.

En général, pour la préparation d'anticorps polyclonaux, monoclonaux, ou leurs fragments, ou encore recombinants, on pourra se référer aux techniques bien connues de l'homme de l'art, techniques qui sont en particulier décrites dans le manuel « Antibodies » (Harlow et al., Antibodies: A Laboratory Manual, Cold Spring Harbor Publications pp. 726, 1988) ou à la technique de préparation à partir d'hybridomes décrite par Kohler et al. (Kôhler et Milstein. Nature, 256: 495-497, 1975). Des anticorps spécifiques du procédé selon l'invention peuvent être obtenus par exemple à partir de sérum ou de cellule d'un animal immunisé spécifiquement contre ces molécules.

Par fragment d'anticorps capable de reconnaître spécifiquement ces molécules, on entend désigner en particulier les fragments d'anticorps comprenant tout fragment dudit anticorps capable de se fixer spécifiquement sur l'épitope de ladite molécule sur laquelle se fixe l'anticorps dont ledit fragment est issu. Des exemples de tels fragments incluent en particulier des anticorps simple chaîne (scFv) ou des fragments monovalents Fab ou Fab' et des fragments divalents tels que F(ab')2, qui possèdent la même spécificité de fixation que l'anticorps dont ils sont issus. Ces fragments d'anticorps peuvent être obtenus à partir des anticorps polyclonaux ou monoclonaux par des méthodes telles que la digestion par des enzymes, comme la pepsine ou la papaïne et/ou par clivage des ponts disulfures par réduction chimique. D'une autre manière ces anticorps, ou leurs fragments, pourront également être obtenus par recombinaison génétique (anticorps recombinants).

Les anticorps ou leurs fragments, capables de reconnaître spécifiquement ces molécules, peuvent également se présenter sous forme d'anticorps marqués afin d'obtenir un signal directement ou indirectement détectable et, de préférence, quantifiable.

Les anticorps marqués, ou leurs fragments, qui peuvent être utilisés dans le procédé d'identification selon la présente invention sont par exemple et de préférence des anticorps dits immunoconjugués qui sont conjugués avec des marqueurs fluorescents qui incluent notamment la fluorescéine et ses dérivés, tels que l'isothiocyanate de fluorescéine (FITC), ou encore l'allophycocyanine (APC), la phycoérythrine-cyanine 5 (PC5) et la phycoérythrine (PE), le diacétate de fluorescéine fluorescente verte, la calcéine AM, la tétraméthyl rhodamine fluorescente rouge ou encore la rhodamine et ses dérivés, la GFP (GFP pour « Green Fluorescent Protein »), le dansyl, l'umbelliférone etc..

De tels anticorps immunoconjugués sont disponibles dans le commerce, notamment l'anti-CD4 APC-conjugué (RPA-T4, Beckton Dickinson), l'anti-CD3 PC5-conjugué (UCHT-1, Caltag, Burlingame, US), l'anti-CD18 PE-conjugué (6.7, Beckton Dickinson) et l'anti-CD49b FITC-conjugué (AK-7, Beckton Dickinson) chez l'homme, et l'anti-CD4 de souris PC5-conjugué (H129-19, Beckton Dickinson), l'anti-CD18 de souris PE-conjugué (C71/16, Beckton Dickinson) et l'anti-CD49b de souris FITC-conjugué (Hal/29, Beckton Dickinson).

Les anticorps marqués, ou leurs fragments, qui peuvent être utilisés également dans le procédé d'identification selon la présente invention incluent également des anticorps immunoconjugués conjugués avec des enzymes telles que la péroxydase, la phosphatase alkaline, la β-D-galactosidase, la glucose oxydase, la glucose amylase, l'anhydrase carbonique, l'acétyl-cholinestérase, le lysozyme, la malate déhydrogénase ou la glucose-6 phosphate déhydrogénase ou par une molécule comme la biotine, la digoxigénine ou la 5-bromo-désoxyuridine.

Dans de tels conjugués, les anticorps ou leurs fragments peuvent être préparés par des méthodes connues de l'homme de l'art. Ils peuvent être couplés aux enzymes ou aux marqueurs fluorescents directement ou par l'intermédiaire d'un groupe espaceur ou d'un groupe de liaisons tel qu'un polyaldéhyde, comme le glutaraldéhyde, l'acide éthylènediaminetétraacétique (EDTA), l'acide diéthylènetriaminepentaacétique (DPTA), ou en présence d'agents de couplage tels que le périodate etc.. Les conjugués comportant des marqueurs de type fluoréscéine peuvent être préparés par réaction avec un isothiocyanate.

D'autres conjugués peuvent inclure également des marqueurs chimioluminescents tels que le luminol et les dioxétanes ou des marqueurs bioluminescents tels que la luciférase et la luciférine.

Parmi les marqueurs pouvant être fixés sur ces anticorps ou sur leurs fragments qui peuvent être utilisés dans le procédé d'identification selon la présente invention, on peut également citer les marqueurs radioactifs tels que ¹⁴C, ³⁶Cl, ⁵⁷Co, ⁵⁸Co, ⁵¹Cr, ¹⁵²Eu, ⁵⁹Fe, ³H, ¹²⁵I, ¹³¹I, ³²P, ³⁵S, ⁷⁵SE et ^{99m}Tc qui peuvent être détectés par des moyens connus tels que le compteur gamma ou à scintillations, par autoradiographie, etc..

Toute méthode classique dépendant de la formation d'un complexe immun anticorps-molécule (antigène) peut être mise en oeuvre pour réaliser une telle identification. Ladite méthode, si elle est de type radio-immunologique ou immuno-enzymatique peut être une méthode par compétition ou par sandwich, ou toute méthode connue de l'homme de l'art.

Dans un mode de réalisation particulièrement préféré, l'étape (a) de détermination du procédé d'identification selon la présente invention est réalisée à l'aide d'anticorps marqués avec des marqueurs fluorescents. De préférence encore, chacun desdits anticorps ou fragment d'anticorps spécifique d'une molécule dont on veut déterminer la présence, sera marqué avec un marqueur différent pour chacune des spécificités. De manière encore plus préférée, ladite méthode d'identification permet également de quantifier dans l'échantillon biologique à tester le nombre ou la proportion de lymphocytes présentant simultanément lesdites molécules dont on cherche à identifier la présence, comme par exemple, mais sans s'y limiter, la méthode d'identification et de quantification utilisée dans les exemples 2, 3 et 4 ci-après.

Selon un mode particulier de réalisation de l'invention, le procédé est caractérisé en ce que lesdits marqueurs sont fluorescents et sont choisis dans le groupe constitué de l'isothiocyanate de fluorescéine (FITC), l'allophycocyanine (APC), la phycoérythrine-cyanine 5 (PC5), la phycoérythrine (PE), le diacétate de fluorescéine fluorescente verte, la calcéine AM, et la tétraméthyl rhodamine fluorescente rouge.

Parmi les procédés d'identification que l'on peut utiliser, il existe la technique FACS (flluorescence-activated cell sorter), qui consiste en un système électronique pour séparer les cellules selon leur taille et l'intensité de la fluorescence qu'elles émettent après divers marquages. L'appareil prépare des microgouttes de la suspension cellulaire qui sont diluées de façon à ne contenir qu'une seule cellule. La microgoutte passe devant un faisceau lumineux à rayon laser et les cellules sont analysées (histogramme) et séparées sur la base de leur fluorescence et/ou de leur taille.

Dans un mode préféré de réalisation, le procédé d'identification selon l'invention est caractérisé en ce qu'à l'étape (a) la détermination de la présence simultanée desdites molécules du groupe A exprimées à la surface desdits lymphocytes est mise en oeuvre par cytométrie de flux.

On entend par cytométrie de flux toute technique permettant le comptage et la mensuration de cellules, notamment la technique FACS telle que décrite plus haut.

Lorsque l'étape (a) de détermination du procédé d'identification selon la présente invention est réalisée à l'aide d'immunoconjugués fluorescents (tels que des anticorps fluorescents), on préfère un procédé d'identification selon l'invention dans lequel, à l'étape (a) dudit procédé, la molécule CD18 est déterminée comme présente à la surface desdits lymphocytes lorsque le niveau d'intensité de fluorescence obtenu pour cette molécule correspond à celui obtenu pour cette même molécule à la surface des cellules monocytaires (« CD18bright »).

Selon un mode préféré de réalisation, le procédé d'identification selon l'invention est caractérisé en ce qu'à l'étape (a) dudit procédé on détermine, pour la molécule CD18, la présence d'une intensité de fluorescence de type CD18bright.

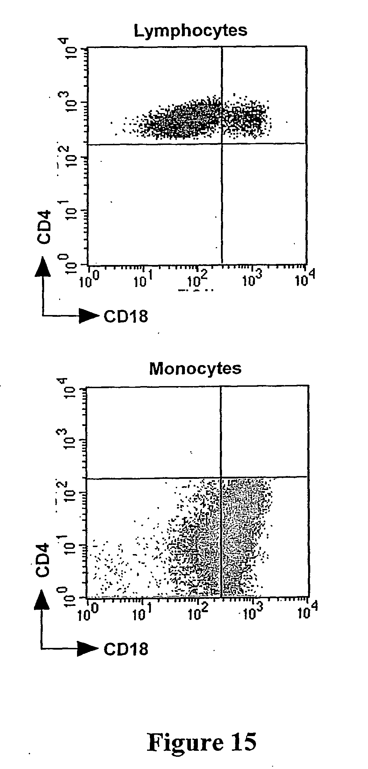

Ainsi, par exemple, par phénotype « CD3+ CD4+ CD18bright » pour des lymphocytes T humains on entend désigner des lymphocytes qui expriment des molécules ou antigènes CD3, CD4 et CD18 à la surface cellulaire, l'intensité de fluorescence du marquage de la molécule CD18 étant égale à l'intensité de fluorescence de ce même marquage retrouvée sur les cellules monocytaires (cf. Figure 15).

La comparaison de l'expression par lesdits lymphocytes des gènes codant pour les molécules du groupe B peut être effectuée par comparaison de la quantité d'ARNm exprimé pour cesdits gènes.

Une telle comparaison de l'expression d'ARNm peut être effectuée en utilisant la technique d'amplification en chaîne par polymérase précédée d'une étape de transcription inverse (RT-PCR).

Selon un mode de réalisation, le procédé d'identification selon la présente invention est caractérisé en ce que :
- à l'étape (a) la comparaison de l'expression par lesdits lymphocytes d'au moins un gène codant pour les molécules du groupe B est effectuée par comparaison de la quantité d'ARNm exprimé pour ledit gène ; et
- en ce qu'à l'étape (b) on identifie comme lymphocytes Tr1 régulateurs les lymphocytes qui surexpriment l'ARNm dudit gène.

Selon un autre mode de réalisation, le procédé d'identification selon la présente invention est caractérisé en ce que l'on mesure la quantité d'ARNm par RT- PCR quantitative.

Selon un autre mode préféré de réalisation, le procédé d'identification selon l'invention est caractérisé en ce que l'échantillon biologique est issu d'un prélèvement de sang périphérique ou d'un organe inflammatoire chez un sujet.

De préférence, le prélèvement dudit échantillon biologique dans le procédé selon l'invention est effectué chez un sujet atteint ou susceptible d'être atteint d'une maladie auto-immune ou inflammatoire.

De manière encore plus préférée, ledit sujet est atteint de la maladie de Crohn ou de la sclérose en plaques.

Dans un autre mode de réalisation, le procédé d'identification de lymphocytes Tr1 régulateurs selon l'invention est caractérisé en ce que l'échantillon biologique est issu d'un procédé de préparation *in vitro* de lymphocytes Tr1 régulateurs à partir d'une population de lymphocytes issus d'un prélèvement chez un sujet.

De préférence, ledit procédé de préparation *in vitro* de lymphocytes Tr1 régulateurs à partir de la population de lymphocytes comprend au moins une étape d'activation de lymphocytes T CD4+ de ladite population de lymphocytes en présence d'un antigène et d'interleukine 10.

Egalement, ledit procédé de préparation *in vitro* de lymphocytes Tr1 régulateurs comprend les étapes suivantes :
a) obtenir un échantillon biologique contenant des cellules présentatrices d'antigène artificielles qui expriment une molécule du système HLA de classe II et une molécule de LFA-3 humain et qui n'expriment aucune des molécules de co-stimulation B7-1, B7-2, B7-H1, CD40, CD23 ou ICAM-1 ;
b) activer *in vitro* les lymphocytes T CD4+ de ladite population de lymphocytes en présence de l'antigène choisi, présenté par les cellules présentatrices d'antigène artificielles obtenues en (a) ; et
c) récupérer, à partir desdits lymphocytes, une population de lymphocytes CD4+ activés comprenant au moins 10% de lymphocytes Tr1 spécifiques de l'antigène choisi.

De manière encore préférée, ledit procédé de préparation *in vitro* de lymphocytes Tr1 régulateurs comprend les étapes suivantes :
a) obtenir *in vitro* une population de cellules progénitrices humaines capables de se différencier en cellules dendritiques ;
b) mettre en culture.lesdites cellules progénitrices humaines en présence d'IL-10 pour obtenir une population desdites cellules dendritiques ; et
c) mettre en présence ladite population de lymphocytes humains avec la population de cellules dendritiques obtenues en (b).

Une telle procédure de préparation est donnée en exemple plus loin (Exemple 5).

Dans un autre mode de réalisation, le procédé d'identification selon l'invention est caractérisé en ce que les produits d'expression par lesdits lymphocytes des gènes codant pour les molécules du groupe A sont des ARNm, et en ce qu'à l'étape (a) la détermination de la présence simultanée desdits ARNm est réalisée par RT- PCR.

Un procédé d'identification selon l'invention par lequel on détermine la présence simultanée des ARNm peut être par exemple la technique d'amplification en chaîne par polymérase précédée d'une étape de transcription inverse (RT-PCR).

Sous un autre aspect, l'invention a également pour objet un procédé de quantification de lymphocytes Tr1 régulateurs présents dans un échantillon biologique comprenant des lymphocytes, caractérisé en ce qu'il comprend les étapes consistant à :
a) identifier les lymphocytes Tr1 régulateurs par un procédé d'identification selon l'invention ; et
b) déterminer la proportion des lymphocytes Tr1 régulateurs identifiés en (a) par rapport à la quantité totale des lymphocytes ou d'une fraction particulière des lymphocytes, présents dans ledit échantillon biologique.

Un tel procédé de quantification peut être réalisé par exemple au moyen de la cytométrie de flux comme décrit plus haut.

L'invention a également pour objet un procédé de pronostic ou de diagnostic *in vitro* d'une maladie auto-immune ou inflammatoire chez un sujet à partir d'un échantillon biologique préalablement prélevé chez ledit sujet, caractérisé en ce qu'il comprend les étapes suivantes :
a) déterminer la proportion de lymphocytes Tr1 régulateurs présents dans ledit échantillon biologique par rapport à la quantité totale des lymphocytes, ou d'une fraction particulière des lymphocytes, selon le procédé de quantification selon l'invention, ou selon tout procédé permettant la quantification de cesdits lymphocytes Tr1 régulateurs ; et
b) comparer la proportion desdits lymphocytes Tr1 régulateurs obtenue à l'étape (a) par rapport à celle présente dans un échantillon biologique prélevé chez un sujet sain.

Dans le procédé de pronostic ou de diagnostic ci-avant, à l'étape (a), on entend désigner par tout procédé permettant la quantification desdits lymphocytes Tr1 régulateurs un procédé tel que notamment en évaluant la proportion de lymphocytes ayant un profil de production des cytokines et/ou capables de diminuer la prolifération des cellules T CD4+ tel que décrit dans « Groux et al., 1997 » et dans le document brevet publié sous le numéro WO 02/092793.

Dans un mode de réalisation préférée, le procédé de pronostic ou de diagnostic *in vitro* d'une maladie auto-immune ou inflammatoire selon l'invention est caractérisé en ce qu'à l'étape (b) dudit procédé on observe une diminution de ladite proportion chez le sujet à tester.

En effet, on a pu observer que des patients atteints d'une maladie auto-immune ou inflammatoire présentent une diminution de la proportion de lymphocytes Tr1 régulateurs par rapport à celle présente chez un sujet sain, c'est-à-dire non atteint par ladite maladie.

Le procédé de pronostic ou de diagnostic *in vitro* d'une maladie auto-immune ou inflammatoire présente l'avantage majeur d'être rapide et efficace, et de pouvoir être effectué par exemple à partir d'un prélèvement sanguin effectué chez un sujet atteint ou susceptible d'être atteint de ladite maladie. Ainsi, les méthodes invasives ou qui nécessitent une hospitalisation peuvent être évitées.

L'invention a encore pour objet un procédé d'enrichissement en lymphocytes Tr1 régulateurs présents dans un échantillon biologique comprenant des lymphocytes, caractérisé en ce qu'il comprend les étapes suivantes :
a) identifier les lymphocytes Tr1 régulateurs par le procédé d'identification selon l'invention ; et
b) éliminer dudit échantillon une part significative des lymphocytes ne présentant pas simultanément lesdites molécules.

Parmi les procédés qui peuvent être mis en oeuvre dans le procédé d'enrichissement selon la présente invention, on préfère un procédé mettant en oeuvre un trieur de cellules basé sur la reconnaissance simultanée desdites molécules, comme notamment un cytomètre de flux capable de trier et de séparer toute cellules présentant simultanément les molécules dont on cherche à déterminer la présence à leur surface.

Toutefois, le procédé d'enrichissement selon l'invention peut également être mis en oeuvre autrement que par cytométrie flux. En effet, l'enrichissement des lymphocytes Tr1, notamment par exemple les lymphocytes Tr1 identifiés selon le procédé d'identification de l'invention par l'expression à leur surface des molécules CD3, CD4, CD18 et CD49b (CD3+CD4+CD18brightCD49b+) à partir d'une population lymphocytaire peut se réaliser à l'aide de billes magnétiques suivant deux étapes : la première étape permettra de dépléter de la population totale à l'aide de billes magnétiques adsorbées par un anticorps anti-Ig de souris les cellules marquées par les anticorps de souris anti-CD8, anti-CD14, anti-CD56 et anti-CD19 humain. En effet, les cellules Tr1 n'expriment pas les molécules CD8, CD14, CD56 et CD19 humaines. La deuxième étape est une sélection positive des cellules exprimant CD49b (CD49b+) par un marquage des cellules enrichies par un anticorps de souris anti-CD49b humain et sélection positive des cellules marquées par billes magnétiques adsorbées par un anticorps anti-Ig de souris. Les méthodes de séparation cellulaires par billes magnétiques et cytométrie de flux peuvent se complémenter pour la purification de lymphocytes Tr1, par exemple ceux identifiés par l'expression CD3+CD4+CD18brightCD49b+, Par exemple par une sélection positive par billes magnétiques des cellules exprimant CD4 (CD4+) puis détection en cytométrie de flux des molécules CD3, CD18 et CD49b ou bien sélection positive par billes magnétiques des cellules exprimant (CD3+) puis détection en cytométrie de flux des molécules CD4, CD 18 et CD49b ou bien encore sélection positive par billes magnétiques des cellules exprimant (CD49b+) puis détection en cytométrie de flux des molécules CD3, CD18 et CD49b. Une telle procédure est également applicable à la détection des autres molécules du procédé d'identification selon l'invention (CD11a, PSGL-1, PECAM-1, alphaV/beta3,...).

Le brevet décrit l'utilisation d'une population enrichie en lymphocytes Tr1 régulateurs par un procédé d'enrichissement selon l'invention pour la fabrication d'un médicament destiné à prévenir et/ou à traiter une maladie auto-immune ou inflammatoire telles que celles citées ci-avant.

De préférence, ladite utilisation est caractérisée en ce que les lymphocytes Tr1 régulateurs sont administrés au niveau d'une zone d'inflammation.

Dans la présente demande, par zone d'inflammation ou inflammée on définit une zone présentant une accumulation de fluides, de protéines de plasma, de cellules immunitaires, de substances telles que des protéines, laquelle accumulation est due à une blessure locale, une infection, une blessure immunitaire locale. L'inflammation pourra être épisodique ou chronique.

De manière encore plus préférée, ladite utilisation est caractérisée en ce que les lymphocytes Tr1 régulateurs sont administrés avec un antigène capable d'activer *in vivo* lesdits lymphocytes.

Ainsi, les lymphocytes Tr1 régulateurs présents au niveau de la zone d'inflammation pourront être activés *in vivo* par ledit anigène.

On peut administrer les lymphocytes Tr1 régulateurs préalablement activés *in vitro* ou *in vivo.*

Enfin, le brevet décrit aussi une méthode de traitement d'une maladie auto-immune ou inflammatoire comprenant l'étape d'administration à un patient d'une population enrichie en lymphocytes Tr1 régulateurs par un procédé d'enrichissement selon l'invention.

Les légendes des figures et exemples qui suivent sont destinées à illustrer l'invention sans aucunement en limiter la portée.

### LEGENDES DES FIGURES

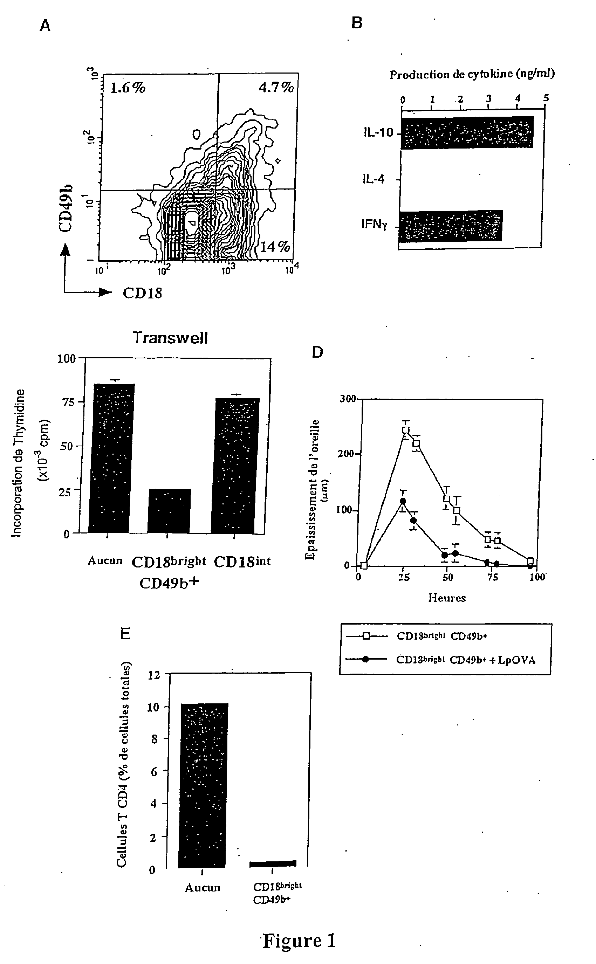

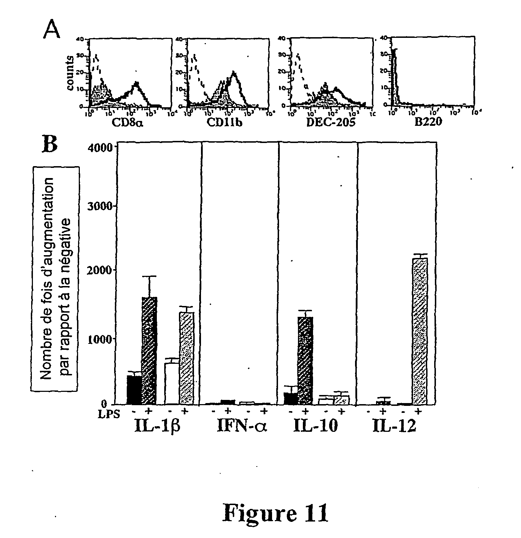

**Figure 1** **: Caractérisation des cellules T CD4+CD18^{bright}CD49b⁺ comme cellules Tr1 chez la souris.**
   **A)** Analyse par FACS des cellules T CD4+CD18bright CD49b+ à l'aide des anticorps anti-CD4, anti-CD18 et anti-CD49b.
   **B)** Profil de production des cytokines des cellules T CD4+CD18bright CD49b+.
   **C)** Mesure de la prolifération de cellules T CD4+ cultivées seules (contrôle) ou en co-culture séparé par une membrane perméable avec des cellules CD4+CD18bright CD49b+ ou CD4+CD18int pendant 48 heures
   **D)** Etude de l'épaississement de l'oreille suite à l'application épicutanée de l'haptène oxazolone chez la souris et suite à l'injection de cellules T CD4+CD18brightCD49b+ et chez des souris traitée par l'haptène mais non injectées.
   **E)** Etude de la proportion de lymphocytes T CD4+ dans le colon chez les souris auxquelles à été induite une inflammation chronique de l'intestin et injectées ou non par des cellules T CD4+CD18bright CD49b+.

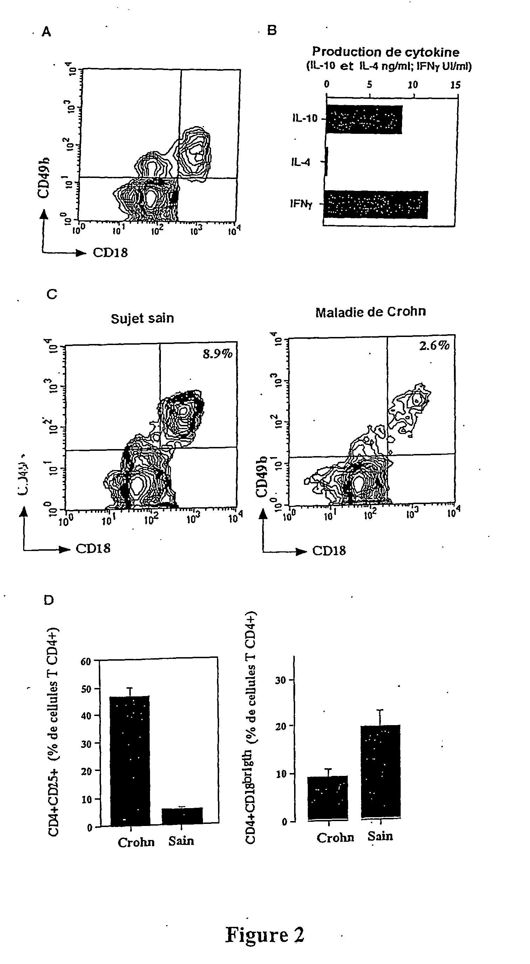

**Figure 2** **: Caractérisation des cellules T CD4⁺CD18^{bright} CD49b⁺ comme cellules Tr1 chez l'homme.**
   **A)** Analyse par FACS des cellules T CD3+CD4+CD18brightCD49b+ à l'aide des anticorps anti-CD3, anti-CD4, anti-CD18 et anti-CD49b.
   **B)** Profil de production des cytokines des cellules T CD4+CD18bright CD49b+.
   **C)** Analyse comparative par FACS des populations de cellules T CD3+CD4+CD18brightCD49b+ présentes dans le sang des sujets sains et de patients atteints de la maladie de Crohn.
   **D)** Etude comparative par FACS des populations de cellules T CD3+CD4+CD18bright et CD4+CD25+ présentes dans le sang des sujets sains et de patients atteints de la maladie de Crohn (analyse par cytometrie de flux).

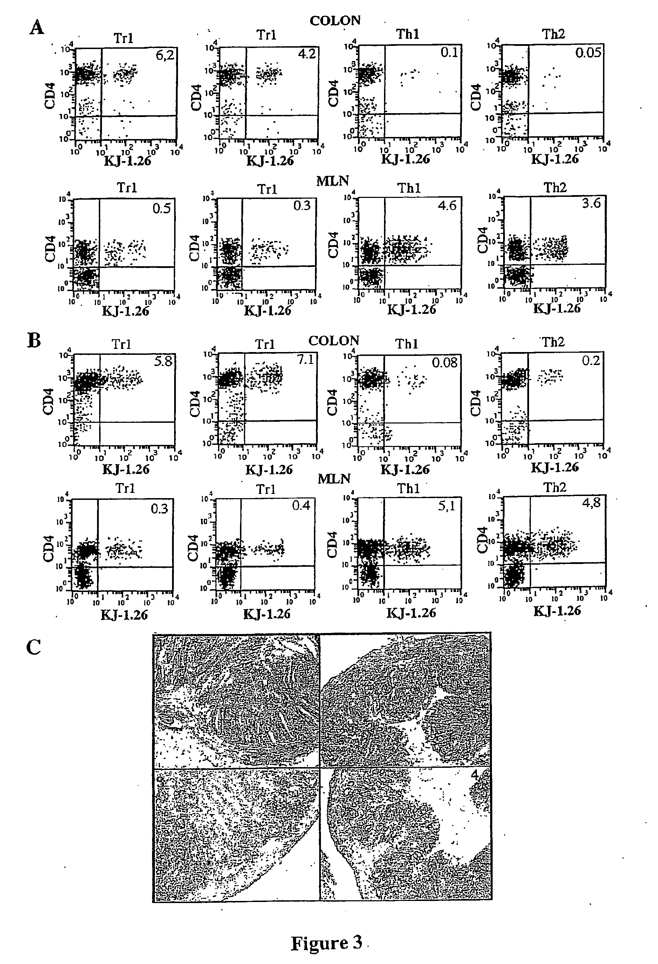

**Figure 3** **: Les cellules Tr1 migrent préférentiellement vers le côlon inflammé**
   **A-B)** les souris scid CB-17 restaurées avec des cellules T CD4⁺CD45Rb^{hi} ont été traitées quatre semaines plus tard avec 5 x 10⁵ clones de cellules T Tr1 (Tr1 1:N10-7 et Trl-2:A-10-9), Th1 (N12-8) et Th2 (N4-9) comme indiqué. Les souris ont soit été nourries avec de l'OVA dans leur eau potable (100 ng/ml) (A) ou non nourries avec de l'OVA (B). Une semaine plus tard, les souris ont été sacrifiées et les cellules présentes dans le côlon et les ganglions lymphatiques mésentériques (MLN) à partir de chaque groupe de 5 souris ont été combinées. La présence de cellules CD4⁺KJ-1,26⁺ a été analysée par cytométrie et les résultats indiqués sur les quadrants. Les résultats représentent une expérimentation représentative sur 4 employant des populations de cellules T Tr1, Th1 ou Th2 ou des clones de cellules T, A-10-11, N-10-11 pour cellules T Tr1, N-12-4 et N-4-12 respectivement pour les clones de cellules T Th1 et Th2.
   **C)** les souris scid CB-17 ont été restaurées avec des cellules T CD4⁺CD45RB^{hi} et traitées avec un clone Tr1 (Nice-2, 4x10⁵ cellules/souris) au jour 0. Un groupe de souris (1 et 3) n'a pas été nourri avec de l'OVA alors que l'autre groupe de souris (2 et 4) a été nourri avec de l'OVA dans son eau potable. Huit semaines plus tard, les souris ont été sacrifiées et leurs côlons examinés par histologie (groupes 1 et 2). La présence de cellules Tr1 a été analysée par immunohistochimie en utilisant un anticorps KJ-1,26 biotinylé (groupes 3 et 4). Une inflammation élevée a été détectée dans les côlons des souris qui n'ont pas été nourries avec de l'OVA (1,3) alors qu'une activation spécifique des clones Tr1 inhibe l'inflammation (2,4). En dépit de l'absence d'OVA, de nombreuses cellules KJ-1,26⁺ ont été détectées dans les côlons inflammés (groupe 3). Des résultats similaires ont été obtenus dans d'autres expérimentations employant les mêmes ou différentes populations ou clones Tr1.

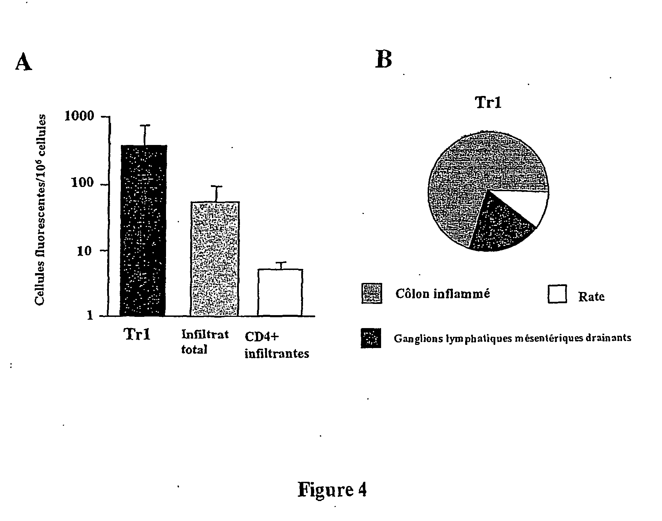

**Figure 4** **: la migration de cellules Tr1 est antigène-indépendante.**
   **A)** Les cellules infiltrant le côlon inflammé des souris scid CB-17 reconstituées avec cellules T CD4⁺CD45Rb^{hi} ont été récupérées quatre semaines après reconstitution. Ces cellules contenant un mélange de neutrophiles, de basophiles et de lymphocytes ont été marquées par de la calcéine fluorescente, AM. 10⁶ de ces cellules fluorescentes (barres grises) ont été co-injectées par voie intrapéritonéale avec 10⁶ cellules Tr1 (barres noires) marquées avec CMTMR orange dans les souris scid C.B.-17 reconstituées quatre semaine auparavant avec des cellules T CD4⁺CD45RB^{hi}. Des expérimentations similaires ont été exécutées avec des cellules T CD4⁺ isolées à partir d'un côlon inflammé de 4 semaines (barres blanches). Vingt-quatre heures plus tard, le contenu cellulaire du côlon inflammé des groupes de deux souris a été examiné par cytométrie. Des histogrammes montrent le nombre absolu de cellules fluorescentes récupérées par million de cellules totales acquises. Les résultats représentent une expérimentation représentative sur 3.
   **B)** Les taux de nombres absolus de cellules fluorescentes Tr1 récupérées par rapport au nombre total de cellules fluorescentes récupérées sont représentés pour chaque organe analysé.

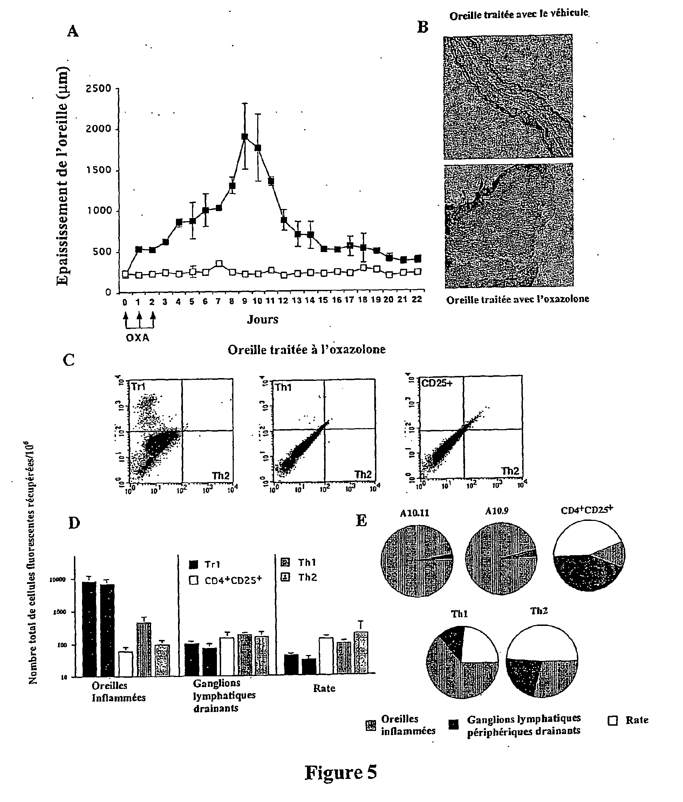

**Figure 5** **: les cellules Tr1 migrent en direction des oreilles inflammées.**
   **A)** Cinétique de la sensibilité de contact à l'oxazolone. Les souris BALB/c ont été traitées avec l'oxazolone (1 mg/oreille) au jour 0, 1 et 2. Le gonflement de l'oreille traitée à l'oxazolone (■) a été comparé à l'oreille traitée avec le véhicule de contrôle pendant 22 jours.
   **B)** Analyse histologique de l'oxazolone ou des oreilles traitées avec le véhicule (grossissement X40) comme indiqué.
   **C-E)** La migration in vivo des cellules Th1, Th2 et Tr1. Les cellules T Tr1, Th1 et Th2 ont été respectivement marquées avec le bleu de calcéine, l'AM et le CMTMR orange et co-injectées chez les souris BALB/c traitées 5 jours auparavant avec de l'oxazolone. Vingt-quatre jours plus tard, le contenu des lymphocytes T fluorescents des oreilles traitées à l'oxazolone, des ganglions lymphatiques drainants et des rates a été analysé par cytométrie, comme représenté en C. Les expérimentations ont été exécutées avec un clone Tr1 (A-10-11, premières barres noires) ou des populations de cellules T Tr1 (deuxièmes barres noires), Th1 et Th2 comme indiqué (D) - Nombres absolus de cellules Tr1, Th1 et Th2 mesurés dans les oreilles inflammées, les ganglions lymphatiques drainants et les rates par 10⁶ événements acquis. Les résultats représentés sont des moyennes de groupes de 4 à 10 souris. (E)- Les taux de nombres absolus de cellules fluorescentes Th1, Th2 ou Tr1 par rapport au nombre total de cellules fluorescentes récupérées sont représentés pour chaque organe analysé.

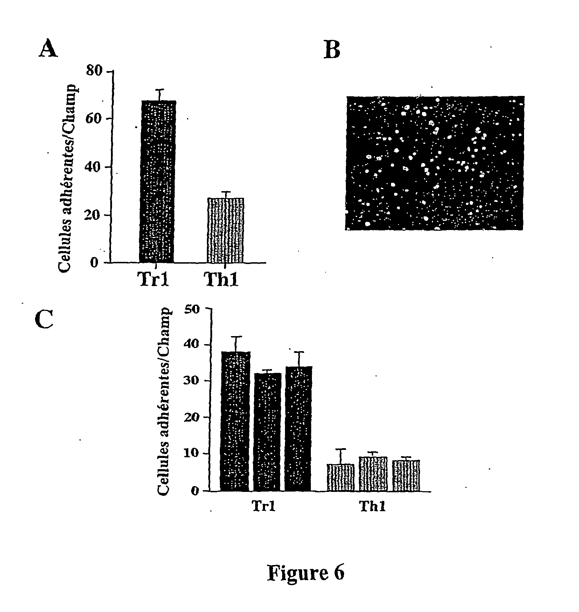

**Figure 6** **: les cellules Tr1 ont affiché un arrêt ferme accru sur des cellules endothéliales vasculaires activées dans des conditions de flux.**
   A) Deux colorants fluorescents différents ont été utilisé dans cette étude : calcéine, AM et CMTMR orange pour marquer des cellules Th1 et Tr1. Les cellules ont été mélangées en nombre égal et perfusées dans une chambre d'écoulement sur une lignée cellulaire endothéliale murine activée par TNF-α, SVEC4-10 à un débit de 2 dyn/cm² pour 15 min. Le nombre de cellules fermement arrêtées a été quantifié en 10 champs indépendants. Les résultats représentent la moyenne ± SD de cinq mesures indépendantes pour deux clones et deux populations dans chaque groupe. Les cellules ont été marquées indifféremment avec les différents colorants et des résultats similaires ont été obtenus.
   **B)** Champ typique représentant des cellules Th1 marquée par la calcéine verte et des cellules Tr1 marquées par le CMTMR orange fermement arrêtées sur la lignée cellulaire endothéliale murine activée par TNF-α, SVEC4-10, grossissement X10.
   **C)** Pour les clones de cellules T humaines, une ferme adhésion a été analysée sur la lignée cellulaire endothéliale murine activée par TNF-α EA en employant la même procédure expérimentale que ci-dessus. Plusieurs clones ont été utilisés, respectivement JDV 15, BJF 308 et BJF 161 pour les clones Tr1 et JDV305 BJF180 et HAT203 pour les clones Th1. Les résultats représentent une expérimentation sur 4 résultats similaires.

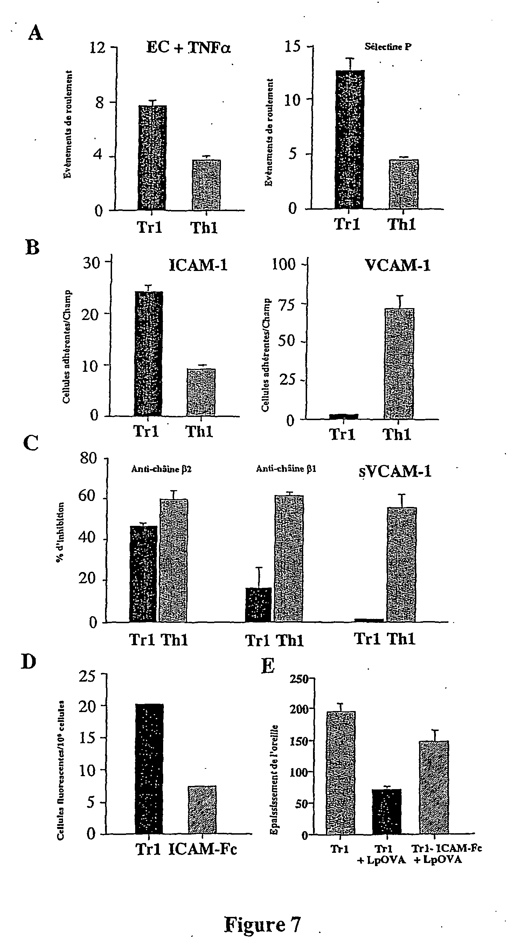

**Figure 7** **: mécanismes pour l'adhésion accrue des cellules Tr1 sur l'endothélium activé**
   Le test d'adhésion sur la chambre d'écoulement avec un taux de 2 dyn/cm² a été utilisé pour disséquer les mécanismes d'adhésion Tr1. Le roulement et l'adhésion ont été comparés pour les cellules Th1 et Tr1 respectivement avec de l'AM calcéine et le CMTMR orange. Les résultats représentent une moyenne ± SD d'une expérimentation représentative pour deux clones et deux populations pour chaque groupe.
   **A)** Les événements de roulement des cellules Th1 et Tr1 sur la lignée cellulaire endothéliale SVEC4-10 activée par TNF-α (EC) ou sur des lames recouvertes de la molécule P-sélectine recombinante ont été comptés après enregistrement vidéo de 9 champs différents. Une expérimentation sur cinq.
   **B)** L'adhésion ferme des cellules Th1 et Tr1 a été analysée sous flux sur des lames recouvertes de recombinant mICAM-1 ou mVCAM-1. Une expérimentation sur trois.
   **C)** Effets inhibiteurs des chaînes de l'intégrine mAb anti-β2, anti-β1 et la molécule sVCAM-1 sur l'arrêt ferme des lymphocytes Tr1 et Th1 sur SVEC4-10 activé par TNF-α a été analysée sous flux. Une expérimentation sur cinq.
   **D)** *Migration inhibée par LFA-1 bloquant des cellules Tr1 dans les oreilles inflammées.* Les cellules Tr1 ont été traitées avec ICAM-Fc (10 µg/ml) pour 30 min à 4°C et marquées avec le bleu de calcéine. Les cellules Tr1 non traitées ont été marquées avec le CMTMR orange. Un mélange de cellules Tr1 traitées avec ICAM-1 Fe non traitées ont été co-injectées chez des souris BALB/c sensibilisés 5 jours auparavant avec de l'oxazolone comme sur la figure 5. Vingt-quatre heures plus tard, le contenu de cellules T fluorescentes des oreilles traitées à l'oxazolone a été analysé par cytométrie. Les résultats montrent les nombres absolus de cellules Tr1 mesurés dans les oreilles inflammées par 10⁶ événements acquis. Les résultats représentés sont tirés de groupes de 4 souris et ont été répétés avec deux clones Tr1 différents (A10.11 et A10.9).

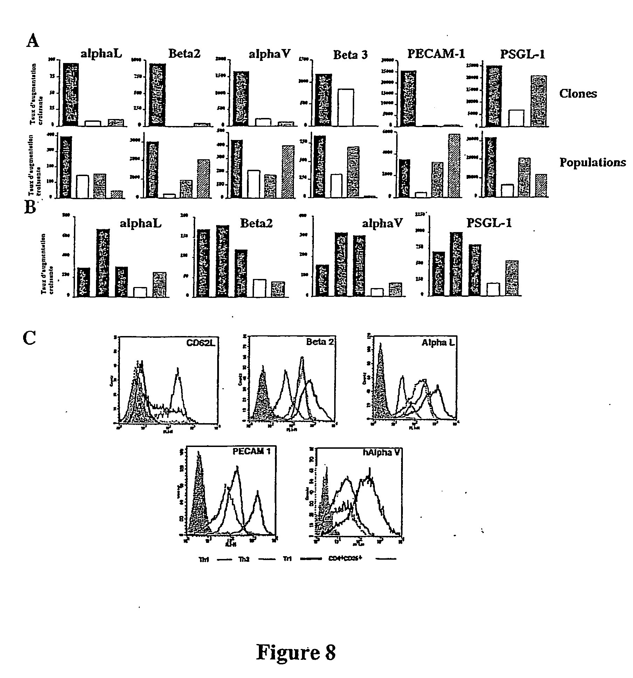

**Figure 8** **: expression des molécules d'adhésion sur les cellules Tr1.**
   **A-B)** Niveaux d'expression génétique des molécules alpha L, beta 2, alpha V, beta 3, PECAM-1 et PSGL-1 sur des clones et populations de cellules T humaines et de souris Tr1 (barres grises), Th2 (barres blanches) et Th1 (barres noires). Pour les clones de cellules T de souris, les résultats représentent une moyenne de deux des trois clones différents analysés. Pour les populations de souris, deux populations différentes ont été analysées et les résultats affichent la moyenne de deux valeurs. Pour les clones de cellules T humaines, trois clones Tr1 différents et un clone représentatif Th1 et Th2 ont été analysés. Les valeurs sont exprimées en taux d'augmentation d'expression par rapport à un contrôle négatif et représentent une expérimentation sur trois. Parmi les 45 gènes différents codant les molécules d'adhésion testés pour les cellules T humaines et de souris, seuls ceux pour lesquels la différence entre les cellules Tr1 et Th1/Th2 était très élevée sont représentés.
   **C)** Analyse FACS des chaînes de l'intégrine alphaL, beta2 et PECAM-1 sur un clone Tr1 de souris représentatif et chaîne de l'intégrine alphaV sur un clone Tr1 humain représentatif comparé aux clones Th1 et Th2 représentatifs. Une expérimentation sur deux.

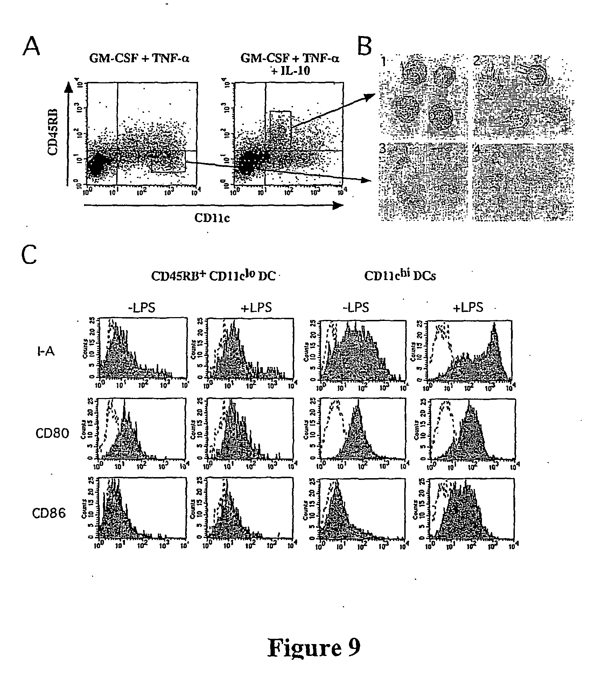

**Figure 9** **: L'IL-10 induit la différenciation des cellules dendritiques CD11c^{low}CD45RB⁺**
   **A)** Des cellules de moëlle osseuse ont été cultivées avec du GM-CSF et du TNF-α en présence ou en l'absence d'IL-10 (50 ou 500 ng/ml) comme indiqué pendant 6 jours, puis activées pendant 24 h avec du LPS. Les cellules dendritiques de moëlle osseuse ainsi générées ont été colorées avec un anticorps anti-CD11c biotinylé, puis avec un conjugué de streptavidine-peroxydase (PE). Les cellules ont ensuite été conjuguées à un anticorps anti-CD45RB couplé au FITC et analysées par cytométrie de flux. Les données représentent les résultats d'une étude représentative sur sept.
   **B)** Les cellules dendritiques ont été triées en fonction de l'expression de CD11c et de CD45RB, comme indiqué sur la figure 11A. Les cellules triées indiquées ont été centrifugées et colorées au May Gründwald Giemsa. 1 - cellules dendritiques CD11c^{low}CD45RB⁺ après 6 jours de différenciation, 2 - cellules dendritiques CD11c^{low}CD45RB⁺ après activation par le LPS, 3 - cellules dendritiques CD11c^{high}CD45RB⁻ après 6 jours de différenciation, 4 - cellules dendritiques CD11c^{high}CD45RB⁺ après activation par le LPS.
   **C)** Les cellules dendritiques obtenues après différenciation *in vitro* en présence de GM-CSF et de TNF-α avec ou sans IL-10, ont été triées en fonction de l'expression de CD11c et de CD45RB, comme indiqué sur la figure 11A. Les cellules triées ont été colorées avec les anticorps marqués au FITC indiqués, et réanalysées au FACS ; elles ont également été stimulées par le LPS (1 µg/ml) pendant 24 h, colorées avec les ACm indiqués et réanalysées. Les histogrammes hachurés représentent les cellules colorées avec les ACm témoins d'isotype correspondant. Les données représentent les résultats d'une expérience représentative sur trois.

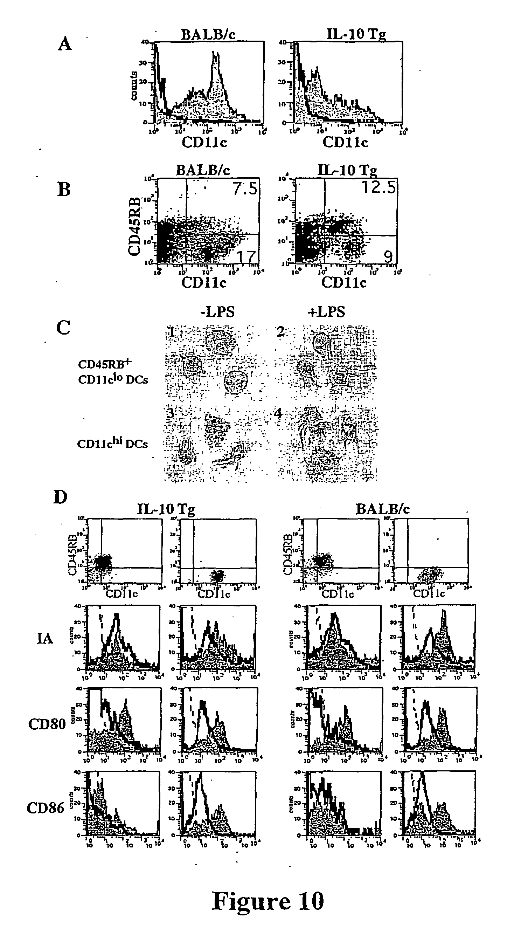

**Figure 10** **: Identification des cellules dendritiques dérivées de l'IL-10 *in vivo.***
   **A)** Des cellules dendritiques (DC) spléniques de souris BALB/c ou C57B1/6 transgéniques pour l'IL-10, IL-10 ^{-/-} et normales ont été isolées, enrichies par déplétion cellulaire avec un cocktail d'anticorps (ACm anti-B220, Gr1 et CD3) et l'expression de surface du marqueur spécifique CD11 a été analysée par cytométrie de flux.
   **B)** Des préparations de DC enrichies par déplétion cellulaire avec un cocktail d'anticorps (ACm anti-B220, Gr1 et CD3) ont été isolées de la rate de souris BALB/c ou C57B1/6 transgéniques pour l'IL-10, IL-10 ^{-/-} et normales et analysées pour l'expression de CD11c-cy5 et de CD45RB-PE afin de distinguer deux populations de DC séparées. Les pourcentages dans les différents quadrants sont indiqués. Les résultats - de plus de 10 expériences - sont typiques. Aucune différence n'a été observée entre les souris BALB/c non transgéniques et les témoins.
   **C)** Les cellules CD11c^{low} CD45RB⁺ (1) et CD11c^{high}CD45RB⁻ (3) triées isolées de la rate de souris BALB/c ont été centrifugées et colorées au May-Grünwald-Giemsa. Les cellules CD11c^{low}CD45RB⁺ (2) et CD11c^{high}CD45RB⁻ (4) triées ont également été stimulées avec du LPS (1 µg/ml) en présence de GM-CSF pendant 24 h, centrifugées et colorées au May-Grünwald-Giemsa.
   **D)** Des préparations enrichies de DC de souris BALB/c ou C57B1/6 IL-10 Tg ou témoins ont été colorées avec du CD11c-cy5 et du CD45RB-PE. Les cellules ont été triées au FACS en fonction de l'expression du CD11c^{low}WCD45RB⁺ ou du CD11c^{high}CD45RB⁻ et réanalysées comme présenté. Les cellules triées ont ensuite été colorées avec le troisième marqueur couplé au FITC et analysées par cytofluorométrie (Histogrammes vides). Les DC triées ont également été stimulées avec du LPS (1 µg/ml) en présence de GM-CSF pendant 24 h, colorées avec les ACm indiqués et réanalysées (histogrammes pleins). Les histogrammes hachurés représentent les ACm témoins d'isotype correspondant.
**Figure 11** **: Caractérisation phénotypique et sécrétion de cytokines du sous-ensemble de cellules dendritiques CD11c^{low}CD45RB⁺B220⁻**
   **A)** ) Pour comparer les cellules dendritiques CD11c^{low} CD45RB⁺ aux cellules plasmocytoïdes précédemment décrites exprimant faiblement le CD11c mais pas les marqueurs B220 et Gr1, une analyse a été conduite sur des préparations de cellules dendritiques enrichies par déplétion cellulaire avec un cocktail d'anticorps contenant (ACm anti-CD19 et CD3). Les cellules dendritiques isolées de la rate de souris BALB/c ou C57B1/6 normales ont été analysées pour l'expression de CD11c-cy5 et de B220-PE.
   **B)** Des préparations enrichies de cellules dendritiques de souris BALB/c ou C57BL/6 ont été colorées avec le CD11c-cy5 et le B220-PE et marquées avec des anticorps couplés au FITC comme indiqué. Les cellules ont été triées en fonction de l'expression de CD11c^{low}CD45RB⁺ (histogrammes pleins) ou de CD11c^{high}CD45RB⁻ (histogrammes vides, lignes droites) et la coloration pour le troisième marqueur a été analysée sur les différentes populations. Les histogrammes hachurés représentent les ACm témoins d'isotype correspondant.
   **C)** Des préparations de cellules dendritiques isolées de souris BALB/c, enrichies par un cocktail d'ACm anti-CD3, CD19, ont été colorées avec du CD11c-cy5 et du B220-PE. Les cellules ont été triées au FACS en se basant sur l'expression de CD11c^{low}B220⁺, CD11c^{high}B220⁻ ou CD 11c^{hi}. Les cellules triées ont ensuite été colorées avec le troisième marqueur couplé au FITC et analysées par cytofluorométrie (Histogrammes vides). Les cellules dendritiques triées ont également été stimulées par le CpG (xxx µg/ml) en présence de GM-CSF pendant 24 h, colorées avec les ACm indiqués et réanalysées (histogrammes pleins). Les histogrammes hachurés représentent les ACm témoins d'isotype correspondant.
   **D-E)** *Profil de cytokines des différentes sous-populations de cellules dendritiques*
   **D)** Dans une première série d'expériences, les sous-ensembles de cellules dendritiques CD11c^{high}CD45RB⁻ (histogrammes vides) et CD11c^{low}CD45RB⁺ (histogrammes pleins) ont été triés en fonction de l'expression de CD11c et CD45RB à partir de cellules dendritiques spléniques enrichies avec un cocktail d'ACm anti-CD3, B220 et Gr1. Les cellules CD11c^{high}CD45RB⁻ (histogrammes hachurés blancs) et CD11c^{low}CD45RB⁺ (histogrammes hachurés noirs) triées ont également été stimulées avec du LPS (1 µg/ml) en présence de GM-CSF pendant 24 h. Une RT-PCR quantitative en temps réel a été effectuée sur les différents échantillons et comparée à un témoin négatif pour chaque produit de transcription donné analysé. Les valeurs sont exprimées en facteurs d'augmentation de l'expression par rapport à un témoin négatif et représentent les moyennes ± ET de trois échantillons distincts.
   **E)** Une population de cellules dendritiques a également été préparée avec un cocktail contenant des ACm anti-CD3 et CD19 et triée en fonction de l'expression de CD11c et B220. Les cellules ont été stimulées avec du LPS ou du CpG pendant 24 ou 48 h et le surnageant a été analysé comme indiqué, pour mesurer la sécrétion éventuelle d'IFN-α ou d'Il-10.

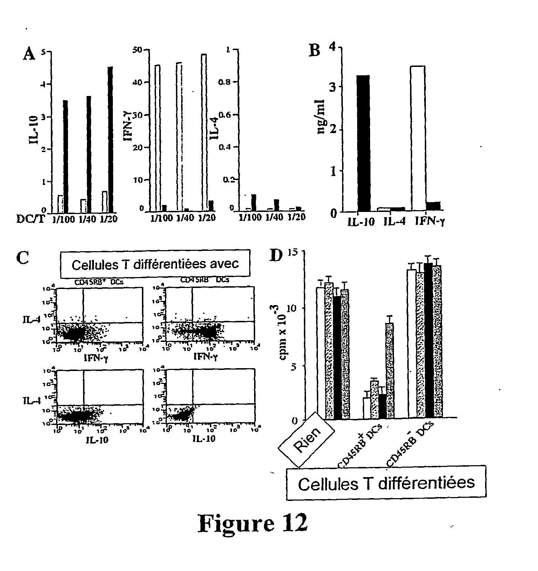

**Figure 12** **: Les cellules dendritiques CD11c^{low}CD45RB⁺ induisent la différenciation des cellules régulatrices Tr1 *in vitro.***
   **A)** Les cellules CD11c^{low}CD45RB⁺ traitent et présentent l'antigène in *vitro*
      Des cellules T DO11-10 CD4⁺ ont été stimulées avec des DC CD11c^{hi} CD45RB⁻ and CD11c^{low} CD45RB⁺ triées en présence de peptide OVA (0.6 µM) ou de protéine OVA (500 ou 250 µg/ml comme indiqué). La réponse proliférative ou la sécrétion d'IFN-γ a été analysée à J3 ou 48 h après stimulation, respectivement. Les résultats représentent la moyenne de 3 mesures et sont exprimés en ng/ml pour l'IFN-γ. Les données sont représentatives de 2 expériences distinctes avec des résultats similaires.
   **B)** *Analyse des cytokines sécrétées par des populations de cellules T CD4⁺ activées avec des cellules dendritiques triées ex vivo*
      Des cellules T transgéniques DO11-10 OVA TCR "vierges" ont été différenciées pendant 3 semaines avec des DC triées CD11c^{high}CD45RB⁻ (barres blanches) ou CD11c^{low}CD45RB⁺ (barres noires), en présence de peptide OVA. Les cellules dendritiques ont été isolées de souris BALB/c normales à différents rapports DC/T comme indiqué. Après trois semaines de culture, les cellules T ont été récoltées et stimulées avec des splénocytes BALB/c irradiés et du peptide OVA (0,3 µM). Quarante-huit heures plus tard, les cytokines ont été analysées par ELISA dans les surnageants de culture. Les résultats représentent la moyenne de 3 mesures et sont exprimés en ng/ml pour l'IL-10 et l'IFN-γ et en pg/ml pour l'IL-4. Les données sont représentatives de 5 expériences distinctes avec des résultats similaires.
   **B)** *Cytokines sécrétées par des populations de cellules T CD4⁺ différenciées avec des cellules dendritiques dérivées in vitro*
      Les cellules dendritiques ont été obtenues par culture de cellules de moëlle osseuse en présence de GM-CSF, de TNF-α et IL-10. Elles ont ensuite été triées en deux sous-ensembles: CD11c^{high}CD45RB⁻ et CD11c^{low}CD45RB⁺. Les cellules dendritiques triées ont été utilisées pour différencier des cellules T CD4+ DO11-10 "vierges", dans un rapport DC/T de 1/20, en présence de peptide OVA (0,6 µM). Au bout d'une semaine, les cellules T ont été récoltées et stimulées avec des CPA spléniques irradiées et du peptide OVA (0,3 µM). Quarante-huit heures plus tard, les cytokines ont été analysées par ELISA dans les surnageants de culture. Les résultats représentent la moyenne de 3 mesures et sont exprimés en ng/ml. Les données sont représentatives de deux expériences distinctes avec des résultats similaires.
   ***C)*** *Dosage des cytokines intracellulaires de cellules T DO11-10 "vierges" stimulées avec du peptide OVA et des DC triées CD11c^{low}CD45RB⁺ ou CD11c^{high}CD45RB⁻*
      Les cellules dendritiques CD11c^{low}CD45RB⁺ et CD11c^{high}CD45RB⁻ ont été isolées de souris BALB/c normales, triées sur FACS et mises en co-culture avec des cellules T DO11-10 "vierges" en présence de peptide OVA. Sept jours plus tard, les cellules ont été récoltées et stimulées pendant 6 h avec des ACm anti-CD3 et CD28 réticulés. De la monensine a été ajoutée pendant les 4 dernières heures de culture. Les cellules ont ensuite été fixées et colorées pour le dosage des cytokines intracellulaires par ACm spécifiques couplés au FITC ou à la PE, comme indiqué. On présente ici une expérience représentative sur trois.
   **D)** *Fonction régulatrice des cellules T différenciées avec des cellules dendritiques CD11c^{low}CD45RB⁺*
      Dans le compartiment inférieur d'un système transwell, des cellules T CD4+ purifiées isolées de souris BALB/c normales ont été stimulées avec des splénocytes BALB/c irradiés et des ACm anti-CD3 (barres blanches). Dans le compartiment supérieur, des cellules T CD4+ différenciées par une stimulation unique avec des cellules dendritiques CD11c^{low}CD45RB⁺ ou CD11c^{hi}CD45RB⁻ ont été stimulées par le peptide OVA et des splénocytes irradiés. Des expériences de co-culture ont également été conduites en présence d'ACm de souris anti-IL-10 (10 µg/ml) (barres hachurées blanches), d'ACm de souris anti-TGF-β (40 µg/ml) (barres noires) ou des deux (barres grises). Trois jours plus tard, le panier a été retiré et la réponse proliférative des cellules T CD4+ spectatrices a été mesurée après un pulse avec 0,5 µCi de ³H-thymidine pendant les 12 dernières heures de la culture de 72 h. Les résultats représentent les moyennes ± ET de trois mesures d'une expérience représentative sur trois.
   **E)** *La sécrétion d'IL-10 n'est pas requise pour la différenciation des cellules Tr1 par les cellules dendritiques CD11c^{low}CD45RB⁺*
      Des cellules T DO11-10 CD4+ "vierges" ont été différenciées pendant une semaine avec des cellules dendritiques CD11c^{low}CD45RB⁺ en présence ou en l'absence de cellules T anti-IL10R. Au bout d'une semaine, les cellules ont été récoltées et stimulées avec des splénocytes BALB/c irradiés et du peptide OVA (0,3 µM). Quarante-huit heures plus tard, les cytokines ont été analysées par ELISA dans les surnageants de culture. Les résultats représentent la moyenne de 3 mesures et sont exprimés en ng/ml pour l'IL-10 et l'IFN-γ et en pg/ml pour l'IL-4. Les données sont représentatives de 5 expériences distinctes avec des résultats similaires. De même, des cellules T CD4+ "vierges" isolées de souris C57B1/6 ont été différenciées avec un ACm anti-CD3 soluble et des cellules dendritiques CD11c^{low}CD45RB⁺ triées isolées de souris IL-10^{-/-} ou C57B1/6. Au bout d'une semaine, les cellules ont été récoltées et stimulées avec des splénocytes C57Bl/6 irradiés et un ACm anti-CD3 soluble (10 µg/ml). Quarante-huit heures plus tard, les cytokines ont été analysées par ELISA dans les surnageants de culture. Les résultats représentent la moyenne de 3 mesures et sont exprimés en ng/ml pour l'IL-10 et l'IFN-γ et en pg/ml pour l'IL-4.

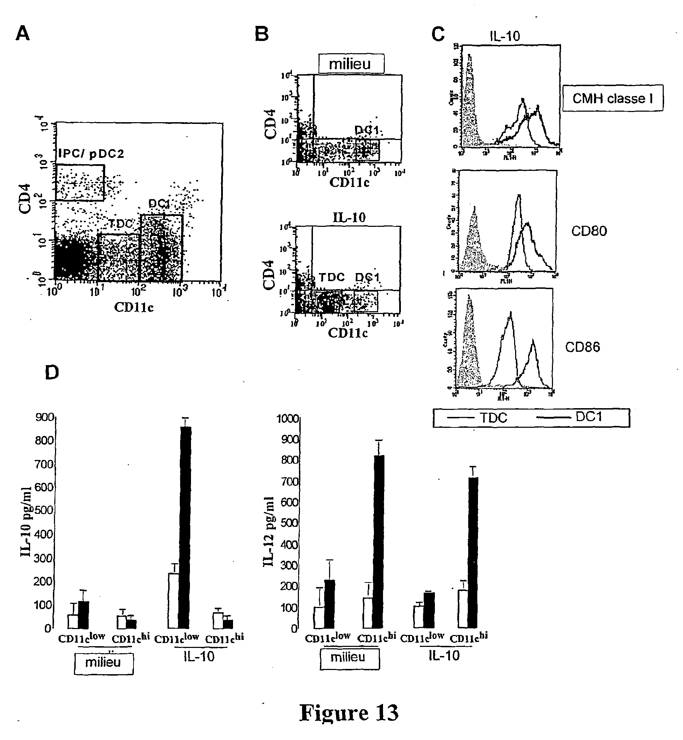

**Figure 13** **: Analyse de cellules dendritiques humaines tolérogènes (TDC)**
   **A)** on a supprimé les cellules T et B de cellules mononucléaires périphériques sanguines (PBMC) avec des anticorps et des billes magnétiques et la population restante a été analysée pour l'expression de CD11c et CD4 par cytométrie. Les carrés représentent les deux populations définies comme DC1 et DC2 par « Siegal et al, 1999, Science, 281, 1835-7 ». Un carré montre la population de DC tolérogènes (TDC).
   **B)** des cellules progénitrices CD34+ ont été différentiées *in vitro* avec du GM-CSF (800 U/ml) et de l'IL-4 (1000 U/ml) en présence ou en absence d'IL-10 (200 u/ml) comme indiqué dans l'exemple 8. on a entouré les populations de TDC et DC1.
   **C)** Les cellules TDC et DC1 ont été riées par FACS selon l'expression de CD11c et analysées par cytofluorométrie pour l'expression de HLA-DR, CD80 et CD86. Les résultats montrent que les TDC expriment de niveaux de ces molécules plus bas que les cellules DC1.
   **D)** Les cellules TDC et DC1 ont été triées par FACS selon l'expression de CD11c et stimulées par le LPS pendant 24 h. Les surnageants ont alors été analysés par ELISA pour la présence d'IL-10 et d'IL-12.

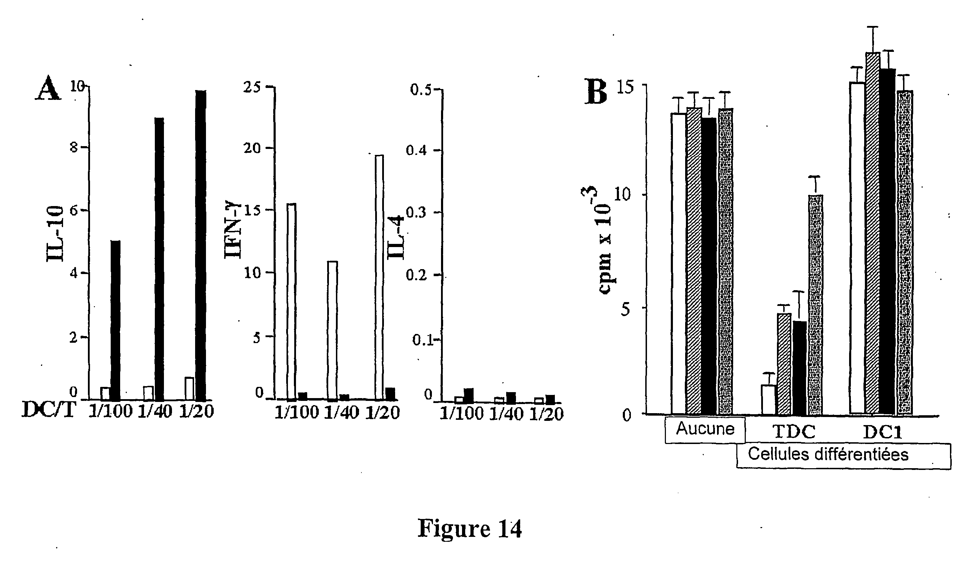

**Figure 14** **: TDC induisent la differentiation de cellules Tr1 sécrétant de haut niveaux d'IL-10**
   **A)** Analyse des cytokines secretes par les populations de cellules T CD4+ activées avec les cellules dendritiques triées.
      Les cellules T CD4+ humaines ont été stimulées pendant trois semaines avec des cellules triées allogéniques DC1 (colonnes blanches) ou TDC (colonnes noires). Les DCs ont été isolées à partir de cultures *in vitro* en présence (TDC) ou en absence (DC1) d'IL-10 et utilisées à différents ratios DC/T comme indiqué. Après la culture pendant trois semaines, les cellules T ont été récoltées et stimulées avec des PBMC irradiés, et un anticorps monoclonal anti -CD3 (10 µg/ml). Après 48 heures, les cytokines dans les surnageants de culture ont été analysées par ELISA. Les résultats représentent les moyennes de trois déterminations ey sont exprimés en ng/ml.
   **B)** Fonction régulatrice des cellules T différentiées avec les TDC.

   Dans le compartiment inférieur d'un système transwell des cellules humaines T CD4+ purifiées ont été stimulées par des PBMC irradiés et un anticoprs monoclonal anti-CD3 (colonnes blanches). Dans le compartiment supérieur, des cellules T CD4+ différentiées avec des cellules TDC ou DC1 allogéniques comme indiqué ont été stimulées avec des PBMC allogéniques irradiés. Les expériences de coculture ont aussi été mise en oeuvre en présence d'anticorps monoclonal de souris bloquant anti-IL-10 (10 µg/ml) (colonnes hachées), anticorps monoclonal anti-TGF-β (40 µg/ml) (colonnes noires) ou des deux (colonnes grises). Après trois jours, on a récupéré le récipient et la réponse proliférative des cellules T CD4+ a été mesurée par ajout de 0,5 µCi de ³H-thymidine pendant les 12 dernières heures de la culture de 72 h.
**Figure 15** **: Définition des CD3+CD4+CD18bright chez l'homme**
   Les cellules CD3+CD4+CD18bright se définissent par l'expression des antigènes CD3 et CD4 à la surface cellulaire et une intensité de fluorescence du marquage CD 18 égale à l'intensité de fluorescence du marquage CD 18 retrouvée sur les cellules monocytaires.

### EXEMPLES

### Exemple 1 : Matériel et méthodes.

### MATERIEL ET METHODES POUR LES EXEMPLES 2 ET 3

### Souris :

Les souris BALB/c et C.B-17 *scid* ont été obtenues du CERJ (Le Genest Saint Isle, France). Les souris homozygotes DO11-10 proviennent d'un don de la part du Dr. S.D. Hurst (DNAX Research Institute, Palo Alto, CA). Toutes les souris sont des femelles âgées de 4 à 8-semaines au début de chaque expérience.

### Anticorps, milieux et reactifs :

Le milieu utilisé pour les cultures de cellules T est du milieu Yssel conplémenté par 10% de SVF (Roche, Meylan, France) et 2 x 10⁻⁵ M b 2 mercaptoethanol (Invitrogen). L'IL-10 et l'IL-4 recombinant de souris proviennent d'un don du Dr R.L. Coffman, DNAX Research Institute, Palo Alto, CA. L'IFN-γ et l'IL-12 recombinante de souris viennent de R&D Systems. Les anticorps purifiés anti-IL-4 (11B11), anti-IL-10 (2A5), anti-IFN-γ (XGM1.2) and biotinylés anti-IL-4 (24G2), anti-IL-10 (SXC1) et anti-IFN-γ (R4-6A2) (Pharmingen Becton Dickinson) ont été utilisés pour les tests ELISA. Les anticorps monoclonaux suivants ont été utilisés pour la purification et phenotypage des cellules de souris: anti-I-Ad (AMS-32.1), anti-CD8 (2-43), anti-CD11b (M1/70), anti-B220 (RA36B2), FITC-anti-CD45RB (16A), PC5-anti-CD4 (H129-19), PE-anti-CD18 (C71/16), FITC-anti-CD49b (Ha1/29), FITC-anti-clonotype Kj1-26 et des contrôles isotypiques couplés aux FITC- ou PE (BD-Pharmingen, Le Pont de Claix, France). Les anticorps dirigés contre les molécules de surface humaines sont: FITC- ou PC5- anti-CD3 (UCHT-1) (Caltag), PC5- ou APC- anti-CD4 (RPA-T4), PE- anti-CD18, et FITC- anti-CD49b (AK-7) (BD-Pharmingen). Le peptide OVA₃₂₃₋₃₃₉, l'Ovalbumine et l'Oxazolone viennent de Sigma (Saint Quentin Fallavier, France). Le lipopeptide OVA₃₂₃₋₃₃₉ vient de Bachem (Voisin-le-Bretonneux, France).

### Cytométrie de flux et purification cellulaire :

Les cellules CD4+ de souris ont été purifiées comme déja décrit (Groux et al, Nature,1997). Les splénocytes ont été déplétés en cellules B220⁺, Mac-1⁺, I-Ad⁺, et CD8⁺ par sélection négatives sur billes magnétiques adsorbées par des anticorps de mouton anti-rat Ig (Dynabeads, Dynal Biotech, Oslo, NO). Les cellules restantes ont été marquées par les anticorps FITC- anti-CD49b, PE- anti-CD18 et PC5- anti-CD4 et separées en CD4⁺CD18^{bright} CD49b⁺ et CD4⁺CD18^{int} CD49b- par tri cellulaire sur un FACStar SE (Becton Dickinson, France). Les cellules humaines CD4⁺CD18^{bright}CD49b⁺ et CD4⁺ CD18^{int}CD49b⁻ du sang périphériques ont été triées après sépération des cellules mononucléées par centrifugation en gradient de Ficoll, sélection positive des cellules CD4⁺ sur billes magnétiques anti-CD4 (Dynal Biotech) et marquage des cellules CD4+ avec les anticorps PC5- anti-CD3, PE-anti-CD18 and FITC- anti-CD49b. Selon ce protocole, les populations cellulaires sont pures à plus de 98%.

### ELISA :

Des Tests ELISAs ont été utilisés pour mesurer l'IL-4, l'IL-10, et l'IFN-γ humaine et de souris. Les concentrations en cytokines on été mesurées sur des surnageants de cellules CD4+CD18brightCD49b+ de Balb/c ou humaines (2.10⁵/puits) activées 48h avec un anticorps anti-CD3 adsorbé (10µg/ml) et un anticorps anti-CD28 soluble (1 µg/ml).

### Induction de la colite inflammatoire chronique :

La colite inflammatoire a été induite par l'injection intraperitonéale à des souris SCID C.B-17 de 2.10⁵ cellules T CD4⁺CD45RB^{hi} dans 100 µl de PBS. Un groupe de souris a été traité par 1.10⁵ cellules T CD4+CD18brightCD49b+ provenant de souris DO11-10. Ces souris ont été nourries par de l'ovalbumine dans l'eau de boisson. 5 semaines après le traitement, les souris ont été sacrifiées et la proportion de cellules TCD4+ dans le colon analysée par cytométrie de flux, après digestion de la muqueuse du colon par collagénase/Dispase.

### Réaction d'hypersensibilité retardée :

La réaction d'hypersensibilité retardée à l'oxazolone a été induite par sensibilisation des souris au jour 0 par apllication de 25 ml d' Oxazolone à 20 mg/ml sur la peau de l'abdomen. La réaction d'hypersensibilité retardée a été ensuite révélée au Jour 5, par 4 µl d'Oxazolone à 4 mg/ml apliquée sur chacune des faces d'une oreille. 4 heures après, 2.10⁵ cellules T CD4⁺CD18^{bright}CD49b⁺ ont été injectées par voie intraveineuse aux souris. 20 µl d'une solution de lipopetide OVA₃₂₃₋₃₃₉ à 500 µM diluée dans l'huile d'olive.a été apliquée aux jours 4, 5 et 6 sur l'oreille inflammatoire. L'épaississement de l'oreille a été mesuré une fois par jour pendant 6 jours.

### Cultures cellulaires en Transwell :

5.10⁵ cellules T CD4+ on été déposées dans un puits de culture en présence de 4.10⁵ splénocytes irradiés et un anticorps anti-CD3 soluble (10 µg/ml). 2.10⁵ cellules T CD4⁺CD18^{bright}CD49b⁺ ou CD4⁺CD18^{int} ont été activées de la même manière et cultivées dans un insert Transwell (pores de 0,4µm) (Dutscher, Brumath, FR) déposés sur le puits contenant les cellules CD4+. L'incorporation de thymidine tritiée par les cellules CD4+ cibles a été mesurée après 72 heures de culture.

### MATERIEL ET METHODES POUR L'EXEMPLE 4

### Souris

Des souris scid BALB/c et C.B-17 exemptes de pathogène spécifique ont été obtenues à partir de CERJ (Le Genest Saint Isle, France). Les souris ont été maintenues dans nos installations pour animaux. Les souris scid C.B-17 ont été hébergées dans des micro-isolateurs avec air filtré stérile (Rec Biozone, Margate, UK). Les souris femelles utilisées étaient âgées de 8 à 12 semaines.

### Anticorps

Les anticorps monoclonaux suivants ont été utilisés pour la purification de cellules de souris : AMS-32.1, anti-Iad, 2-43, anti-souris CD8, MI/70, anti-souris CD11b, RA36B2, anti-souris B220, 16A conjugué FITC, anti-souris CD45RB, GK1.5 conjugué PE ou TC, anti-souris CD4, KJ-1,26 mAb biotynilé ou FITC révélé par la streptavidine marquée PE, des anticorps de contrôles d'isotypes conjugués FITC et PE (BD-Pharmingen, Le Pont de Claix, France).

Les anticorps dirigés contre les molécules d'adhésion (BD-Pharmingen) étaient les suivantes l'intégrine alphaV (23C6), l'intégrine alphaL (M17/4), l'intégrine beta2 (Game-46), l'intégrine beta1 (HMβ1-1) et la PECAM-1 (MEC 13.3). Pour l'analyse d'expression de la PECAM-1, l'intégrine alphaL et l'intégrine beta2, la coloration a été révélée par des immunoglobines anti-rat lapin couplés FITC (Dako, Trappes, France).

### Purification des cellules et cytométrie

Des sous-ensembles de cellules T CD4⁺ ont été purifiés à partir de rates de souris comme décrit précédemment (Groux and all, 1997). En bref, les cellules ont été réduites de cellules B220⁺, Mac-1⁺, I-Ad⁺ et CD8⁺ par une sélection négative utilisant les Dynabeads recouverts d'anti-rat chez le mouton (Dynal, Oslo, Norvège). Les cellules restantes ont été marquées par des anti-CD45RB (25 µg/ml) conjugués avec des FITC et des anti-CD4 (10 µg/ml) conjugués avec des PE et séparées en fractions de CD4⁺CD45RB^{hi} et CD4⁺CD45RB^{lo} en tri de deux couleurs sur le FACStar SE (Becton Dickinson, France). Toutes les populations étaient pures à plus de 98% après nouvelle analyse. Pour l'analyse des cellules T infiltrant le côlon, des portions de côlons ont été stimulées et digérées par collagénase (Life Technologies, Cergy Pontoise, France) pendant 2h à 37°C. Des cellules ont été réduites de cellules B220⁺, Mac-1⁺, I-Ad⁺ et CD8⁺ par une sélection négative utilisant les Dynabeads recouverts d'anti-rat chez le mouton. Les cellules T ont ensuite été analysées par cytométrie avec un Tricolor anti-CD4 et KJ-1,26 mAb marqué par FITC. Pour l'analyse des cellules de ganglions lymphatiques mésentériques (MLN), des ganglions lymphatiques mésentériques combinés ont été stimulés et les cellules analysées par cytométrie avec un anti-CD4 mAb marqué par des FITC et un KJ-1,26 mAb biotynilé révélé par la streptavidine PE. Pour les études de migration, après digestion par collagénase, les cellules CD4⁺ du côlon ont été triées en utilisant de l'anti-CD4 conjugué avec du PE. Afin de récupérer les lymphocytes des oreilles inflammées, les oreilles ont légèrement été immergées dans du PBS IX. Des morceaux de tissus ont ensuite été lavés une fois avec du PBS IX et digérés pendant 30 minutes à 37°C avec de la Trypsine EDTA dans la Solution saline équilibrée de Hank (toutes deux de Life Technologies). Les tissus traités à la Trypsine ont été lavés avec 1 mg/ml de dispase de collagénase pendant 1 heure à 37°C sous agitation constante. Les suspensions cellulaires ont été lavées dans du PBS IX et les surnageants contenant des lymphocytes ont été récupérés.

### Les cellules T

Les clones de cellules T de la souris ont été obtenus à partir de souris DO11-10 après différenciation in vitro comme décrit précédemment (Groux and all, 1997). Les cellules KJ-1,26⁺ CD4⁺ naïves (MEL-14^{hngnt}) ont été stimulées de manière répétée avec du peptide d'OVA 323-339 chaque semaine pendant 3 semaines, en la présence de l'IL-4 et l'anti-IL-12, l'IL-12 et l'anti-IL-4 ou l'IL-10 respectivement pour les cellules Th2, Th1 ou Tr1. Des populations différenciées de trois semaines ont été utilisées au cours de l'étude. Afin de générer des clones de cellules T, les différentes populations de cellules T ont été clonées à une cellule/puits par cytométrie (FACS vantage SE, Becton Dickinson) et stimulées par des splénocytes irradiés (4500 rad) et du peptide d'OVA. Les clones ont ensuite été dilatés et analysés pour la sécrétion de cytokine après activation avec les APCs et la peptide d'OVA. Les clones sélectionnés ont ensuite été stimulés avec des splénocytes irradiés et du peptide d'OVA toutes les 2 semaines et un peu plus dilatés avec de l'IL-2 (R&D system, Minneapolis, MN, 10 ng/ml. Les clones des cellules T ont été utilisés au moins 10 jours après la dernière stimulation.

Les différents clones de cellules T JDB humaines ont été décrits précédemment (Groux and all, 1997) et obtenus après stimulation MLR en la présence ou l'absence d'IL-10. Les autres clones de cellules T utilisés ont été isolés à partir de biopsies cutanées telles que décrites (Lecart and all, J Invest Dermatol. 2001 Aug; 117(2):318-25.).

### Les dosages de cytokine

Les tests ELISA sandwich ont été utilisés pour doser les IL-4, IL-10 et IFN-γ humains et de souris comme décrit précédemment (Groux and all, 1997).

### Induction de la maladie inflammatoire des intestins

L'IBD a été induite chez des souris, scid CB-17 avec des cellules T CD4⁺CD45RB^{hi} injectées par voie intra péritonéale dans 100 µl de contenant PBS. Le contrôle de l'inflammation a été fourni par injection de 2x10⁵ cellules T CD4⁺CD45RB^{lo} ou différentes cellules T spécifiques d'OVA comme indiqué.

### Examen au microscope des côlons

Des côlons ont été retirés de souris et fixés dans du PBS contenant 10% de formaldéhyde. 6 mm de sections avec inclusion de paraffine ont été coupées et colorées avec de l'hématokyline et de l'éosine. Les tissus ont été évalués semi-quantitativement de 0 à 5 de manière anonyme. Une cote 0 était attribuée quand aucun changement n'était observé. Les changements associés de façon typique avec les autres cotes se présentent comme suit : cote 1, infiltrats dispersés de cellules inflammatoires des muqueuses, avec ou sans hyperplasie épithéliale minimale ; cote 2, infiltrats de cellules inflammatoires bénins dispersés à diffus, s'étendant parfois dans les sous-muqueuses et associés avec les érosions, avec une hyperplasie épithéliale minimale à bénigne et une déplétion de mucine minimale à bénigne des cellules caliciformes ; cote 3, infiltrats de cellules inflammatoires bénins à modérés qui étaient parfois transmuraux, souvent associés à l'ulcération, avec une hyperplasie épithéliale et une déplétion de mucine modérées ; cote 4, infiltrats de cellules inflammatoire marqués qui étaient souvent transmuraux et associés à l'ulcération, avec un hyperplasie épithéliale et une déplétion de mucine marquées ; et cote 5, inflammation transmurale marquée avec ulcération aiguë et perte des glandes intestinales.

### Immuno-histochimie

Des portions de côlons ont été congelées dans du nitrogène liquide et stockés à -70°C. 5 µm des sections congelées ont été coupées et collées sur lames de verre. Elles ont été complètement séchées à température ambiante pendant 1 heure et fixées dans l'acétone à 4°C pendant 15 min. Des sections ont été colorées par une technique immunoenzymatique employant un système de péroxydase-avidine-biotine. En bref, des sections ont été lavées dans du PBS pendant 5 min. Puis les sections ont été saturées avec de la biotine et de l'avidine (Vector) selon les instructions du fabricant et incubées avec de la biotine KJ-1,26 ou l'isotype de contrôle. Après avoir été lavées pendant 5 min dans du PBS, les sections ont été incubées avec de la péroxydase-streptavidine. Après un dernier lavage, la péroxydase a été développée par le kit de coloration de vecteur DAB (Vectastain, Vector), qui donne une couleur marron.

### Colorations fluorescentes

Différentes colorations ont été utilisées dans cette étude à partir de Sondes Moléculaires (Molecular Probes, Eugene OR, USA). La calcéine bleue est un marqueur bleu fluorescent avec excitation à 322 nm et émission à 435 nm ; le diacétate de fluorescéine fluorescente verte est un marqueur fluorescent vert avec excitation à 522 nm et émission à 529 nm, la calcéine AM est un marqueur fluorescent vert avec excitation à 494 nin et émission à 517 nm ; la tétraméthyl rhodamine fluorescente orange est un marqueur fluorescent rouge avec excitation à 541 nm et émission à 567 nm. Les cellules T ont été marquées au milieu avec différentes sondes (1 µg/ml) pendant 30 min à 37°C dans l'obscurité et nettoyées trois fois avant utilisation.

### Etudes de flux in vitro et chambre d'écoulement

La lignée de cellules endothéliales de la veine ombilicale humaine transformée EA hy926 a été aimablement fournie par le Dr Edgell (Université de Caroline du Nord, Chapel Hill, N.C.) et mise en culture dans du DMEM (Life Technologies, Cergy Pontoise, France) additionné d'un supplément de 20% de FCS. La lignée de cellules endothéliales murine transformée SVEC4-10 a été achetée à ATCC et mise en culture avec du DMEM additionné d'un supplément de 10% de FCS. La chambre d'écoulement a été achetée chez Immuneties (Cambridge, MA). Elle a été conçue pour permettre un écoulement laminaire stabilisé entre 0,1 et 2 dyn/cm². Les cellules T à une concentration de 1 x 10⁶ dans du HBSS (Life Technologies) additionné d'un supplément de 1 mM de CaCI₂ et MgCI₂ ont été perfusées à travers la chambre sur une monocouche de cellules endothéliales employant une pompe-seringue de prélèvement (Harvard Apparatus, Boston, MA). Dans la plupart des expérimentations, les différentes lignées de cellules T ont été marquées de manière fluorescente, lavées trois fois, puis mélangées et perfusées sur la lignée de cellules endothéliales murine SVEC4-10 ou la lignée de cellules EA humaines pendant 15 min. Le médium a été perfusé pour retirer les lymphocytes qui n'adhèrent pas fermement avant quantification sur 10 champs aléatoires de 0,65 mm² avec objectif 10X. Pour des études sur l'inhibition, le recombinant soluble mVCAM-1, les chaînes d'intégrine anti-beta1 et anti-beta2 ont été utilisés à 10 µg/mml et incubés avec des cellules à +4°C, 30 minutes avant l'essai. Les chimères recombinantes mICAM-1 Fc, les chimères recombinantes mVCAM-1 Fc et les chimères recombinantes en sélectine mP Fe ont été recouvertes à 2 µg/ml (R&D systems). Pour les études sur le roulement, les cellules perfusées ont été surveillées par enregistrement vidéo et les cellules mobiles ont été comptées sur 9 champs distincts pour chaque sous-ensemble de cellules T fluorescentes.

### Tests de migration in vivo

Les lymphocytes T ont d'abord été marqués par des colorants fluorescents. Les cellules Th1, Th2 et Tr1 ont été incubées à une concentration de 2,10⁶ cellules / ml dans du PBS IX et marquées soit avec 1 µg/ml de calcéine, AM ; 2µg/ml de CMTMR orange ou 2 µg/ml de bleu de calcéine, pendant 30 minutes à 37°C. Les cellules ont ensuite été lavées deux fois dans du PBS IX (Life Technologies). Les suspensions cellulaires fluorescentes ont été mélangées. Les souris ont reçu par voie intraveineuse 100µl de PBS IX contenant 1 million de chaque population de cellules. Vingt-quatre heures après le transfert de cellules, la distribution de tissus de cellules fluorescentes a été analysée par cytométrie sur un appareil FACS SE (Becton Dickinson, Le Pont de Claix, France). Afin d'analyser de manière spécifique les cellules T injectées, l'acquisition de FACS a été effectuée sur une porte définie sur des paramètres SSC-FSC (SSC pour side light scatter ; et FSC pour forward light scatter) de cellules fluorescentes mélangées prélevées avant le transfert de cellules. Le nombre de Th1, Th2 ou Tr1 dans chaque organe a été évalué pour un total de 10⁶ cellules acquises. Pour le blocage de la LFA-1, la chimère ICAM 1Fc (R&D systems) a été utilisé à 10 µg/ml pendant 30 min à +4°C, avant l'injection de cellules.

### Sensibilité de contact à l'oxazolone

La sensibilité de contact à l'oxazolone (Sigma, L'Isle d'Abeau, France) a été exécutée en appliquant 20 µl d'une solution d'oxazolone de 50 mg/ml dans de l'huile d'olive/acétone (4:1, vol:vol) de manière épicutanée sur l'oreille droite une fois par jour pendant trois jours. L'oreille gauche n'a reçu que le véhicule. L'épaisseur de l'oreille a été surveillée chaque jour.

### Chimiotactisme in vitro

Avant la migration, les cellules Th1 et Th2 ont d'abord été marquées par la calcéine, AM et CMTMR orange comme décrit précédemment. Les cellules Tr1 sont restées non marquées. 10⁵ des cellules de chaque population dans 150 µl de RPMI chauffé, 20 mM Hepes, 1% de FCS (tous de Life Technologies) ont été appliquées dans un insert entre puits de pores de 5 µm (Corning Costar, Brumath, France). Seulement 600 µl de médium de migration ou contenant SDF-1/CXCL12 (Prepotech, Rocky Hill, NJ), Mig/CXCL9 ou SLC/CCL21 (R&D systems) ont été distribués dans la chambre inférieure, à côté de l'insert entre puits. Après 3 heures à 37°C, les cellules qui ont migré en direction de la chambre inférieure ont été récoltées et la migration différentielle de chaque population de cellules a été analysée par cytométrie.

### Test RT-PCR quantitatif en temps réel

L'ARN total a été préparé en employant du TRIZOL (Life Technologies) et tout ADN chromosomique contaminant potentiel a été digéré par la DNase 1 selon les instructions du fabricant (Gene Hunter, Nashville, TX). Puis, l'ARN a été transcrit à l'inverse en employant obligo(dT)12-18 et la transcriptase inverse de Superscript II (Life Technologies). Le PCR quantitatif en temps réel a été exécuté avec la trousse de réactifs PCR vert SYBR dans les plaques à microtitration spéciales des 96 puits optiques (Applied Biosystems, Courtaboeuf, France) dans un Système de détection de séquence ABI PRISM 5700 (Applied Biosystems), selon les instructions du fabricant. Des signaux de fluorescence ont été générés au cours de chaque cycle PCR par incorporation directe de l'ADN double brin vert SYBR afin de fournir une information PCR quantitative en temps réel. Les amorces (MWG Biotech, Ebersbert, Allemagne) ont été conçues pour mesurer les jonctions exon-intron afin de prévenir l'amplification d'ADN génomique et pour obtenir des amplimères entre 100 et 150 bp afin d'accroître l'efficacité de l'amplification par PCR. Toutes les amorces ont été utilisées dans des conditions qui ont prévenu la formation de dimères et l'exactitude des produits amplifiés a été testée par électrophorèse et les cartes de digestion par enzymes de restriction. Tous les ADNc ont été titrés sur la valeur de l'expression moyenne de 4 gènes domestiques différents. Les conditions PCR étaient de 10 min à 94°C, 40 cycles de 30 s à 94°C, 30 s à 60°C et 30 s à 72°C pour chaque amplification dans un volume final de 20 µl. L'expression de gènes cibles a été mesurée après normalisation des concentrations d'ARN avec 4 gènes domestiques différents et les valeurs sont exprimées en expression accrue au-dessus d'un contrôle négatif.

### Exemple 2 : Caractérisation des cellules T CD4⁺CD18^{bright}CD49b⁺ T comme cellules Tr1 chez la souris.

### Analyse FACS des cellules T CD4⁺CD18^{bright}CD49b⁺ à partir d'un échantillon biologique de splénocytes murins.

Des splénocytes de souris BALB/c ont été marqués avec des anticorps anti-CD3 FITC-conjugués, des anticorps anti-CD4 PC5-conjugués et des anticorps anti-CD18 PE-conjugués. L'analyse FACS des cellules T CD3⁺CD4⁺ montre la présence d'une population capable de surexprimer l'antigène CD18 et qui représente 14% de la totalité des cellules T CD4⁺ **(****Fig.1A****).** Des splénocytes de souris BALB/c ont été marqués avec des anticorps anti-CD4 PC5-conjugués, des anticorps anti-CD18 PE-conjugués et des anticorps anti-CD49b FITC-conjugués. L'analyse FACS des cellules T CD4⁺ montre que 35% des cellules T CD18^{bright}CD4⁺ chez la souris surexpriment également l'antigène CD49b **(****Fig.1B****).**

### Profil de production des cytokines par les cellules T CD4⁺CD18^{bright}CD49b⁺.

Des cellules T CD4⁺CD18^{bright} CD49b⁺ ont été triées par technique FACS après détection CD4, CD18 et CD49b des splénocytes de souris BALB/c. les cellules T CD4⁺CD18^{bright}CD49b⁺ ont ensuite été activées *in vitro* à l'aide d'anticorps marqués anti-CD3 (10 µg/ml) et d'anticorps solubles anti-CD28 (1 µg/ml). Les surnageants ont été récupérés après 48 heures de culture et un test ELISA a été effectué pour déterminer la présence d'IL-10, d'IL-4 d'IFNγ. Les résultats montrent que les cellules T CD4⁺CD18^{bright}CD49b⁺ T présentent le même profil de production de cytokines que celles décrites pour les lymphocytes T Tr1 (production importante d'IL-10, production d'IFNγ et absence de production d'IL-4) **(****Fig.1C****).**

### Etude proliférative des cellules T CD4+ par incorporation de la ³H-thymidine.

Les cellules T triées CD4⁺CD18^{bri}gh^{t}CD49b⁺ ou CD4⁺CD18^{bright}CD49b⁻ et les cellules CD4⁺ sont mises en culture en présence de splénocytes irradiés et d'anticorps soluble anti-CD3 (10 µg/ml). Les deux populations cellulaires ont été séparées par une membrane de polycarbonate (pores de 0,4 µm). Après trois jours de culture, la prolifération de la population totale de cellules T CD4⁺ a été mesurée par le biais de l'incorporation de ³H-thymidine. Ces expériences montrent que les cellules T CD4⁺CD18^{bright}CD49b⁺ sont capables d'inhiber la prolifération des cellules T CD4⁺ en attente *(bystander)* selon un mécanisme dépendant de facteurs solubles, mécanisme spécifique des cellules Tr1 **(****Fig.1D****).**

### Injection de cellules T CD4⁺CD18^{bright}CD49b⁺ chez des souris présentant une inflammation de l'oreille.

L'hypersensibilité retardée cutanée à l'haptène oxazolone a été induite chez les souris BALB/c par application épicutanée de l'haptène. Les souris traitées à l'oxazolone ont été injectées avec des cellules T triées CD4⁺CD18^{bright}CD49b⁺ à partir de souris transgéniques DO11-10 pour un TCR (« T-Cell Receptor ») anti-ovalbumine. Les souris ont ensuite été traitées ou non avec le lipopeptide ovalbumine au niveau de l'oreille inflammée afin d'activer spécifiquement les cellules injectées. Le gonflement de l'oreille a été mesuré dans le temps à partir de l'injection cellulaire et pendant quatre jours. Les résultats montrent que les cellules T CD4⁺CD18^{bright}CD49b⁺ sont capables d'inhiber *in vivo* l'inflammation cutanée uniquement lorsqu'elles sont activées avec le lipopeptide ovalbumine. Ces résultats sont en corrélation avec l'effet inhibiteur *in vivo* des clones cellulaires T Tr1 dans le même modèle pathologique **(****Fig.1E****).**

### Etude proliférative des cellules T CD4+ chez des souris atteintes de la maladie de Bowel après injection des cellules T CD4⁺CD18^{brignt}CD49b⁺.

Des souris SCID ont été injectées avec CD4⁺CD45RB^{high} pour induire la maladie inflammatoire de Bowel. Un groupe de souris a également été traité avec des cellules T triées CD4⁺CD18^{bright}CD49b⁺ à partir de souris transgéniques DO11-10 et nourries avec de l'ovalbumine. Huit jours après l'injection cellulaire, la proportion de cellules T CD4⁺ dans la muqueuse du colon a été analysée par cytométrie de flux. Alors que les souris contrôle présentent une inflammation sévère du colon et une proportion élevée de cellules T CD4⁺ à l'intérieur de l'infiltrat cellulaire, les souris traitées avec les cellules T CD4⁺CD18^{bright}CD49b⁺ sont protégées de l'inflammation et ont une petite infiltration de cellules T CD4⁺ au niveau du colon. Ces expériences montrent qu'en tant que cellules T Tr1, les cellules T CD4⁺CD18^{bright}CD49b⁺ sont capables de protéger les souris de la maladie inflammatoire de Bowel **(Fig.1F).**

### Exemple 3 : Caractérisation des cellules Tr1 CD4⁺CD18^{bright}CD49b⁺ T chez l'homme.

### Analyse FACS des cellules T CD4⁺CD18^{bright}CD49b⁺ à partir d'un échantillon biologique de cellules PBMC.

Des cellules monnonucléaires de sang périphérique ont été séparées par centrifugation dans un gradient de Ficoll et ont été marquées avec des anticorps anti-CD4 APC-conjugués, des anticorps anti-CD3 PC5-conjugués, des anticorps anti-CD18 PE-conjugués et des anticorps anti-CD49b FITC-conjugués. L'analyse FACS des cellules T CD3⁺CD4⁺ montre la présence d'une population capable de surexprimer l'antigène. CD18 et représentant 20% des cellules T CD4⁺CD3⁺ dans le sang humain (**Fig.2A**). L'analyse FACS des cellules T CD3⁺CD4⁺ montre que les cellules T CD18^{bright} CD4⁺ dans le sang humain surexpriment également l'antigène CD49b (Fig.2B).

### Profil de production des cytokines par les cellules T CD4⁺CD18^{bright}CD49b⁺.

Les cellules T humaines CD4⁺CD18^{bight} ont été séparées par tri cellulaire selon la technique FACS après marquage CD4 et CD18 des cellules mononucléaires sanguines. Les cellules T CD4⁺CD18^{bright} ont ensuite été activées *in vitro* avec l'anticorps marqué anti-CD3 (10 µg/ml) et des anticorps solubles anti-CD28 (1 µg/ml). Les surnageants ont été récupérés après 48 heures de culture et un test ELISA a été effectué pour détecter la présence d'IL-10, d'IL-4 et d'IFN-γ. Les résultats montrent que les cellules T humaines CD4⁺CD18^{bright}, comme leurs homologues murins, possèdent le même profil de production de cytokine que celui décrit pour les lymphocytes T Tr1 (production importante d'IL-10, production d'IFNγ et absence de production d'IL-4) (**Fig.2C**).

### Analyse FACS comparative des cellules T CD4⁺CD18^{bright}CD49b⁺ présentes chez des sujets sains et des patients atteints de la maladie de Crohn.

La proportion de cellules T CD4⁺CD18^{bright} CD49b⁺ dans le sang a été comparée entre des sujets sains et des patients atteints de la maladie de Crohn. L'analyse des cellules T CD3⁺CD4⁺ montre, par rapport à un sujet représentatif de chaque groupe, que la proportion de cellules T CD4⁺CD18^{bright} CD49b⁺ est diminuée chez les patients atteints de la maladie de Crohn (**Fig.2D**).

### Population de cellules T CD25⁺ CD4⁺ et de cellules T CD4⁺CD18^{bright} chez des sujets sains et des patients atteints de la maladie de Crohn.

La proportion de cellules T CD4⁺CD18^{bright} chez les sujets sains et chez les patients atteints de la maladie de Crohn correspond inversement avec celle des cellules T activées CD25⁺ CD4⁺, comme le montre la cytométrie de flux. Les patients atteints de la maladie de Crohn (n=4) montrent une augmentation du nombre de cellules T sanguines CD25⁺ CD4⁺ et une diminution du nombre de cellules T CD4⁺CD18^{bright} comparé aux sujets sains (n=5) (**Fig.2E**).

### Exemple 4: La surexpression d'un jeu de molécules d'adhésion sur les lymphocytes Tr1 régulateurs permet leur migration spécifique vers les tissus inflammés

Il existe maintenant une preuve incontestable d'une sous-population de cellules T CD4⁺ régulatrices qui, in vivo, module des réponses immunopathologiques nocives. Ces cellules ont des avantages thérapeutiques potentiels pour traiter des maladies auto-immunes mais on ignore encore un grand nombre des principaux aspects de leurs mécanismes régulateurs, particulièrement en ce qui concerne leur comportement migratoire, leur site d'action exact et les voies moléculaires impliquées dans ces mécanismes. Dans deux modèles différents d'inflammation, les schémas de migration des cellules Tr1 ont été comparé par rapport à ceux des cellules effectrices Th1 et Th2 obtenues après différenciation in vitro. Il a été démontré que les cellules Tr1 migrent de préférence vers des tissus inflammés et très peu vers des organes immuns secondaires, contrairement aux cellules Th1 et Th2. L'analyse de tous les récepteurs de chimiokines connus n'a pas révélé d'expression spécifique qui pourrait expliquer la migration spécifique des cellules Tr1. Cependant, dans des conditions de flux, les cellules Tr1 humaines ou de souris ont un arrêt accru sur l'endothélium vasculaire inflammatoire. L'analyse des molécules d'adhésion a révélé un dessin moléculaire spécifique sur des cellules Tr1 avec fonction et expression régulées à la hausse de PSGL-1, LFA-1, alphaV/beta3 et PECAM-1. Ces résultats indiquent que les cellules Tr1 représentent un sous-ensemble de cellules T régulatrices spécialisées dans le contrôle des sites inflammatoires.

### Les cellules Tr1 migrent de préférence vers le côlon inflammé.

Au cours d'expérimentations précédentes, les inventeurs ont montré que les cellules Tr1 pouvaient prévenir l'inflammation dans un modèle de maladie inflammatoire des intestins (IBD) provoquée de manière expérimentale chez des souris scid (Groux and all, 1997 ; Groux and all, Immunol Today. 1999 Oct;20(10):442-5). Afin de déterminer le site d'action des cellules Tr1 in vivo au cours du contrôle de l'1BD, des souris scid CB-17 ont été rétablies avec des cellules pathogènes T⁺ CD4⁺CD45RB^{hi} pour provoquer une IBD. Quatre semaines après le transfert, elles ont subi des injections par intraveineuse de différentes cellules spécifiques Th1, Th2 et Tr1 d'OVA (2 x 10⁶ cellules) et ont été nourries avec de l'OVA dans leur eau de boisson, une situation au cours de laquelle on observe une inhibition complète de la colite avec les cellules Tr1. Une semaine plus tard, les souris ont été sacrifiées et la présence de cellules KJ-1,26⁺ (cellules T spécifiques d'OVA) a été analysée dans leur côlon, leur rate et leurs ganglions lymphatiques mésentériques. De nombreuses cellules KJ-1,26⁺ ont été observées dans les côlons inflammés des souris traitées avec les cellules Tr1 (**Fig. 3A**). Par contraste, chez des souris traitées avec des cellules Th1 et Th2 spécifiques d'OVA, il a été détecté moins de cellules T CD4⁺ KJ-1,26⁺ parmi les cellules qui s'infiltraient dans le côlon. L'analyse des ganglions lymphatiques mésentériques drainants et des rates (non représenté) a révélé une accumulation de cellules Th1 ou Th2 alors que seulement quelques cellules Tr1 ont été observées (**Fig.3A**). Des résultats similaires ont été observés lorsque les différentes cellules T ont été injectées au jour 0 en même temps que des cellules T pathogènes CD4⁺ CD45RB^{hi} T (non représenté). Les inventeurs et d'autres (Groux and all, 1997 ; Barrat and all, J Exp Med 2002 Mar 4;195(5):603-16) ont montré dans deux différents modèles d'inflammation que l'antigène spécifique (ovalbumine) doit être injecté localement (par voie orale) dans le modèle IBD (Groux and all, 1997) ou par voie intracrânienne dans le modèle EAE (Barrat and all, 2002) pour introduire l'effet protecteur des cellules Tr1, et que l'injection systémique de l'antigène (injection iv ou ip) était inefficace. Ces résultats ont suggéré que l'activation locale de cellules Tr1 était nécessaire. Les inventeurs ont donc effectué des analyses pour voir si la présence de l'antigène sur le site de l'inflammation était également requise pour la migration des cellules Tr1 dans le côlon inflammé. Des souris scid CB17 ont reçu des injections de cellules T CD4⁺ CD45RB^{hi} et des cellules Tr1 spécifiques d'OVA simultanément (**Fig. 3C**) ou quatre semaines après reconstitution (**Fig. 3B**), en la présence ou l'absence d'OVA. L'analyse des cellules Tr1 KJ1-26⁺ injectées par immunochimie (**Fig. 3C**) ou par cytométrie en flux (**Fig. 3B**) a montré que les cellules Tr1 migrent de manière spécifique vers le côlon inflammé même en l'absence de leur antigène spécifique. Toutefois, l'activation de l'antigène spécifique des cellules Tr1 a été nécessaire pour déclencher leur fonction régulatrice comme illustrée par l'infiltration de leucocytes et l'inflammation observée chez les souris qui n'ont pas été traitées avec de l'ovalbumine, en dépit de la présence de cellules Tr1 infiltrées (**Fig. 3C**).

Etant donné que la migration des cellules Tr1 ne dépendait pas de la présence de leur antigène spécifique, les inventeurs ont pu comparer leur migration à des cellules purifiées ex-vivo. Tout d'abord, afin de s'assurer que la migration accrue des cellules Tr1 par rapport aux cellules Th1 vers le côlon inflammé n'était pas dû à une migration relativement pauvre de la population de cellules Th1 différenciées in vitro, les inventeurs ont comparé la migration des cellules Tr1 à l'infiltrat cellulaire total (80% des polynucléaires neutrophiles) ou des cellules T CD4⁺ purifiées, isolés à partir du côlon inflammé des souris scid reconstituées avec des cellules T CD4⁺ CD45RB^{hi} (**Fig. 4A**)**.** Les différentes populations de cellules ont été marquées avec différentes sondes fluorescentes et co-injectées par voie intraveineuse chez des souris scid reconstituées avec des cellules T CD4⁺ CD45RB^{hi} de quatre semaines. Vingt-quatre heures après l'injection, la migration des cellules fluorescentes vers le côlon inflammé a été analysée par cytométrie en flux. La migration accrue des cellules Tr1 vers les tissus inflammés a été confirmée car leur migration vers les côlon inflammé était trois fois supérieure à la migration de la population totale de leucocytes et plus de 30 fois supérieure à la migration de cellules T CD4⁺ purifiées isolées à partir du côlon inflammé (**Fig. 4A**).

### La migration spécifique de cellules Tr1 ne se limite pas à l'intestin inflammé.

L'injection de cellules Tr1, Th1 et Th2 chez des souris scid non traitées ou chez des souris normales BALB/c n'a pas révélé de tropisme spécifique des cellules Tr1 vers les tissus intestinaux normaux (données non représentées). C'est pourquoi, afin d'analyser si la migration spécifique de cellules Tr1 en l'absence d'antigène était limitée au tissu intestinal inflammé ou si elle était spécifique aux signaux inflammatoires présents dans tout tissu donné, les inventeurs ont constitué un modèle d'inflammation de la peau en appliquant de l'oxazolone d'haptène sur la peau de l'oreille (**Fig. 5A**). L'inflammation était caractérisée par une infiltration de leucocytes (**Fig. 5B**) avec à la fois des cellules T CD4⁺ CD8⁺ (non représentée).

Afin d'analyser la migration des cellules T dans la peau inflammée, les cellules Th1, Th2 et Tr1 ont été marquées par de la calcéine fluorescente et injectées 5 jours après le traitement à l'oxazolone. De manière similaire au modèle IBD, les cellules Tr1 ont affiché une migration accrue vers l'oreille inflammée (**Fig. 5C et D**) par comparaison aux cellules Th1 et Th2 qui ont migré de préférence vers les ganglions lymphatiques drainants et la rate (**Fig. 5C et D**). Pour chaque population, le taux de cellules dans un tissu donné par rapport au nombre total de cellules récupérées peut être pris comme une mesure de la capacité relative de ces cellules à se diriger vers ces organes. Ces taux diffèrent de manière significative entre les quatre populations analysées : 90% pour des cellules Tr1, 60% pour des cellules Th1 et 30% pour des cellules Th2 dans l'oreille inflammée, et 10% pour les cellules Tr1, 40% pour les cellules Th1 et 70% pour les cellules Th2 dans les organes lymphoïdes (**Fig. 5E**). En tout, les données montrent que les cellules Tr1 migrent vers les tissus inflammés et suggèrent que la migration spécialisée des cellules Tr1 dépend des signaux inflammatoires et n'est pas dépendant des antigènes ni spécifique aux tissus.

### Adhésion ferme des cellules Tr1 sur les cellules endothéliales vasculaires activées dans des conditions de flux

L'adhésion aux veinules inflammées est un événement précoce et essentiel dans le recrutement de leucocytes circulants en direction du site de l'inflammation. Afin d'approfondir l'analyse des mécanismes qui entraînent la migration accrue des cellules Tr1 vers les tissus inflammés, l'interaction entre les cellules Tr1 et Th1 et les cellules endothéliales activées TNF-α a été comparée. Afin d'analyser ce processus, les inventeurs ont réalisé des expérimentations dans une chambre d'écoulement revêtue d'une lignée cellulaire endothéliale vasculaire activée TNF-α (SVEC4-10) et l'adhésion des cellules T au marquage fluorescent été analysée par images vidéo, comme décrit précédemment (Ticchioni and all, FASEB J. 2001 Feb;15(2):341-50). En ce qui concerne les expérimentations in vivo, des colorants fluorescents ont été utilisés pour marquer les cellules Tr1 et Th1 de la souris. Les cellules T ont été mélangées en nombre égal et perfusées sur une monocouche de SVEC4-10 à un débit de 2 dyn/cm2. Les résultats ont montré que les cellules Tr1 avaient une capacité accrue à s'arrêter sur des cellules endothéliales vasculaires activées par comparaison aux cellules Th1 (**Fig. 6** **A et B**). Au cours d'expérimentation similaires, les cellules Th2 ont montré un arrêt minimal sur des cellules endothéliales activées (non représenté).

Afin de confirmer que cette adhésion ferme aux cellules endothéliales vasculaires activées est une marque des cellules Tr1, les inventeurs ont réalisé des expérimentations en chambre d'écoulement similaires sur des cellules humaines Tr1, Th1 et Th2 **(Tableau 1)**(Groux and all, 1997 ; Lecart and all, J Invest Dermatol. 2001 Aug;117(2):318-25). Les cellules humaines Th1, Th2 et Tr1 isolées à partir de différents donneurs ont été marquées par du diacétate de fluorescéine fluorescente verte et perfusées séparément sur une lignée cellulaire transformée humaine préalablement activée par du TNF-α. La figure 8C montre que les cellules humaines Tr1 affichent des niveaux supérieurs d'arrêt ferme sur des cellules endothéliales activées par comparaison aux cellules Th1 et Th2. Ce résultat suggère que l'accumulation de cellules Tr1 observée dans les tissus inflammés in vivo est dû en partie à l'adhésion accrue des cellules circulantes Tr1 à l'endothélium activé.

### Mécanisme d'adhésion des cellules Tr1.

L'événement précoce de l'adhésion a d'abord été analysé, la phase mobile. Le nombre de cellules mobiles au cours de tests en chambre d'adhésion d'écoulement a été comparé entre différentes cellules Tr1 et Th1 marquées à la calcéine fluorescente. De plus grands nombres de lymphocytes Tr1 ont roulé sur des cellules endothéliales activées par comparaison aux lymphocytes Th1 (**Fig. 7A**). Etant donné que la sélectine P est une molécule importante qui entraîne le roulement des lymphocytes T sur des veinules inflammées in vivo (Hirata and all, J Exp Med. 2000 Dec 4;192(11):1669-76), le nombre de cellules mobiles Tr1 et Th1 sur les lames recouvertes de sélectine P a été énuméré. Les lymphocytes Tr1 ont affiché des capacités de roulement supérieures sur la sélectine P par rapport aux cellules Th1 (**Fig.7A**). Le roulement accru des cellules Tr1 sur les lames recouvertes de sélectine P est en corrélation avec l'expression ARNm accrue des PSGL-1 (P-selectin glycoprotein-ligand-1) à la fois sur les cellules Tr1 humaines et de souris (**Fig. 8A et B**). Ces résultats suggèrent que la surexpression de PSGL-1 sur les cellules Tr1 joue un rôle important dans la capacité supérieure de ces cellules à migrer vers les tissus inflammés.

Les LFA-1/ICAM-1 et VLA-4/VCAM sont des médiateurs importants de l'adhésion des cellules T au cours du processus inflammatoire (Alon and all, Semin Immunol. 2002 Apr;14(2):93-104). Par conséquent, la contribution à la fois des LFA-1 et VLA-4 à l'adhésion accrue des cellules Tr1 à l'endothélium activé a été déterminée. En utilisant des tests d'adhésion de chambre d'écoulement recouverte d'ICAM-1 recombiné comme substrat, il a été observé une adhésion accrue des cellules Tr1 à l'ICAM-1 par comparaison aux cellules Th1. Ces résultats sont en corrélation avec ceux obtenus avec des cellules endothéliales activées, suggérant que l'interaction LFA-1/ICAM-1 joue un rôle prépondérant dans l'adhésion des cellules Tr1 à l'endothélium inflammé (**Fig. 7B**).

Le rôle important de la LFA-1 dans l'adhésion des cellules Tr1 a été confirmé par les expérimentations utilisant des anticorps bloquants de chaîne d'intégrine anti-beta-2 qui ont inhibé l'arrêt des cellules Tr1 sur l'endothélium activé dans les expérimentations en chambre d'écoulement (**Fig. 7C**) et par des expérimentations in vivo où la pré-incubation de cellules Tr1 avec des molécules ICAM-Fc a inhibé leur migration vers les oreilles inflammées (**Fig.7D**). Par contraste avec le rôle crucial joué par la LFA-1, l'interaction VLA-4/ICAM-1 n'apparaît pas être importante pour l'adhésion des cellules Tr1 car les cellules Tr1 ne s'arrêtent pas sur les lames recouvertes de VCAM-1 (Fig.7B). De plus, l'anticorps bloquant anti-beta1 ou la VCAM-1 soluble n'ont pas empêché l'arrêt des cellules Tr1 sur l'endothélium activé alors qu'elles ont inhibé l'adhésion des cellules Th1 (**Fig. 7C**).

### Expression de molécules d'adhésion sur des cellules Tr1

Afin d'accomplir l'étude sur les mécanismes qui expliquent la migration sélective des cellules Tr1 *in vivo,* une analyse quantitative complète des molécules d'adhésion les plus connues sur plusieurs cellules Tr1, Th1 et Th2 humaines et de souris (**Fig. 8A et B**) a été réalisée. En accord avec les études fonctionnelles, il a été observé une surexpression des chaînes αL et β2 de l'intégrine (LFA-1) ARNm sur des cellules Tr1 par comparaison aux autres populations de cellules analysées. Ce résultat a été confirmé par une cytométrie en flux alors que l'expression de la membrane à la fois alphaL et beta2 a été régulée à la hausse sur les lymphocytes Tr1 par comparaison aux cellules Th1 et Th2 (**Fig. 8C**)**.** Il a également été observé des expressions de membrane et mARN supérieures de la molécule CD31/PECAM-1 sur des cellules Tr1 (**Fig. 8A et B**) par comparaison aux cellules Th1 et Th2 (**Fig. 8B**)**.** La molécule CD31/PECAM-1 joue un rôle crucial dans la diapédèse (Liao and all, J Exp Med. 1997 Apr 7;185(7):1349-57) ; une expression plus élevée de cette molécule sur des cellules Tr1 pourrait faciliter leur passage dans le compartiment sous-endothélial. Les inventeurs ont également trouvé des expressions de gènes et de membranes plus élevées des deux chaînes de l'intégrine alpha V et beta3 (**Fig. 8A****, B et C**) qui produisent une fois dimérisées un récepteur de fibronectine, de vitronectine et d'autres composants de la matrice extracellulaire (Huang and all, Oncogene. 2000 Apr 6;19(15):1915-23). Une expression de membrane plus élevée d'alpha V a également été confirmée sur des cellules Tr1 humaines (**Fig. 8B**)**.** L'expression élevée d'alphaV/beta3 sur des cellules Tr1 pourrait accélérer leur progression du compartiment sous-endothélial en direction des autres compartiments cellulaires profondément au sein de l'organe inflammé.

### Discussion

Les inventeurs ont ainsi montré que des cellules Tr1 affichent une capacité accrue et sélective à migrer vers des tissus inflammés. Cette migration spécifique de cellules T Tr1 dans les organes inflammés telle qu'observée in vivo dans deux modèles différents ne dépend pas du type de tissu, car elle est observée de la même manière dans le côlon et la peau inflammés, ni du type d'inflammation, qu'elle soit induite par des cellules T Th1 CD4⁺ dans le modèle IBD (Powrie, Immunity. 1994 Oct;1(7):553-62) ou principalement par des cellules T CD8⁺ dans le modèle irritant de la peau (Kehren and all, J Exp Med. 1999 Mar 1;189(5):779-86 ; Bour and all, Acta Derm Venereol 1995 May,75(3):218-21) et ne dépend pas de la présence de l'antigène spécifique. Ces résultats suggèrent que les signaux inflammatoires, générés dans la plupart des tissus et au cours de différents types de réponses immunes, peuvent déclencher rapidement le recrutement de cellules Tr1 et que, en corrélation avec leurs capacités migratoires, l'effet inhibiteur de ces cellules se produit localement et de manière sélective dans des tissus inflammés périphériques. Il est intéressant de noter que des études récentes ont indirectement confirmé ces observations en utilisant deux modèles différents de transplantation : les auteurs ont montré que les cellules T régulatrices sont enrichies au sein des greffons tolérées par comparaison aux tissus lymphoïdes secondaires (Sawitzki, Transplant Proc. 2001 May;33(3):2092-3 ; Graca, J Exp Med. 2002 Jun 17;195(12):1641-6). Ce tropisme sélectif vers les organes inflammés périphériques aide à expliquer l'un des paradoxes de la fonction de la cellule régulatrice T. En effet, il est désormais évident que les cellules T régulatrices fonctionnent à travers un mécanisme de suppression du voisinage induite par antigène, ce qui signifie que les cellules T régulées et régulatrices doivent être très proches mais ne reconnaissent pas nécessairement le même antigène (Groux and all, 1997). De plus, plusieurs rapports ont suggéré que les cellules T régulatrices naturelles sont dirigées contre le soi ou les antigènes communément rencontrés (Cong and all, J Immunol. 2002 Dec 1;169(11):6112-9). Par conséquent, on peut se demander comment des mécanismes de protection, qui dépendent de cellules T régulatrices, permettent encore le développement de réponses immunes bénéfiques aux pathogènes in vivo. La migration pauvre de cellules Tr1, par comparaison aux cellules effectrices T Th1 et Th2, en direction des organes lymphoïdes secondaires, où les réponses immunes de protection commencent, peut expliquer ces observations.

La migration de leucocytes du flux sanguin vers les tissus est facilitée par un processus à plusieurs phases qui, en de nombreuses occasions, implique (i) la capture et le roulement de leucocytes par sélectines, (ii) l'activation rapide des intégrines de leucocytes, (iii) l'adhésion à des ligands endothéliaux à travers des intégrines activées et (iv) la diapédèse (Kubes, Semin Immunol. 2002 Apr;14(2):65-72. Review ; Springer, Cell. 1994 Jan 28;76(2):301-14. Review ; Butcher and all, Science. 1996 Apr 5;272(5258):60-6. Review). Il a été montré que les chimiokines et les récepteurs de chimiokines apportent une importante contribution à la chimio-attraction des sous-ensembles sélectifs de leucocytes dans différents tissus différents mais également pour l'activation des intégrines sur des leucocytes afin d'induire un arrêt ferme sur les cellules endothéliales activées (Constantin and all, Immunity. 2000 Dec;13(6):759-69; Campbell, Science. 1998 Jan 16;279(5349):381-4.). Toutefois, l'analyse de l'expression des récepteurs de chimiokines sur les cellules Tr1, Th1 et Th2 révèle une régulation à la baisse surprenante dans l'expression de tous les récepteurs de chimiokine connus sur les cellules Tr1, par comparaison aux cellules Th1 et Th2. Cette régulation à la baisse a été confirmée par une migration décroissante en réponse aux chimiokines impliquées dans l'inflammation (MIG) ou dans l'adressage vers les organes lymphoïdes (SLC et SDF-1) (Baggiolini, 1998). Ces résultats suggèrent qu'aucune sur-expression d'un récepteur particulier de chimiokines ne peut expliquer la migration spécifique des cellules Tr1 en direction des sites inflammatoires. En dépit de ce manque de sur-expression des récepteurs de chimiokines, les cellules Tr1 à la fois humaines et de souris affichent une adhésion plus élevée sur des cellules endothéliales vasculaires activées par comparaison aux cellules Th1 et Th2.

Les inventeurs ont d'abord observé que les cellules Tr1 affichaient des capacités de roulement sur des cellules endothéliales vasculaires activées et la sélectine P sous flux. Il a également été observé un meilleur arrêt ferme des cellules Tr1 sur des cellules endothéliales vasculaires activées par comparaison aux lymphocytes Th1. Au cours de la dissection des mécanismes d'adhésion accrue des cellules Tr1 sur des cellules endothéliales vasculaires activées, il a été observé que l'expression de la membrane et la fonction de LFA-1 étaient régulées à la hausse sur des cellules Tr1 par comparaison à d'autres sous-ensembles de cellules T. L'importance du rôle du LFA-1 pour la migration spécifique des cellules Tr1 a été confirmée en environnement *in vivo* où les LFA-1 bloquants par les molécules ICAM-Fc ont inhibé la migration des cellules Tr1 en direction du tissu inflammé.

En fait, une analyse complète de l'expression des molécules d'adhésion sur des sous-ensembles de cellules T CD4⁺ a révélé que des cellules Tr1 sur-expriment un ensemble spécifique de molécules d'adhésion qui coopèrent pour induire leur migration vers les tissus abîmés, PSGL-1, LFA-1, alphaV/beta3 et PECAM-1. En effet, la sur-expression de PSGL-1 accroît la capacité de roulement des cellules Tr1. Il a été montré que le LFA-1 est impliqué dans les étapes d'arrêt et d'extravasation. La PECAM-1 est également une molécule hautement impliquée dans l'étape d'extravasation et enfin, l'alphaV/beta3 induit la migration des cellules au sein des tissus à travers la matrice extracellulaire (Liao and all, 1997 ; Huang and all, 2000). Il est intéressant de noter que de manière controversée par rapport à la sur-expression de plusieurs molécules d'adhésion, les cellules Tr1 ont une capacité décroissante à adhérer à la VCAM-1 par comparaison aux cellules Th1. Bien que l'expression de la VCAM-1 soit induite dans des sites inflammatoires, il a également été rapporté que la VCAM-1 est une molécule centrale dans la génération des réponses humorales à travers les interactions des cellules T-B. les inventeurs ont par conséquent fait l'hypothèse que l'absence de la fonction VLA-4 sur les lymphocytes Tr1 peut assurer que les cellules régulatrices T soient exclues des compartiments des cellules B au cours de l'initiation de réponses immunes à médiation de cellules B (Leuker and all J Exp Med. 2001 Mar 19;193(6):755-68).

### Tableau 1 : profil de cytokine des clones et populations de cellules T différentes utilisés

| Espèces | Nom | Type | IL-2 (pg/ml) | IL-4 (pg/ml) | IL-10 (pg/ml) | IFN-γ (ng/ml) |
|---|---|---|---|---|---|---|
| souris | **A-10-9** | **Tr1** | <40 | <50 | 1874±217 | 65±9 |
| souris | A-10-11 | **Tr1** | <40 | <50 | 1595±184 | 42±4 |
| souris | Nice-1 | Tr1 | <40 | <50 | 1936±502 | 38±12 |
| souris | Nice-2 | Tr1 | <40 | <50 | 1273±298 | 51±9 |
| souris | N10-7 | Tr1 | <40 | <50 | 1659±432 | 37±7 |
| souris | N10-11 | Tr1 | <40 | <50 | 1804±394 | 41±5 |
| souris | N10-23 | Tr1 | <40 | <50 | 1493±276 | 13±3 |
| souris | N12-4 | Th1 | 219±42 | <50 | <75 | 73±13 |
| souris | N12-8 | Th1 | 275±31 | <50 | <75 | 97±10 |
| souris | N12-13 | Th1 | 196±54 | <50 | <75 | 84±17 |
| souris | N4-2 | Th2 | <40 | 912±81 | 305±49 | <1 |
| souris | N4-9 | Th2 | <40 | 1065±103 | 287±36 | <1 |
| souris | N4-12 | Th2 | <40 | 715±59 | 412±67 | <1 |
| | Pop.Tr1 | | <20 | 112±19 | 12865±1457 | 5±0,1 |
| | Pop.Tr1 | | <20 | 86±21 | 14945±1065 | 2,8±0,2 |
| | Pop.Th1 | | 513±106 | <40 | 11045±984 | 156±4 |
| | Pop.Th1 | | 312±95 | <40 | 16321±1203 | 124±15 |
| | Pop.Th2 | | <20 | 2321±769 | 12378±834 | <0,2 |
| | Pop.Th2 | | <20 | 998±143 | 13241±984 | <0,2 |
| humain | JDV15 | **Tr1** | <20 | <40 | 12865±1457 | 4±0,2 |
| humain | JDV308 | Tr1 | <20 | <40 | 14945±1065 | 3,1±0,3 |
| humain | BJF161 | Tr1 | <20 | <40 | 11045±984 | 2,6±0,2 |
| humain | HA-IAJ2 | Tr1 | <20 | <40 | 16321±1203 | 2,1±0,1 |
| humain | HA-IE7 | Tr1 | <20 | <40 | 12378±834 | 1,5±0,2 |
| humain | HA-2D5 | Tr1 | <20 | <40 | 13241±984 | 0,8±0,1 |
| humain | JDV305 | Th1 | 523±41 | <40 | 239±98 | 8,3±0,6 |
| humain | BJF180 | Th1 | 613±74 | <40 | 329±274 | 9,2±0,7 |
| humain | HAT203 | Th1 | 712±65 | <40 | 121±32 | 7,9±0,8 |
| humain | BJF116 | Th2 | <20 | 1234±96 | 2345±175 | <0,5 |
| humain | PUEF39 | Th2 | <20 | 863±71 | 3059±234 | <0,5 |
| humain | BJF157 | Th2 | <20 | 1457±102 | 2019±124 | <0,5 |

Les clones de cellules T et les populations de cellules T humaines et de souris sont générés comme décrits ci-dessus. Les cellules T murines ont été stimulées avec du peptide d'OVA (0,6 µM) et des splénocytes totales irradiées (2x10⁶ cellules/ml). Les cytokines ont été analysées par ELISA dans des surnageants de culture récupérées après 24h pour IL-2 et IL-4 et après 48 h pour IL-10 et IFN-γ. Les clones des cellules T humaines ont été activés avec des anticorps monoclonaux réticulés anti-CD3 (10 µg/ml) et anti-CD28 (1 µgml), les surnageants ont été récupérés après 24h pour IL-2 et IL-4 et après 48h pour IL-10 et IFN-γ. Les résultats représentent des données combinées des 3 expérimentations représentatives.

### Exemple 5 : Procédé de préparation de cellules T et de cellules dendritiques pour la caractérisation des cellules Tr1 CD4⁺CD18^{bright}CD49b⁺

### Procédures expérimentales

### Souris

Les souris BALB/cAnN ont été obtenues auprès du CERJ (Le Genest Saint Isle, France), les souris DO11-10 homozygotes ont été généreusement données par le Dr S. D. Hurst (DNAX Research Institute, Palo Alto, CA). Les souris transgéniques BALB/c IL-10 ont été obtenues en utilisant l'ADNc hIL-10 sous le contrôle du promoteur10 du CMH de classe II Ea. Tous les animaux ont été élevés dans les conditions aseptiques courantes, dans notre animalerie. Toutes les souris étaient des femelles âgées de 4 à 8 semaines au début de chaque expérience.

### Milieu, anticorps et réactifs

Le milieu utilisé pour les cultures de cellules T était le milieu Yssel (Yssel et al., 1984). Les cellules dendritiques ont été cultivées dans du RPMI 1640 supplémenté avec 10% de SVF (Roche, Meylan, France), 2 mM de L-glutamine, 1% de pyruvate de sodium, 2 x 10⁻⁵ M de β2-mercaptoéthanol (tous d'Invitrogen). Pour la purification des cellules dendritiques, les inventeurs ont utilisé du milieu HBSS sans Ca⁺⁺ et Mg⁺⁺ contenant 2 mM d'EDTA (tous d'Invitrogen) et de la collagénase D (Roche, Meylan, France).

Le GM-CSF, le TNF-α et l'IFN-γ recombinants de souris venaient de R&D Systems, Abington, R-U. L'IL-10 et l'IL-4 recombinantes de souris ont été généreusement offertes par le Dr R. L. Coffman, DNAX Research Institute, Palo Alto, CA.

La purification et la caractérisation des DC ont été effectuées au moyen d'anticorps anti-CD3 (17A2), anti-CD4 (GK1.5) anti-CD8 (53-6.7), anti-CD11b (M1/70), anti-CD11c (HL3), CD28 (37.51) anti-I-Ad (AMS-32.1), anti-CD62L (Mel-14), anti-CD80 (16-10A1), anti-CD86 (GL1), anti-B220 (RA3-6B2), CD45RB (16A), anti-Gr1 (RB6-8C5) (Tous de Pharmingen Becton Dickinson) et de DEC-205 (NLDC-145) (Serotec, R-U.).

Les dosages de cytokines ont été effectués au moyen d'anticorps anti-IL-4-PE ou -FITC (11B11), anti-IFN-γ-PE (XGM1.2), anti-IL-10-FITC (JES-16E3), anti-IL-4 (11B11) et anti-IL-10 (2A5) purifiés, anti-IFN-γ (XGM1.2) et d'anti-IL-4 (24G2), d'anti-IL-10 (SXC1), d'anti-IFN-γ (R4-6A2) biotinylés (Tous de Pharmingen Becton Dickinson).

Les anticorps anti-IgE (R35-72) et anti- IgG1 (A85-1) utilisés pour l'analyse des IgG sériques spécifiques de l'OVA étaient de Pharmingen Becton Dickinson.

Le tampon de lyse, la métrizamide, le LPS, le peptide OVA ₃₂₃₋₃₃₉, l'ovalbumine et l'alun étaient de Sigma, Saint Quentin Fallavier, France.

### Cytométrie de flux

L'analyse phénotypique des sous-ensembles de DC a été effectuée par double ou triple coloration avec des anticorps biotinylés anti-CD11c, suivis de streptavidine-Cy-Chrome, d'anti-CD45RB couplés à la PE et d'un troisième anticorps couplé au FITC. Toutes les étapes de coloration ont été conduites à 4°C dans du tampon PBS avec 0,1 % de SAB et 0,02 mM de NaN3. Après trois lavages, les cellules marquées ont été analysées sur un FACScan (Becton Dickinson).

### Coloration des cytokines intracellulaires

L'analyse des cytokines intracellulaires par cytométrie de flux a été effectuée comme décrit (Groux et al., 1997). Les cellules (10⁶/ml) ont été activées pendant 6 h avec des ACm anti-CD3 et anti-CD28 immobilisés. La monensine a été ajoutée à 10 µg/ml et, 4 h plus tard, les cellules ont été récoltées, lavées et fixées dans du formaldéhyde à 2%. Pour la coloration intracellulaire, les cellules ont été incubées avec les ACm suivants : anti-IL-4-FITC ou-PE, anti-IFN-γ-PE et anti-IL-10-FITC, ou des anticorps témoins d'isotype correspondant, tous à 5 µg/ml. Les échantillons ont été analysés sur un FACScan (Becton Dickinson).

### Cellules dendritiques dérivées de la moëlle osseuse (DC-MO)

Les DC-MO ont été générées à partir de progéniteurs médullaires, comme décrit précédemment, avec quelques modifications (Inaba et al., 1992). En bref, la moelle osseuse a été extraite par rinçage de tibias et de fémurs avant d'éliminer les globules rouges avec 0,83 % de chlorure d'ammonium. Les cellules ont été cultivées à 37°C, sur des plaques à 24 puits (Becton Dickinson) (10⁶ cellules/ml/puits), dans du milieu RPMI complet supplémenté avec 10 ng/ml de GM-CSF recombinant murin et 2,5 ng/ml de TNF-α recombinant murin, avec ou sans IL-10 recombinante murine (500 ng/ml). Les DC-MO ont été récoltées à J6.

### Purification des cellules dendritiques spléniques

Des rates ont été découpées en menus fragments qui ont été digérés par la collagénase D (1 mg/ml) dans du HBSS pendant 20 mn à 37°C, sous agitation constante. Les fragments digérés ont été filtrés sur grille de 0,7 µm (Becton-Dickinson) et la suspension cellulaire a été lavée deux fois dans le milieu de purification. Les cellules ont alors été déposées en couche sur un gradient de métrizamide et centrifugées à 600 g pendant 10 min. Les cellules concentrées à l'interface ont été récupérées, lavées une fois et remises en suspension dans le milieu de purification, pour dissocier les DC des lymphocytes. Les différentes lignées de cellules T, les cellules B et les granulocytes ont été déplétées en traitant les cellules de basse densité récupérées, pendant 30 min à 4°C, avec un mélange d'anticorps monoclonaux composé d'anti-CD3, d'anti-B220 et d'anti-GR-1. Les cellules positives ont été récupérées magnétiquement, après incubation pendant 1 h à 4°C avec des billes magnétiques recouvertes d'Ig anti-rat, dans un rapport de 10 billes pour 1 cellule.

### Préparation des cellules T transgéniques TCR "vierges"

Des cellules T spléniques de souris DO11.10 transgéniques OVA TCR ont été préparées comme décrit ailleurs (Groux et al., 1997). En bref, des cellules T CD4+ ont été enrichies par sélection négative au moyen de billes magnétiques avec un mélange d'ACm anti-CD8, anti-B220 et anti-CD11b. Les cellules T CD4⁺/Mel14^{brigh} ont ensuite été triées par cytométrie de flux en utilisant des anticorps anti-CD4-PE et anti-CD62L-FITC (Mel14). Les populations de cellules T triées étaient typiquement positives à 99 % pour les deux marqueurs.

### Différenciation des cellules T transgéniques TCR par les cellules dendritiques

Les essais *in vitro* de différenciation des cellules T ont été effectués dans du milieu Yssel. Les cultures primaires de stimulation ont été établies en procédant à une activation de cellules T CD4+ "vierges" purifiées (2,5 x 10⁵) par des populations de cellules dendritiques triées, pulsées avec le peptide OVA ₃₂₃₋₃₃₉ (0,6 µM) dans un volume total de 1 ml, sur des plaques de 24 puits (Becton Dickinson). Les cellules ont été multipliées, récoltées à J7, lavées trois fois, comptées et restimulées par des populations de cellules dendritiques fraîchement triées + 0,3 µM de peptide OVA 323-339 pour la seconde différenciation. La même procédure a été appliquée pour le troisième cycle de différenciation. Après la différenciation, les cellules T ont été récoltées, lavées et restimulées avec 0,3 µM d'OVA 323-339 et des CPA spléniques irradiées. La prolifération des cellules T a été mesurée par l'incorporation de ³H-thymidine au cours des 12 dernières heures de l'incubation de 72 h. La production d'IL-4, d'IL-10 et d'IFN-γ a été mesurée par ELISA dans les surnageants recueillis 48 h après la restimulation des cellules T.

### Expériences transwell

Dans le compartiment inférieur d'un système transwell (0,4 µm Costar-Dutscher, Brumath, France), 10⁶ cellules T CD4+ purifiées isolées de souris BALB/c normales ont été stimulées avec 10⁶ splénocytes irradiés de souris BALB/c et des ACm anti-CD3 (10 µg/ml). Dans le compartiment supérieur, des cellules T CD4+ différenciées par une stimulation unique avec des cellules dendritiques ont été stimulées avec 0,3 µM d'OVAp et 10⁶ splénocytes irradiés. Trois jours plus tard, le panier a été retiré et les cellules T des puits inférieurs ont été transférées (en triple) dans des plaques à 96 puits. La réponse proliférative a été analysée par l'incorporation de ³H-thymidine au cours des 12 dernières heures de culture.

### Cytologie

Les DC CD11c^{low}CD45RB⁺ et CD11c^{high}CD45RB⁻ triées, générées *in vitro* ou isolées de rates de souris BALB/c, ont été incubées pendant 24 h avec du LPS (1 µg/ml) en présence de GM-CSF (10 ng/ml). Les DC fraîchement triées ou stimulées ont été centrifugées (Cytospin2, Shandon) et colorées au May-Grünwald-Giemsa.

### Dosage par RT-PCR quantitative en temps réel

L'ARN total des sous-ensembles de DC triées (1 x 10⁶; pureté >99%) a été préparé avec du TRIZOL (Life Technologies), comme décrit ailleurs (Cottrez et al., 1994), et toute trace éventuelle d'ADN chromosomique contaminant a été digérée par la DNase I, en suivant les instructions du fabricant (Gene Hunter, Nashville, TX). Puis l'ARN a fait l'objet d'une transcription inverse avec un oligo(dT)12-18 et la transcriptase inverse Superscript II (Life Technologies), comme décrit ailleurs (Cottrez et al., 1994). La PCR quantitative en temps réel a été effectuée avec la trousse SYBR Green PCR Core Reagents Kit, sur des plaques de microtitrage spéciales, à 96 puits (Applied Biosystems, Courtaboeuf, France) dans un appareil ABI PRISM 5700 Sequence Detection System (Applied Biosystems), en suivant les instructions du fabricant. Les signaux de fluorescence ont été générés pendant chaque cycle de PCR par incorporation directe du SYBR Green dans la chaîne d'ADN bicaténaire, pour donner des informations quantitatives en temps réel. Des amorces (MWG Biotech, Ebersberg, Allemagne) englobant les jonctions exons-introns ont été conçues pour éviter l'amplification de l'ADN génomique et donner des amplicons de 100 à 150 pb, afin d'accroître l'efficacité de l'amplification par PCR. Toutes les amorces ont été utilisées dans des conditions qui ont évité la formation de dimères, et la fidélité des produits d'amplification a été vérifiée par électrophorèse et cartes de restriction enzymatique. Tous les ADNc ont été titrés sur la valeur de l'expression moyenne de 4 différents gènes ordinaires (*housekeeping*)*.* Les conditions expérimentales de la PCR étaient les suivantes : 10 mn à 94°C, et 40 cycles de 30 s à 94°C, 30 s à 60°C et 30 s à 72°C pour chaque amplification, dans un volume final de 20 µl. L'expression des gènes cibles a été mesurée, après normalisation des concentrations d'ARN, avec les 4 gènes ordinaires et les valeurs sont exprimées en facteurs d'augmentation de l'expression par rapport à un témoin négatif

### Dosages des cytokines

L'IL-4, l'IL-10 et l'IFN-γ ont été dosés par une méthode ELISA en sandwich, comme décrit ailleurs (Cottrez et al., 2000). En bref, des plaques ELISA (Polylabo, France) ont été recouvertes d'ACm anti-cytokines appropriés en tampon carbonate et incubées à 4°C pendant une nuit. Les réactions ont été bloquées pendant 30 min à température ambiante avec 150 µl de PBS/SVF à 20% dans chaque puits ; 50 µl de surnageants dilués des cellules T CD4+ stimulées *in vitro* ont ensuite été ajoutés aux puits, avant d'incuber les plaques à 4°C pendant une nuit. Après une étape de lavage, 50 µl de l'anticorps biotinylé de la seconde étape ont été ajoutés dans chaque puits. Les plaques ont été incubées pendant 1 h à température ambiante et lavées. Le conjugué enzymatique (streptavidine-peroxydase) a ensuite été ajouté dans chaque puits. Les plaques ont été incubées à température ambiante pendant 1 h, lavées, et 100 µl de substrat (ABTS, 1 mg/ml) ont été ajoutés par puits. Les plaques ont été lues sur un lecteur ELISA à 405 nm, après formation de la couleur (Labsystems iEMS reader, Helsinki, Finlande).

### L'IL-10 induit la différenciation d'un sous-ensemble distinct de DC.

On sait que l'IL-10 induit la différenciation des cellules Tr1 par un effet indirect (Wakkach et al., 2001). Etant donné que les DC sont des acteurs essentiels dans la différenciation des cellules T, les inventeurs ont analysé l'effet de l'IL-10 sur la différenciation des DC à partir de cellules progénitrices de la moëlle osseuse cultivées en présence de GM-CSF et de TNF-α (les expériences préliminaires ont montré que la présence de TNF-α n'influence pas le phénotype et la fonction des DC, mais aide toutefois à augmenter le rendement et la viabilité des populations de cellules dendritiques [données non présentées]). L'addition d'IL-10 au jour 0 de la culture a induit la différenciation d'une population de DC avec une faible expression de CD11c et une forte expression de CD45RB (figure 9A). En revanche, en l'absence d'IL-10, l'addition de GM-CSF et de TNF-α a induit la différenciation de DC exprimant de fortes concentrations de CD11c (figure 9A) (Inaba et al., 1992). Après triage des cellules au FACS en fonction de l'expression spécifique de CD11c et de CD45RB, les DC CD11c^{low}CD45RB⁺ différenciées *in vitro* présentaient une morphologie plasmocytoïde, avec une membrane plasmique lisse et un noyau excentrique (figure 9B-1). D'autre part, les DC CD11c^{high} avaient une morphologie différente, avec présence de petites dendrites (figure 9B-3). Après une nouvelle maturation avec le LPS, les deux populations ont acquis une morphologie de DC pleinement matures, avec de longues dendrites (figure 9B-2 et 4). L'analyse de l'expression des molécules du CMH II et des costimulateurs a révélé de faibles niveaux d'expression du CD80, du CD86 et de l'I-A sur les DC CD11c^{low}CD45RB⁺ (figure 9C) par rapport aux DC CD11c^{high}. La maturation des cellules dendritiques avec le LPS n'a pas modifié le phénotype des DC CD11c^{low}CD45RB⁺ (figure 9C), tandis qu'elle renforçait l'expression des molécules de CD80, CD86 et I-A sur les DC CD11c^{high}. Ces résultats montrent que l'IL-10 induit la différenciation d'un sous-ensemble distinct de DC caractérisées par l'expression spécifique de CD45RB, qui présentent un phénotype pseudo-immature qui ne peut être modifié par une stimulation avec le LPS.

### Isolement de l'équivalent naturel des DC dérivées par l'IL-10

Les inventeurs ont donc abordé la question de l'influence de l'IL- 10 *in vivo* sur la différenciation des cellules dendritiques. Des DC spléniques enrichies isolées de souris Tg IL-10 non transgéniques ou de souris BALB/c ont été analysées pour l'expression du marqueur spécifique des DC murines CD11c (figure 10A). Chez les souris Tg IL-10, il a été observé un plus grand nombre de DC exprimant faiblement le CD11 c par rapport aux souris BALB/c normales (figure 10A) ou aux témoins non transgéniques (données non présentées). Comme pour les DC CD11c^{low} différenciées *in vitro* en présence d'IL-10, les DC spléniques CD11c^{low} exprimaient fortement le marqueur CD45RB (figure 10B). Par ailleurs, les DC dérivées *in vitro* et les DC CD11c^{low}CD45RB⁺ spléniques ont toutes deux une morphologie plasmocytoïde (figure 10C-1), contrairement aux DC CD11c^{high} qui présentent de petites dendrites (figure 10C-3). Après une maturation complète avec le LPS, les deux populations de DC spléniques se différencient en DC pleinement matures avec longues dendrites (figures 10C-2 et 4).

Les inventeurs ont ensuite étudié l'expression des molécules I-A^{d} du CMH et des costimulateurs CD80 et CD86 sur les DC spléniques CD11c^{low}CD45RB⁺ et CD11c^{high}CD45RB⁻ triées sur FACS, provenant de souris Tg IL-10 et de souris BALB/c témoins. Comme le montre la figure 10D, il a été constaté que les DC CD11^{low}CD45RB⁺ purifiées *ex vivo* exprimaient faiblement les molécules du CMH de classe II, le CD80 et le CD86, par rapport aux DC CD11c^{high}CD45RB⁻. Pour déterminer si les DC CD11c^{low}CD45RB⁺ représentent un sous-ensemble distinct de DC et le produit d'une lignée développementale séparée, ou si elles représentent un stade immature de DC plus conventionnelles, la maturation *in vitro* des DC isolées a été induite par des cultures à court terme en présence de GM-CSF (afin d'augmenter la durée de vie et le taux de récupération) et de LPS. Dans ces conditions, après maturation *in vitro* avec le LPS, les DC CD11c^{low}CD45RB⁺ conservaient toujours une faible expression des molécules du CMH de classe II et du CD86, ce qui suggère que leur phénotype pseudo-immature est stable, à l'exception de la surexpression du CD80. En revanche, après incubation *in vitro* avec le LPS, les DC CD11c^{high}CD45RB⁻ montrent une maturation accrue, avec augmentation de l'expression des molécules du CMH de classe II et des deux costimulateurs (CD80 et CD86) (figure 10D). Globalement, ces résultats montrent que les DC CD11c^{low}CD45RB⁺ isolées de la rate représentent des équivalents *in vivo* des DC dérivées par l'IL-10.

### Caractérisation phénotypique et sécrétion des cytokines du sous-ensemble de cellules dendritiques CD11c^{low} CD45RB⁺

Pour mieux caractériser le sous-ensemble de DC CD11c^{low}CD45RB⁺, les inventeurs ont analysé l'expression des différents marqueurs des cellules dendritiques sur les populations cellulaires CD11c^{low}CD45RB⁺ et CD11c^{high}CD45RB⁻ triées provenant de souris BALB/c (figure 11A). Les DC CD11c^{low}CD45RB⁺ expriment faiblement le CD11b et le DEC 205 et pas du tout le CD8α, ce qui suggère qu'elles n'appartiennent pas à la lignée "classique" des DC myéloïdes (CD11b^{high}) ou lymphoïdes (CD8α⁺) (Shortman and Liu, 2002). Par ailleurs, contrairement à une population de DC tolérogènes récemment décrite différenciée à partir d'un précurseur de cellule B, les DC CD11c^{low}CD45RB⁺ sont négatives en B220 (Lu et al., 2001 ; Martin et al., 2002). Enfin, ces cellules n'expriment pas les marqueurs spécifiques des cellules T (CD4 et CD2) (données non présentées).

Une part importante de la fonction spécifique des DC est dictée par la sécrétion d'ensembles distincts de cytokines. Les inventeurs ont donc analysé, par RT-PCR quantitative, l'expression relative de plusieurs cytokines importantes sur des cellules dendritiques CD11c^{low}CD45RB⁺ et CD11c^{high}CD45RB⁻ fraîchement triées ou activées par le LPS. Après activation par le LPS, les DC CD11c^{low}CD45RB⁺ sécrétaient de l'IL-10 alors que les DC CD11c^{high}CD45RB⁻ sécrétaient de l'IL-12. Les deux populations montraient une sécrétion d'IL-1β, encore augmentée par l'activation par le LPS, mais ne sécrétaient pas d'IFN-α, même après 24 h d'activation par le LPS (figure 11B). Ces résultats montrent que les DC CD11c^{low}CD45RB⁺ représentent un sous-ensemble distinct de cellules dendritiques qui sécrètent principalement de l'IL-10 après activation.

### Les DC CD11c^{low} CD45RB⁺ différentient les cellules Tr1 in vitro

Pour analyser l'influence des différents sous-ensembles de DC sur l'amorçage et la différenciation de cellules T spécifiques jamais exposées à l'OVA ("vierges") isolées de souris DO11-10, plusieurs cycles de stimulation des cellules T ont été établis avec des DC CD11c^{low}CD45RB⁺ ou CD11c^{high}CD45RB⁻ triées isolées de la rate de souris BALB/c (figure 12A) ou différenciées *in vitro* (figure 12B). Les cellules T CD4+ "vierges" purifiées ont été stimulées par l'OVA (0,6 µM), à différents rapports DC/T, pendant une semaine. Puis les cellules T ont été récoltées, lavées et stimulées une nouvelle fois, dans les mêmes conditions, avec 0,3 µM d'OVA. Après une troisième stimulation dans les mêmes conditions, les populations de cellules T polarisées ont été récupérées, lavées et restimulées avec des splénocytes irradiés de souris BALB/c et 0,3 µM d'OVA pour analyser leur profil de cytokines (figure 12A). Nos résultats montrent que les DC CD11c^{low}CD45RB⁺ purifiées *ex vivo* ou différenciées *in vitro,* quel que soit le rapport DC/T utilisé, induisaient la différenciation de cellules Tr1 sécrétant fortement l'IL-10, faiblement l'IFN-γ et pas d'IL-4 (ou alors en quantités négligeables) (Groux et al., 1997) (figures 12 A et B). Contrairement aux cellules dendritiques CD11c^{low}CD45RB⁺, celles du sous-ensemble CD11c^{high}CD45RB⁻ amorçaient les cellules Th1 qui sécrètent fortement l'IFN-γ (figures 12A et B). Ces résultats sont corrélés au profil de cytokines de la sous-population de cellules dendritiques, c-à-d. CD11c^{low}CD45RB⁺, qui différencient les cellules Tr1, secrètent l'IL-10, alors que l'IL-12 sécrétée par les DC CD11c^{high}CD45RB⁻ induit la différenciation des cellules Th1.

Par ailleurs, la différenciation spécifique des cellules Tr1 avec les DC CD11c^{low}CD45RB⁺ a été observée après une stimulation unique, comme l'a montré l'analyse intracytoplasmique de la sécrétion de cytokines dans les différentes sous-populations de cellules T (figure 12C). Pour analyser les propriétés fonctionnelles des cellules T obtenues après une stimulation unique par les DC CD11c^{low}CD45RB⁺, des expériences transwell ont été conduites. Les cellules T obtenues après stimulation par des DC CD11c^{low}CD45RB⁺ de souris BALB/c ont inhibé la prolifération des cellules T CD4+ en attente (*bystander*) stimulées par des splénocytes irradiés et un anticorps monoclonal anti-CD3 (figure 12D). D'autre part, l'addition d'ACm inhibiteurs de souris anti-IL-10 et anti-TGFβ a levé l'effet suppresseur de ces cellules T, confirmant la différenciation des cellules Tr1 au plan phénotypique et fonctionnel (figure 12D). A titre de contrôle, des populations de cellules Th1 (induites par des DC CD11c^{higb}CD45RB⁻) n'ont pas eu d'effet inhibiteur sur la prolifération des cellules T CD4+ en attente (figure 12D). Globalement, ces résultats montrent que les DC CD11c^{low}CD45RB⁺ induisent la différenciation des cellules Tr1 *in vitro.*

### Cellules humaines

Des cellules dendritiques ont été différentiées à partir de cellules progénitrices CD34+ avec du GM-CSF et de l'IL-4 en présence ou en absence d'IL-10, comme indiqué.

Après 6 jours, les cellules dendritiques ont été triées en fonction de l'expression de CD11c et marquées avec les anticorps indiqués.

Les cellules purifiées ont aussi été stimulées avec du LPS pendant 24 heures, puis analysées par cytofluorométrie.

Les cellules dendritiques tolérogènes différentiées en présence d'IL-10 exprimaient de bas niveaux de CD11c, et de bas niveaux des molécules HLA-DR, CD80 et CD86.

L'activation avec le LPS n'a pas augmenté l'expression de HLA-DR et CD86 pour cette population, au contraire de l'effet du LPS sur les autres populations de DC testées.

**Tableau: effet de l'activation avec le LPS sur les populations de cellules dendritiques triées différentiées en présence ou absence d'IL-10.**

| Pourcentage de cellules positives | | | | | | | | |
|---|---|---|---|---|---|---|---|---|
| Stimulation | - | - | LPS | LPS | - | - | LPS | LPS |
| Différentiation | - | - | - | - | IL-10 | IL-10 | IL-10 | IL-10 |
| | CD11c^{low} | CD11c^{hi} | CD11c^{low} | CD11c^{hi} | CD11c^{low} | CD11c^{hi} | CD11c^{low} | CD11c^{hi} |
| CD14 | 12±3 | 14±4 | 12±4 | 13±3 | **15±3** | 9±2 | **13±3** | 13±3 |
| CD4 | 4±2 | 3±1 | 3±2 | 2±1 | **4±1** | 5±3 | **5±2** | 3±2 |
| CD11b | 13±1 | 24±8 | 23±6 | 21±8 | **19±6** | 21±4 | **18±5** | 15±6 |
| HLA-DR | 75±12 | 80±9 | 91±15 | 89±12 | **50±8** | 85±12 | **52±6** | 92±12 |
| CD80 | 24±5 | 28±6 | 80±13 | 73±12 | **14±2** | 26±11 | **45±12** | 84±7 |
| CD86 | 53±6 | 54±5 | 85±15 | 79±123 | **26±4** | 54±9 | **28±6** | 73±11 |
| CD40 | 81±9 | 94±2 | 98±12 | 96±11 | **74±8** | 81±15 | **82±13** | 95±11 |

| Moyenne d'intensité de fluorescence | | | | | | | | |
|---|---|---|---|---|---|---|---|---|
| Stimulation | - | - | LPS | LPS | - | - | LPS | LPS |
| Différentiation | - | - | - | - | IL-10 | IL-10 | IL-10 | IL-10 |
| | CD11c^{low} | CD11c^{hi} | CD11c^{low} | CD11c^{hi} | CD11c^{low} | CD11c^{hi} | CD11c^{low} | CD1 1c^{hi} |
| CD14 | 250±130 | 320±110 | 210±150 | 350±80 | **210±180** | 350±87 | **250±130** | 390±102 |
| CD4 | 80±63 | 86±38 | 91±58 | 86±27 | **70±53** | 83±48 | **91±54** | 75±52 |
| CD11b | 370±356 | 350±256 | 367±298 | 357±241 | **324±293** | 360±255 | **357±297** | 354±291 |
| HLA-DR | 470±570 | 590±350 | 890±480 | 981±210 | **310±150** | 520±350 | **350±218** | 846±520 |
| CD80 | 134±102 | 290±130 | 357±150 | 481±213 | **102±80** | 198±97 | **283±110** | 435±115 |
| CD86 | 400±270 | 595±600 | 813±370 | 789±483 | **254±130** | 587±480 | **312±178** | 834±420 |
| CD40 | 320±230 | 580±256 | 680±269 | 710±297 | **367±260** | 539±200 | **340±264** | 730±260 |

## Revendications

1. Procédé d'identification de lymphocytes Tr1 régulateurs présents dans un échantillon biologique comprenant des lymphocytes, **caractérisé en ce qu'**il comprend les étapes suivantes :
a) déterminer la présence simultanée des produits d'expression par lesdits lymphocytes des gènes codant pour la molécule CD4 et l'ensemble des molécules du groupe A, ledit groupe A étant constitué par les molécules CD18 et/ou CD11a, et CD49b ; et
b) identifier comme lymphocytes Tr1 régulateurs les lymphocytes qui expriment simultanément les gènes codant pour la molécule CD4 et l'ensemble des molécules du groupe A.

2. Procédé selon la revendication **1**, **caractérisé en ce que** :
- à l'étape (a) on compare en outre l'expression par lesdits lymphocytes d'au moins un gène choisi parmi les gènes codant pour les molécules du groupe B suivant : CD11a, CD18, PSGL-1, PECAM-1 et alphaV/beta3, ladite expression étant comparée avec l'expression dudit même gène par des lymphocytes de type Th1 ou Th2 ; et
- **en ce qu'**à l'étape (b) on identifie comme lymphocytes Tr1 régulateurs les lymphocytes qui surexpriment au moins l'un desdits gènes codant pour les molécules du groupe B.

3. Procédé selon la revendication **2**, **caractérisé en ce qu'**à l'étape (a) on compare l'expression d'au moins deux desdits gènes du groupe B et **en ce qu'**à l'étape (b) on identifie comme lymphocytes Tr1 régulateurs les lymphocytes qui surexpriment les deuxdits gènes du groupe B.

4. Procédé selon la revendication **3**, **caractérisé en ce qu'**à l'étape (a) on compare l'expression de tous les gènes du groupe B et qu'en ce qu'à l'étape (b) on identifie comme lymphocytes Tr1 régulateurs les lymphocytes qui surexpriment tous les gènes du groupe B.

5. Procédé d'identification selon l'une quelconque des revendications **1 à 4**, **caractérisé en ce qu'**à l'étape (a) on détermine en outre et simultanément la présence dudit produit d'expression par lesdits lymphocytes du gène codant pour la molécule CD3 et **en ce qu'**à l'étape (b) on identifie comme lymphocytes Tr1 régulateurs les lymphocytes qui expriment en outre simultanément le gène codant pour la molécule CD3.

6. Procédé selon l'une quelconque des revendications **1 à 5**, **caractérisé en ce qu'**à l'étape (a) on détermine la présence simultanée desdites molécules du groupe A exprimées à la surface desdits lymphocytes.

7. Procédé selon la revendication **6**, **caractérisé en ce qu'**à l'étape (a) on détermine la présence simultanée desdites molécules exprimées à la surface desdits lymphocytes au moyen d'anticorps spécifiques desdites molécules.

8. Procédé selon la revendication **7**, **caractérisé en ce que** lesdits anticorps spécifiques sont marqués par un marqueur capable d'être détecté de manière directe ou indirecte.

9. Procédé selon la revendication **8**, **caractérisé en ce que** chacun desdits anticorps est marqué par un marqueur différent.

10. Procédé selon la revendication **8 ou 9**, **caractérisé en ce que** lesdits marqueurs sont fluorescents et sont choisis dans le groupe constitué de l'isothiocyanate de fluorescéine (FITC), l'allophycocyanine (APC), la phycoérythrine-cyanine 5 (PC5), la phycoérythrine (PE), le diacétate de fluorescéine fluorescente verte, la calcéine AM, et la tétraméthyl rhodamine fluorescente rouge.

11. Procédé selon l'une quelconque des revendications **6 à 10**, **caractérisé en ce qu'**à l'étape (a) la détermination de la présence simultanée desdites molécules du groupe A exprimées à la surface desdits lymphocytes est mise en oeuvre par cytométrie de flux.

12. Procédé selon la revendication **10 ou 11**, **caractérisé en ce qu'**à l'étape (a) dudit procédé on détermine, pour la molécule CD18, la présence d'une intensité de fluorescence de type CD 18bright.

13. Procédé selon l'une des revendications **2 à 12, caractérisé en ce que** :
- à l'étape (a) la comparaison de l'expression par lesdits lymphocytes d'au moins un gène codant pour les molécules du groupe B est effectuée par comparaison de la quantité d'ARNm exprimé par ledit gène ; et
- **en ce qu'**à l'étape (b) on identifie comme lymphocytes Tr1 régulateurs les lymphocytes qui surexpriment l'ARNm dudit gène.

14. Procédé selon la revendication **13**, **caractérisé en ce que** l'on mesure la quantité d'ARNm par RT-PCR quantitative.

15. Procédé selon l'une quelconque des revendications **1 à 14**, **caractérisé en ce que** l'échantillon biologique est issu d'un prélèvement de sang périphérique ou d'un organe inflammatoire chez un sujet.

16. Procédé selon la revendication **15**, **caractérisé en ce que** le prélèvement est effectué chez un sujet atteint ou susceptible d'être atteint d'une maladie auto-immune ou inflammatoire.

17. Procédé selon la revendication **16**_{,} **caractérisé en ce que** ledit sujet est atteint de la maladie de Crohn ou de la sclérose en plaques.

18. Procédé selon l'une quelconque des revendications **1 à 14**, **caractérisé en ce que** l'échantillon biologique est issu d'un procédé de préparation *in vitro* de lymphocytes Tr1 régulateurs à partir d'une population de lymphocytes issus d'un prélèvement chez un sujet.

19. Procédé selon la revendication **18**, **caractérisé en ce que** le procédé de préparation de lymphocytes Tr1 régulateurs comprend au moins une étape d'activation de lymphocytes T CD4+ de ladite population de lymphocytes en présence d'un antigène et d'interleukine 10.

20. Procédé selon la revendication **18**, **caractérisé en ce que** le procédé de préparation de lymphocytes Tr1 régulateurs comprend les étapes suivantes :
a) activer in vitro les lymphocytes T CD4+ de ladite population de lymphocytes en présence de l'antigène choisi, présenté par les cellules présentatrices d'antigène artificielles qui expriment une molécule du système HLA de classe II et une molécule de LFA-3 humain et qui n'expriment aucune des molécules de co-stimulation B7-1, B7-2, B7-H1, CD40, CD23 ou ICAM-1 obtenue à partir d'un échantillon biologique ; et
b) récupérer, à partir desdits lymphocytes, une population de lymphocytes T CD4+ activés comprenant au moins 10% de lymphocytes Tr1 spécifiques de l'antigène choisi.

21. Procédé selon la revendication **18**_{,} **caractérisé en ce que** le procédé de préparation de lymphocytes Tr1 régulateurs comprend les étapes suivantes :
a) obtenir in vitro une population de cellules progénitrices humaines capables de se différencier en cellules dendritiques ;
b) mettre en culture lesdites cellules progénitrices humaines en présence d'IL-10 pour obtenir une population desdites cellules dendritiques ; et
c) mettre en présence ladite population de lymphocytes humains avec la population de cellules dendritiques obtenues en (b).

22. Procédé selon l'une quelconque des revendications **1 à 5**, **caractérisé en ce que** les produits d'expression par lesdits lymphocytes des gènes codant pour les molécules du groupe A sont des ARNm, et **en ce qu'**à l'étape (a) la détermination de la présence simultanée desdits ARNm est réalisée par RT-PCR.

23. Procédé de quantification de lymphocytes Tr1 régulateurs présents dans un échantillon biologique comprenant des lymphocytes, **caractérisé en ce qu'**il comprend les étapes consistant à :
a) identifier les lymphocytes Tr1 régulateurs par un procédé d'identification selon l'une quelconque des revendications **1 à 21** ; et
b) déterminer la proportion des lymphocytes Tr1 régulateurs identifiés en (a) par rapport à la quantité totale des lymphocytes ou d'une fraction particulière des lymphocytes, présent dans ledit échantillon biologique.

24. Procédé de pronostic ou de diagnostic in vitro d'une maladie auto-immune ou inflammatoire chez un sujet à partir d'un échantillon biologique préalablement prélevé chez ledit sujet, **caractérisé en ce qu'**il comprend les étapes suivantes :
a) déterminer la proportion de lymphocytes Tr1 régulateurs présents dans ledit échantillon biologique par rapport à la quantité totale des lymphocytes, ou d'une fraction particulière des lymphocytes, selon le procédé de quantification de la revendication **23** ; et
b) comparer la proportion desdits lymphocytes Tr1 régulateurs obtenue à l'étape (a) par rapport à celle présente dans un échantillon biologique prélevé chez un sujet sain.

25. Procédé de pronostic ou de diagnostic in vitro d'une maladie auto-immune ou inflammatoire selon la revendication **24**, **caractérisé en ce qu'**à l'étape (b) on observe une diminution de ladite proportion chez le sujet à tester.

26. Procédé d'enrichissement en lymphocytes Tr1 régulateurs présents dans un échantillon biologique comprenant des lymphocytes, **caractérisé en ce qu'**il comprend les étapes suivantes :
a) identifier les lymphocytes Tr1 régulateurs par un procédé d'identification selon l'une quelconque des revendications **1 à 22** ; et
b) éliminer dudit échantillon une part significative des lymphocytes ne présentant pas simultanément lesdites molécules.

## Claims

1. Method for identifying Tr1-regulatory lymphocytes present in a biological sample comprising lymphocytes, **characterized in that** it comprises the following steps:
a) determining the simultaneous presence of the expression products by said lymphocytes of genes encoding the CD4 molecule and all of the molecules of group A, said group A being constituted of CD8 and/or CD11a molecules and CD49b; and
b) identifying, as Tr1-regulatory lymphocytes, the lymphocytes that simultaneously express the genes encoding the CD4 molecule and all of the molecules of Group A.

2. Method according to claim 1, **characterized in that**:
- step (a) involves the comparison of the expression by said lymphocytes of at least one gene selected from the genes encoding the molecules of following group B: CD11a, CD18, PSGL-1, PECAM-1 and alphaV/beta3, wherein said expression is compared with the expression of said same gene by Th1 or Th2 lymphocytes; and
- step (b) involves the identification, as Tr1-regulatory lymphocytes, of the lymphocytes that overexpress at least one of said genes encoding the molecules of group B.

3. Method according to claim 2, **characterized in that** step (a) involves the comparison of the expression of at least two of said genes of group B and **in that** step (b) involves the identification, as Tr1-regulatory lymphocytes, of the lymphocytes that overexpress said two genes of group B.

4. Method according to claim 3, **characterized in that** step (a) involves the comparison of the expression of all of the genes of group B and **in that** step (b) involves the identification, as Tr1-regulatory lymphocytes, of the lymphocytes that overexpress all the genes of group B.

5. Identification method according to any one of claims 1 to 4, **characterized in that** step (a) consists of determining, additionally and simultaneously, the presence of the expression product by said lymphocytes of the gene encoding the CD3 molecule and **in that** step (b) consists of identifying, as Tr1-regulatory lymphocytes, the lymphocytes that also simultaneously express the gene encoding the CD3 molecule.

6. Method according to any one of claims 1 to 5, **characterized in that** step (a) consists of determining the simultaneous presence of said molecules of group A expressed at the surface of said lymphocytes.

7. Method according to claim 6, **characterized in that** step (a) consists of determining the simultaneous presence of said lymphocytes by means of antibodies specific to said molecules.

8. Method according to claim 7, **characterized in that** said specific antibodies are marked with a marker capable of being detected directly or indirectly.

9. Method according to claim 8, **characterized in that** each of said antibodies is marked by a different marker.

10. Method according to claim 8 or 9, **characterized in that** said markers are fluorescent and are selected from the group consisting of fluorescein isothiocyanate (FITC), or allophycocyanin (APC), phycoerythrin-cyanin 5 (PC5), phycoerythrin (PE), green fluorescent fluorescein diacetate, calcein AM and red fluorescent tetramethyl rhodamine.

11. Method according to any one of claims 6 to 10, **characterized in that** step (a) consists of determining the simultaneous presence of said molecules of group A expressed at the surface of said lymphocytes by flow cytometry.

12. Method according to claim 10 or 11, **characterized in that** step (a) of said method consists of determining, for the CD18 molecule, the presence of a CD18bright fluorescence intensity.

13. Method according to any one of claims 2 to 12, **characterized in that**:
- in step (a), the comparison of the expression by said lymphocytes of at least one gene encoding the molecules of group B is carried out by comparing the amount of mRNA expressed for said gene; and
- step (b) involves identifying, as Tr1-regulatory lymphocytes, the lymphocytes that overexpress the mRNA of said gene.

14. Method according to claim 13, **characterized in that** the amount of mRNA is measured by quantitative RT-PCR.

15. Method according to any one of claims 1 to 14, **characterized in that** the biological sample is from a peripheral blood sample or an inflammatory organ in a subject.

16. Method according to claim 15, **characterized in that** the sample is take from a subject affected or likely to be affected by autoimmune or inflammatory disease.

17. Method according to claim 16, **characterized in that** said subject had Crohn's disease or multiple sclerosis.

18. Method according to any one of claims 1 to 14, **characterized in that** the biological sample is obtained from a method for *in vitro* preparation of Tr1-regulatory lymphocytes using a population of lymphocytes obtained from a sample of a subject.

19. Method according to claim 18, **characterized in that** the Tr1-regulatory lymphocyte preparation method comprises at least one step of activating CD4+ T lymphocytes of said lymphocyte population in the presence of an antigen and interleukin 10.

20. Method according to claim 18, **characterized in that** the Tr1-regulatory lymphocyte preparation method comprises the following steps:
(a) activating, *in vitro,* the CD4+ T lymphocytes of said lymphocyte population in the presence of the selected antigen, presented by artificial antigen-presenting cells expressing a molecule of the HLA class-II system and a human LFA-3 molecule and that do not express any co-stimulation molecules B7-1, B7-2, B7-H1, CD40, CD23 or ICAM-1; and
(b) collecting, from said lymphocytes, an activated CD4+ lymphocyte population comprising at least 10 % Tr1 lymphocytes specific to the selected antigen.

21. Method according to claim 18, **characterized in that** the Tr1-regulatory lymphocyte preparation method comprises the following steps:
(a) obtaining, *in vitro,* a population of human progenitor cells capable of differentiating into dendritic cells;
(b) placing said human progenitor cells in a culture in the presence of IL-10 so as to obtain a population of said dendritic cells; and
(c) placing said human lymphocyte population in the presence of the dendritic cell population obtained in (b).

22. Method according to any one of claims 1 to 5, **characterized in that** the expression products by said lymphocytes of genes encoding the molecules of group A are mRNAs, and **in that**, in step (a), the determination of the simultaneous presence of said mRNA is performed by RT-PCR.

23. Method for quantification of Tr1-regulatory lymphocytes present in a biological sample comprising lymphocytes; **characterized in that** it comprises the steps of:
(a) identifying Tr1-regulatory using an identification method according to anyone of claims 1 to 21;
(b) determining the proportion of Tr1-regulatory lymphocytes identified in (a) with respect to the total amount of lymphocytes or a particular fraction of lymphocytes, present in said biological sample.

24. Method for *in vitro* prognosis or diagnosis of an autoimmune or inflammatory disease in a subject, using a biological sample previously taken from said subject, **characterized in that** it comprises the following steps:
(a) determining the proportion of Tr1-regulatory lymphocytes present in said biological sample with respect to the total amount of lymphocytes or a particular fraction of lymphocytes, according to the quantification method of claim 23; and
(b) comparing the proportion of said Tr1-regulatory lymphocytes obtained in step (a) with that present in a biological sample taken from a healthy subject.

25. Method for *in vitro* prognosis or diagnosis of an autoimmune or inflammatory disease according to claim 24, **characterized in that**, in step (b), a reduction in said proportion is observed in the subject to be tested.

26. Method for enrichment of Tr1-regulatory lymphocytes present in a biological sample comprising lymphocytes, **characterized in that** it comprises the following steps:
(a) identifying the Tr1-regulatory lymphocytes using the identification method according to any one of claims 1 to 22; and
(b) removing a significant portion of the lymphocytes not simultaneously having said molecules from said sample.

## Patentansprüche

1. Verfahren zur Identifizierung von regulatorischen Tr1-Lymphozyten, die in einer biologischen Probe, die Lymphozyten umfasst, vorhanden sind, **dadurch gekennzeichnet, dass** es die folgenden Schritte umfasst:
a) Bestimmen der gleichzeitigen Anwesenheit von Expressionsprodukten dieser Lymphozyten in Form von Genen, die für das Molekül CD4 und für die Gesamtheit der Moleküle der Gruppe A kodieren, wobei die Gruppe A aus den Molekülen CD18 und/oder CD11a und CD49b besteht; und
b) Identifizieren von Lymphozyten, die gleichzeitig Gene exprimieren, die für das Molekül CD4, sowie die Gesamtheit der Moleküle der Gruppe A kodieren als regulatorische Tr1-Lymphozyten.

2. Verfahren gemäß Anspruch 1, **dadurch gekennzeichnet, dass**:
- man in dem Schritt (a) unter anderem die Expression von mindestens einem Gen, ausgewählt aus den Genen, die für die Moleküle der folgenden Gruppe B kodieren: CD11a, CD18, PSGL-1, PECAM-1 und alphaV/beta3 durch diese Lymphozyten vergleicht, wobei die Expression mit der Expression des gleichen Gens von Lymphozyten vom Typ Th1 oder Th2 verglichen wird; und
- **dadurch gekennzeichnet, dass** man im Schritt (b) die Lymphozyten als regulatorische Tr1-Lymphozyten identifiziert, die mindestens eines der Gene, welche die Moleküle der Gruppe B kodieren, überexprimieren.

3. Verfahren gemäß Anspruch 2, **dadurch gekennzeichnet, dass** man im Schritt (a) die Expression mindestens zweier der genannten Gene der Gruppe B vergleicht, und dass man im Schritt (b) die Lymphozyten als regulatorische Tr1-Lymphozyten identifiziert, die zweierlei Gene der Gruppe B überexprimieren.

4. Verfahren gemäß Anspruch 3, **dadurch gekennzeichnet, dass** man im Schritt (a) die Expression aller Gene der Gruppe B vergleicht, und dass man im Schritt (b) die Lymphozyten, die alle Gene der Gruppe B überexprimieren, als regulatorische Tr1-Lymphozyten identifiziert.

5. Verfahren zur Identifizierung gemäß irgendeinem der Ansprüche 1 bis 4, **dadurch gekennzeichnet, dass** man im Schritt (a) unter anderem und gleichzeitig das Vorhandensein des Expressionsproduktes des Gens, welches für das Molekül CD3 kodiert, durch die Lymphozyten bestimmt, und **dadurch gekennzeichnet, dass** man im Schritt (b) die Lymphozyten, die unter anderem gleichzeitig das für das Molekül CD3 kodierende Gen exprimieren als regulatorische Tr1-Lymphozyten identifiziert.

6. Verfahren gemäß irgendeinem der Ansprüche 1 bis 5, **dadurch gekennzeichnet, dass** man im Schritt (a) das gleichzeitige Vorliegen der Moleküle der Gruppe A, welche auf der Oberfläche dieser Lymphozyten exprimiert werden, bestimmt.

7. Verfahren gemäß Anspruch 6, **dadurch gekennzeichnet, dass** man im Schritt (a) das gleichzeitige Vorhandensein der Moleküle, die auf der Oberfläche dieser Lymphozyten exprimiert sind, mit Hilfe spezifischer Antikörper gegen diese Moleküle bestimmt.

8. Verfahren gemäß Anspruch 7, **dadurch gekennzeichnet, dass** diese spezifischen Antikörper mit einer Markierung markiert sind, die in der Lage sind, in direkter oder indirekter Weise nachgewiesen zu werden.

9. Verfahren gemäß Anspruch 8, **dadurch gekennzeichnet, dass** jeder dieser Antikörper mit einer unterschiedlichen Markierung markiert ist.

10. Verfahren gemäß Anspruch 8 oder 9, **dadurch gekennzeichnet, dass** diese Markierungen fluoreszent sind und aus der Gruppe ausgewählt sind, die aus Fluoreszinisothiocyanat (FITC), Allophycocyanin (APC), Phycoerytrhincyanin 5 (PC5), Phycoerythrin (PE), grün fluoreszierendem Fluorescindiacetat, AM-Calcein und rot fluoreszierendem Tetramethylrhodamin besteht.

11. Verfahren gemäß irgendeinem der Ansprüche 6 bis 10, **dadurch gekennzeichnet, dass** man die Bestimmung des gleichzeitigen Vorliegens dieser Moleküle der Gruppe A, welche auf der Oberfläche dieser Lymphozyten exprimiert sind, im Schritt (a) mit Hilfe der Durchflusscytometrie durchführt.

12. Verfahren gemäß Anspruch 10 oder 11, **dadurch gekennzeichnet, dass** man im Schritt (a) des Verfahrens für das Molekül CD18 das Vorhandensein einer starken CD18-Fluoreszenzintensität nachweist.

13. Verfahren gemäß irgendeinem der Ansprüche 2 bis 12, **dadurch gekennzeichnet, dass**:
- im Schritt (a) der Vergleich der Expression durch die Lymphozyten von mindestens einem für die Moleküle der Gruppe B kodierenden Gen durch einen Vergleich der exprimierten mRNA-Menge dieses Gens durchgeführt wird; und
- dass man im Schritt (b) die Lymphozyten als regulatorische Tr1-Lymphozyten identifiziert, die die mRNA dieses Gens überexprimieren.

14. Verfahren gemäß Anspruch 13, **dadurch gekennzeichnet, dass** man die mRNA-Menge durch quantitative RT-PCR misst.

15. Verfahren gemäß irgendeinem der Ansprüche 1 bis 14, **dadurch gekennzeichnet, dass** die biologische Probe aus einer peripheren Blutabnahme oder aus einem entzündeten Organ bei einem Patienten stammt.

16. Verfahren gemäß Anspruch 15, **dadurch gekennzeichnet, dass** die Blutabnahme bei einem Individuum durchgeführt wird, das an einer Autoimmunerkrankung oder einer entzündlichen Erkankung leidet oder verdächtigt wird, darunter zu leiden.

17. Verfahren gemäß Anspruch 16, **dadurch gekennzeichnet, dass** das Individuum am Morbus Crohn oder an der Multiplen Sklerose leidet.

18. Verfahren gemäß irgendeinem der Ansprüche 1 bis 14, **dadurch gekennzeichnet, dass** die biologische Probe aus einem Herstellungsverfahren in vitro von regulatorischen Tr1-Lymphozyten ausgehend von einer Lymphozytenpopulation hervorgeht, die aus einer Blutabnahme bei einem Individuum stammen.

19. Verfahren gemäß Anspruch 18, **dadurch gekennzeichnet, dass** das Herstellungsverfahren der regulatorischen Tr1-Lymphozyten mindestens einen Aktivierungsschritt der CD4+ T Lymphozyten der Lymphozytenpopulation in Gegenwart eines Antigens und Interleukin 10 umfasst.

20. Verfahren gemäß Anspruch 18, **dadurch gekennzeichnet, dass** das Herstellungsverfahren der regulatorischen Tr1-Lymphozyten die folgenden Schritte umfasst:
a) Aktivieren von CD4+ T-Lymphozyten der Lymphozytenpopulation in Gegenwart eines ausgewählten Antigens in vitro, welches durch die künstlichen Antigen präsentierenden Zellen, die ein Molekül des HLA Klasse II-Systems exprimieren und ein menschliches LFA-3-Molekül, aber keines der Moleküle für die Co-Stimulation B7-1, B7-2, B7-H1, CD40, CD23 oder ICAM-1 exprimieren, welche aus einer biologischen Probe stammen; und
b) Gewinnen einer Population von CD4+ T-Lymphozyten, die aktiviert sind, aus diesen Lymphozyten, welche mindestens 10 % Tr1-Lymphozyten umfassen, die für das gewählte Antigen spezifisch sind.

21. Verfahren gemäß Anspruch 18, **dadurch gekennzeichnet, dass** das Herstellungsverfahren der regulatorischen Tr1-Lymphozyten die folgenden Schritte umfasst:
a) In vitro-Gewinnung einer Population menschlicher Vorläuferzellen, die in der Lage sind, sich in dendritische Zellen zu differenzieren;
b) In-Kultur-Bringen dieser menschlichen Vorläuferzellen in Gegenwart von IL-10, um eine Population der dendritischen Zellen zu gewinnen; und
c) Zusammengeben der menschlichen Lymphozytenpopulation mit der in (b) gewonnen Population dendritischer Zellen.

22. Verfahren gemäß irgendeinem der Ansprüche 1 bis 5, **dadurch gekennzeichnet, dass** die Expressionsprodukte dieser Lymphozyten bezüglich der Gene, die für Moleküle der Gruppe A kodieren, mRNA sind, und dass im Schritt (a) die Bestimmung des gleichzeitigen Vorhandenseins dieser mRNAs durch RT-PCR durchgeführt wird.

23. Verfahren zur Quantifizierung von regulatorischen Tr1-Lymphozyten, welche in einer biologischen Probe vorhanden sind, welche Lymphozyten umfasst, **dadurch gekennzeichnet, dass** sie die Schritte umfasst, die bestehen aus:
a) Identifizieren von regulatorischen Tr1-Lymphozyten durch ein Identifizierungsverfahren gemäß irgendeinem der Ansprüche 1 bis 21; und
b) Bestimmen des Anteils der regulatorischen Tr1-Lymphozyten, die in (a) identifizerte worden sind, bezogen auf die gesamte Zahl der Lymphozyten oder einer besonderen Fraktion von Lymphozyten, welche in dieser biologischen Probe vorhanden sind.

24. Prognostisches oder diagnostisches in vitro-Verfahren einer Autoimmunerkrankung oder entzündlichen Erkrankung bei einem Individuum, ausgehend von einer biologischen Probe, die vorzugsweise dem Individuum abgenommen wurde, **dadurch gekennzeichnet, dass** es die folgenden Schritte umfasst:
a) Bestimmen des Anteils der regulatorischen Tr1-Lymphozyten, welche in dieser biologischen Probe vorhanden sind, bezogen auf die gesamte Anzahl an Lymphozyten, oder einer besonderen Fraktion der Lymphozyten, gemäß dem Verfahren zur Quantifizierung nach Anspruch 23; und
b) Vergleichen des Anteils dieser regulatorischen Tr1-Lymphozyten, welche in Schritt (a) gewonnen wurden, bezogen auf den, der in der biologischen Probe vorhanden ist, welche einem gesunden Individuum abgenommen wurde.

25. Prognostisches oder diagnostisches in vitro-Verfahren einer Autoimmun- oder entzündlichen Erkrankung gemäß Anspruch 24, **dadurch gekennzeichnet, dass** man in Schritt (b) eine Verminderung dieses Anteils bei dem zu untersuchenden Individuum beobachtet.

26. Verfahren zur Anreicherung von regulatorischen Tr1-Lymphozyten, welche in einer biologischen Probe vorhanden sind, welche Lymphozyten umfasst, **dadurch gekennzeichnet, dass** es die folgenden Schritte umfasst:
a) Identifizieren von regulatorischen Tr1-Lymphozyten durch ein Identifizierungsverfahren gemäß irgendeinem der Ansprüche 1 bis 22; und
b) Eliminieren eines signifikanten Teils von Lymphozyten, die nicht gleichzeitig diese Moleküle aufweisen aus dieser Probe.
